# FASCICULE DE BREVET EUROPEEN

(11) **EP 1 299 365 B1**
(45) Date de publication et mention de la délivrance du brevet: **28.09.2005**
(21) Numéro de dépôt: 01943580.9
(22) Date de dépôt: 07.06.2001
(51) Int. Cl.: C07D 277/18, C07D 417/06, C07D 417/12, C07D 277/28, A61K 31/426, A61K 31/427, A61K 31/4439, A61K 31/497, A61P 25/00, C07D 213/38, C07D 213/40, C07D 333/20, C07D 241/12, C07D 213/70, C07D 213/89, C07C 335/06, C07C 215/00, C07C 229/00, C07C 233/00, C07C 271/00, C07C 333/00

(54) **DERIVES DE 2-AMINOTHIAZOLINE ET LEUR UTILISATION COMME INHIBITEURS DE NO-SYNTHASE**
2-AMINOTHIAZOLINDERIVATE UND IHRE VERWENDUNG ALS NO-SYNTHASE INHIBITOREN
2-AMINOTHIAZOLINE DERIVATIVES AND THEIR USE AS NO-SYNTHASE INHIBITORS

(30) Priorité: 09.06.2000 FR 0007397
(43) Date de publication de la demande: 09.04.2003
(73) Titulaire: Aventis Pharma S.A., 92160 Antony (FR)
(72) Inventeur: CARRY, Jean-Christophe, F-94100 Saint Maur des Fossés (FR); DAMOUR, Dominique, F-94310 Orly (FR); GUYON, Claude, F-94100 Saint Maur des Fossés (FR); MIGNANI, Serge, F-92290 Chatenay-Malabry (FR); BIGOT, Antony, F-91300 Massy (FR); BACQUE, Eric, 91190 Gif sur Yvette (FR); TABART, Michel, F-91290 La Norville (FR)
(74) Mandataire: Rousseau, Pierrick Edouard
(86) Numéro de dépôt international: PCT/FR2001/001760
(87) Numéro de publication internationale: WO 2001/094325

(56) Documents cités:
- WO-A-01/47931
- WO-A-94/12165
- WO-A-95/11231
- WO-A-96/14842
- US-A- 5 066 662
- LEVY O E ET AL: "POTENT AND SELECTIVE THROMBIN INHIBITORS INCORPORATING THE CONSTRAINED ARGININE MIMIC L-3-PIPERIDYL(N-GUANIDINO)ALANINE AT P1" JOURNAL OF MEDICINAL CHEMISTRY, AMERICAN CHEMICAL SOCIETY. WASHINGTON, US, vol. 39, no. 23, 8 novembre 1996 (1996-11-08), pages 4527-4530, XP000974280 ISSN: 0022-2623
- FRYDMAN B ET AL: "1,4-Diaminobutanes From Furans: A New Synthetic Approach to Substituted Putrescines" TETRAHEDRON LETTERS, ELSEVIER SCIENCE PUBLISHERS, AMSTERDAM, NL, vol. 39, no. 27, 2 juillet 1998 (1998-07-02), pages 4765-4768, XP004120752 ISSN: 0040-4039
- DEFAUW J M ET AL: "Synthesis and protein kinase C inhibitory activities of acyclic balanol analogs that are highly selective for protein kinase C over protein kinase A" JOURNAL OF MEDICINAL CHEMISTRY, vol. 39, no. 26, 20 décembre 1996 (1996-12-20), pages 5215-5227, XP002177939
- ENDO Y ET AL: "Synthesis, conformation, and biological activity of teleocidin mimics, benzolactams. A clarification of the conformational flexibility problem in structure-activity studies of teleocidins" JOURNAL OF THE AMERICAN CHEMICAL SOCIETY, vol. 118, no. 8, 28 février 1996 (1996-02-28), pages 1841-1855, XP002177940
- OCAIN T D ET AL: "Synthesis of sulfur-containing analogues of bestatin. Inhibition of aminopeptidases by alpha-thiolbestatin analogues" JOURNAL OF MEDICINAL CHEMISTRY, vol. 31, no. 11, novembre 1988 (1988-11), pages 2193-2199, XP002177941
- FENG X ET AL: "Synthesis of (S,S)-isodityrosinol in a fully differentiated form via Diels-Alder methodology" JOURNAL OF ORGANIC CHEMISTRY, vol. 57, no. 22, 23 octobre 1992 (1992-10-23), pages 5811-5812, XP002177942

## Description

La présente invention concerne l'utilisation de dérivés de 2-aminothiazoline de formule : 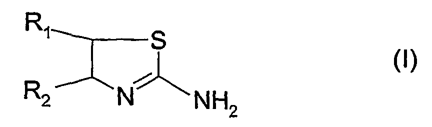 ou leurs sels pharmaceutiquement acceptables comme inhibiteurs de NO-synthase inductible.

L'invention a pour objet l'utilisation des dérivés de 2-aminothiazoline de formule (I) et leurs sels pharmaceutiquement acceptables pour la préparation de compositions pharmaceutiques destinées à prévenir et traiter les maladies dans lesquelles une production anormale de monoxyde d'azote (NO) par induction de NO-synthase inductible (NOS-2 ou iNOS) est impliquée, les compositions pharmaceutiques contenant les nouveaux dérivés de 2-aminothiazoline et leurs sels pharmaceutiquement acceptables et les dérivés de 2-aminothiazoline nouveaux et leurs sels pharmaceutiquement acceptables.

Le monoxyde d'azote (NO) est un radical diffusible impliqué dans de nombreux processus physiologiques et pathologiques. Il est synthétisé par oxydation de la L-arginine, une réaction catalysée par une famille d'enzymes appelée synthase du monoxyde d'azote ou NO-Synthase (NOS), référencée dans le système international de nomenclature des enzymes sous le numéro E.C. 1.14.13.39.

Trois isoformes de NOS, dont deux sont constitutives et une inductible, sont connues :
- une NOS neuronale (NOS-1 ou nNOS) a été isolée et clonée à l'origine à partir de tissu nerveux où c'est une enzyme constitutive. La NOS-1 produit du NO en réponse à divers stimuli physiologiques tels que l'activation de récepteurs membranaires selon un mécanisme dépendant du calcium et de la calmoduline.
- une NOS inductible (NOS-2 ou iNOS) peut être induite en réponse à des stimuli immunologiques tels que par exemple des cytokines ou des antigènes bactériens dans différentes cellules tels que par exemple les macrophages, les cellules endothéliales, les hépatocytes, les cellules gliales, ainsi qu'un grand nombre d'autres types de cellules. L'activité de cette isoforme n'est pas régulée par le calcium. C'est pourquoi une fois induite elle produit de grandes quantités de NO sur des durées prolongées.
- une NOS endothéliale (NOS-3 ou eNOS) est constitutive et calcium/calmoduline dépendante. Elle a été identifiée à l'origine dans les cellules de l'endothélium vasculaire où elle génère du NO en réponse à des stimuli physiologiques tels que l'activation de récepteurs membranaires.

Le NO produit par les isoformes constitutives neuronales et endothéliales (NOS-1 et NOS-3) est généralement impliqué dans des fonctions de signalisation intercellulaire. Par exemple, les cellules endothéliales qui tapissent la paroi interne des vaisseaux sanguins induisent la relaxation des cellules musculaires lisses sous-jacentes *via* la production de NO. Il contribue ainsi à la régulation de la pression artérielle.

Le NO produit en grande quantité par l'isoforme inductible NOS-2 est, entre autre, impliqué dans les phénomènes pathologiques associés aux processus inflammatoires aiguës et chroniques dans une grande variété de tissu et d'organes.

Une production excessive de NO par induction de NOS-2 participe ainsi de pathologies dégénératives du système nerveux comme par exemple la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie. De même, en dehors du système nerveux central, l'induction de NOS-2 est impliquée dans de nombreuses pathologies à composantes inflammatoires comme par exemple le diabète, l'athérosclérose, la myocardite, l'arthrite, l'arthrose, l'asthme, le syndrome du colon irritable, la maladie de Crohn, la péritonite, le reflux gastro-oesophagien, l'uvéite, le syndrome de Guillain-Barré, la glomérulo-néphrite, le lupus érythémateux, le psoriasis. La NOS-2 a également été impliquée dans la croissance de certaines formes de tumeurs comme par exemple des épithéliomes, des adénocarcinomes ou des sarcomes, et dans les infections par des bactéries intracellulaires ou extracellulaires, Gram-plus ou Gram-moins.

Dans toutes les situations où une surproduction de NO est néfaste, il apparaît donc souhaitable de diminuer la production de NO par l'administration de substances capables d'inhiber la NOS-2. Cependant, compte tenu des rôles physiologiques importants joués par l'isoforme constitutive NOS-3 en particulier dans la régulation de la pression artérielle, il est primordial que l'inhibition de l'isoforme NOS-2 affecte le moins possible l'isoforme NOS-3. En effet, il est connu que l'administration d'inhibiteurs non-sélectifs des isoformes de NOS conduit à une vasoconstriction et à un accroissement de la pression artérielle (Moncada, S., Palmer, R.M.J. et Higgs, E.A., Biosynthesis of nitric oxide from L-arginine : a pathway for the regulation of cell function and communication, *Biochem. Pharmacol.,* 1989, 38: 1709-1715). Ces effets sur le système cardiovasculaire sont délétères dans la mesure où ils diminuent l'apport en nutriments aux tissus. Par conséquent, la présente invention concerne des composés présentant une activité inhibitrice vis-à-vis de la NOS-2 significativement plus puissante que son activité inhibitrice vis-à-vis de la NOS-3.

Des inhibiteurs de NOS dérivés de thiazoline sont notamment décrits dans les demandes de brevet WO94/12165, WO95/11231 et WO96/14842.

La présente invention concerne l'utilisation des dérivés de 2-aminothiazoline de formule (I) dans laquelle :
soit R₁ est un atome d'hydrogène ou un radical alkyle et R₂ est un radical alkyle, -alk-NH₂, -CH₂-R₃, -CH₂-S-R₄ ou phényle substitué par un radical nitro ou -NH-C(=NH)CH₃,
soit R₁ est un radical alkyle et R₂ est un atome d'hydrogène,
R₃ est un radical cycloalkyle (3-6C), pyridyle, N-oxyde de pyridyle, thiényle, thiazolyle, imidazolyle, pyrazinyle, triazolyle, phényle ou phényle substitué par un radical nitro, hydroxy ou carboxy,
R₄ représente un radical pyridyle ou N-oxyde de pyridyle
alk représente un radical alkylène
pour la préparation de médicaments utiles pour prévenir ou traiter les maladies dans lesquelles une production anormale de monoxyde d'azote (NO) par induction de NO-synthase inductible (NOS-2 ou iNOS) est impliquée.

Dans les définitions précédentes et celles qui suivent, les radicaux alkyle et alkylène contiennent 1 à 6 atomes de carbone en chaîne droite ou ramifiée.

Les composés de formule (I) présentent un ou plusieurs carbones asymétriques et peuvent donc se présenter sous forme de racémique, d'énantiomères et de diastéréoisomères; ceux-ci font également partie de l'invention ainsi que leurs mélanges.

Par ailleurs les composés de formule (I) peuvent se présenter sous la forme tautomère (Ia) :

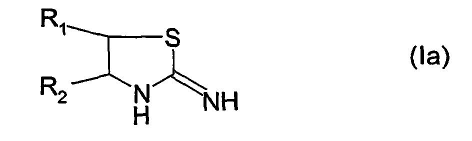

Ces tautomères font également partie de l'invention.

Parmi les composés de formule (I) utiles selon l'invention on peut citer les composés suivants :
4-(3-pyridylméthyl)-4,5-dihydro-1,3-thiazol-2-ylamine
4-(3-nitrobenzyl)-4,5-dihydro-1,3-thiazol-2-ylamine
4-benzyl-5-méthyl-4,5-dihydro-1,3-thiazol-2-ylamine
4-(3-thiénylméthyl)-4,5-dihydro-1,3-thiazol-2-ylamine
4-(2-thiénylméthyl)-4,5-dihydro-1,3-thiazol-2-ylamine
[3-(2-amino-4,5-dihydro-thiazol-4-yl)-phényl]-(1-imino-éthyl)-amine
4-benzyl-4,5-dihydro-1,3-thiazol-2-ylamine
4-(3-carboxybenzyl)-4,5-dihydro-1,3-thiazol-2-ylamine
4-(4-aminobutyl)-4,5-dihydro-1,3-thiazol-2-ylamine
4-butyl-4,5-dihydro-1,3-thiazol-2-ylamine
5-méthyl-4,5-dihydro-1,3-thiazol-2-ylamine
4-cyclohexylméthyl-4,5-dihydro-1,3-thiazol-2-ylamine
4-(3-nitrophényl)-4,5-dihydro-1,3-thiazol-2-ylamine
4-(4-pyridylméthyl)-4,5-dihydro-1,3-thiazol-2-ylamine
5-méthyl-4-(4-pyridylméthyl)-4,5-dihydro-1,3-thiazol-2-ylamine
5-éthyl-4-(4-pyridylméthyl)-4,5-dihydro-1,3-thiazol-2-ylamine
4-(4-thiazolylméthyl)-4,5-dihydro-1,3-thiazol-2-ylamine
4-(1-imidazolylméthyl)-4,5-dihydro-1,3-thiazol-2-ylamine
4-(2-pyrazinylméthyl)-4,5-dihydro-1,3-thiazol-2-ylamine
4-(5-thiazolylméthyl)-4,5-dihydro-1,3-thiazol-2-ylamine
4-(4-hydroxybenzyl)-4,5-dihydro-1,3-thiazol-2-ylamine
4-(4-pyridylsulfanylméthyl)-4,5-dihydro-1,3-thiazol-2-ylamine
4-(3-aminopropyl)-4,5-dihydro-1,3-thiazol-2-ylamine
4-(1-triazolylméthyl) -4,5-dihydro-1,3-thiazol-2-ylamine
N-oxyde de 4-(4-pyridylméthyl)-4,5-dihydro-1,3-thiazol-2-ylamine
leurs racémiques, énantiomères, diastéréoisomères, tautomères ainsi que leurs sels pharmaceutiquement acceptables,
et plus particulièrement les composés suivants :
(+)-(4R)-4-(3-pyridylméthyl)-4,5-dihydro-1,3-thiazol-2-ylamine
(+)-(4R)-4-(3-nitrobenzyl)-4,5-dihydro-1,3-thiazol-2-ylamine
(+)-(4R,5S)-4-benzyl-5-méthyl-4,5-dihydro-1,3-thiazol-2-ylamine
(+)-(4R,5R)-4-benzyl-5-méthyl-4,5-dihydro-1,3-thiazol-2-ylamine
(-)-(4R)-4-(3-thiénylméthyl)-4,5-dihydro-1,3-thiazol-2-ylamine
(4R)-4-(2-thiénylméthyl)-4,5-dihydro-1,3-thiazol-2-ylamine
[3-(2-amino-4,5-dihydro-thiazol-4-yl)-phényl]-(1-imino-éthyl)-amine
(+)-(4R)-4-benzyl-4,5-dihydro-1,3-thiazol-2-ylamine
(+)-(4R)-4-(3-carboxybenzyl)-4,5-dihydro-1,3-thiazol-2-ylamine
(+)-(4R)-4-(4-aminobutyl)-4,5-dihydro-1,3-thiazol-2-ylamine
(-)-(4S)-4-(3-nitrobenzyl)-4,5-dihydro-1,3-thiazol-2-ylamine
(-)-(4S,5S)-4-benzyl-5-méthyl-4,5-dihydro-1,3-thiazol-2-ylamine
(-)-(4S)-4-(4-aminobutyl)-4,5-dihydro-1,3-thiazol-2-ylamine
(4S,5R)-4-benzyl-5-méthyl-4,5-dihydro-1,3-thiazol-2-ylamine
(-)-(4R)-4-butyl-4,5-dihydro-1,3-thiazol-2-ylamine
(+)-(5S) -5-méthyl-4,5-dihydro-1,3-thiazol-2-ylamine
(-)-(4S)-4-cyclohexylméthyl-4,5-dihydro-1,3-thiazol-2-ylamine
(+)-(4R)-4-cyclohexylméthyl-4,5-dihydro-1,3-thiazol-2-ylamine
4-(3-nitophényl)-4,5-dihydro-1,3-thiazol-2-ylamine
(+)-(4R)-4-(4-pyridylméthyl)-4,5-dihydro-1,3-thiazol-2-ylamine
(+)-(4R,5R)-5-méthyl-4-(4-pyridylméthyl)-4,5-dihydro-1,3-thiazol-2-ylamine
(+)-(4R,5R)-5-éthyl-4-(4-pyridylméthyl)-4,5-dihydro-1,3-thiazol-2-ylamine
(+)-4-(5-thiazolylméthyl)-4,5-dihydro-1,3-thiazol-2-ylamine
(-)-4-(5-thiazolylméthyl)-4,5-dihydro-1,3-thiazol-2-ylamine
(+)-(4R)-4-(2-pyrazinylméthyl)-4,5-dihydro-1,3-thiazol-2-ylamine
(4R)-4-(1-imidazolylméthyl)-4,5-dihydro-1,3-thiazol-2-ylamine
(4S)-4-(1-imidazolylméthyl)-4,5-dihydro-1,3-thiazol-2-ylamine
(+)-(4R)-4-(4-thiazolylméthyl)-4,5-dihydro-1,3-thiazol-2-ylamine
(+)-(4R)-4-(3-aminopropyl)-4,5-dihydro-1,3-thiazol-2-ylamine
(+)-(4R)-4-(4-hydroxybenzyl)-4,5-dihydro-1,3-thiazol-2-ylamine
(+)-4-(4-pyridylsulfanylméthyl)-4,5-dihydro-1,3-thiazol-2-ylamine
N-oxyde de (4R)-4-(4-pyridylméthyl)-4,5-dihydro-1,3-thiazol-2-ylamine
(+)-4-(1-triazolylméthyl) -4,5-dihydro-1,3-thiazol-2-ylamine
leurs tautomères ainsi que leurs sels pharmaceutiquement acceptables.

Particulièrement préférés sont les composés de formule (I) utiles selon l'invention pour lesquels R₁ est un atome d'hydrogène ou un radical alkyle et R₂ est un radical -alk-NH₂, phényle substitué par un radical -NH-C(=NH)CH_{3,}-CH₂-R₃ ou -CH₂-S-R₄ R₃ est un radical pyridyle, thiényle, thiazolyle, imidazolyle, pyrazinyle, triazolyle, phényle ou phényle substitué par un radical nitro ou carboxy, R₄ est un radical pyridyle.

En particulier, lorsque R₂ est une chaîne -CH₂-R₃ ou -CH₂-S-R₄, R₃ est un radical 3-ou 4-pyridyle, 2- ou 3-thiényle, 4- ou 5-thiazolyle, 1-imidazolyle, 1-triazolyle, 2-pyrazinyle, phényle ou phényle substitué en position -3 par un radical nitro ou carboxy et R₄ est un radical 4-pyridyle.

Parmi les composé utiles selon l'invention et particulièrement préférés on peut citer les composés suivants :
4-(3-pyridylméthyl)-4,5-dihydro-1,3-thiazol-2-ylamine
4-(3-nitrobenzyl)-4,5-dihydro-1,3-thiazol-2-ylamine
4-benzyl-5-méthyl-4,5-dihydro-1,3-thiazol-2-ylamine
4-(3-thiénylméthyl)-4,5-dihydro-1,3-thiazol-2-ylamine
4-(2-thiénylméthyl)-4,5-dihydro-1,3-thiazol-2-ylamine
[3-(2-amino-4,5-dihydro-thiazol-4-yl)-phényl]-(1-imino-éthyl)-amine
4-benzyl-4,5-dihydro-1,3-thiazol-2-ylamine
4-(3-carboxybenzyl)-4,5-dihydro-1,3-thiazol-2-ylamine
4-(4-aminobutyl)-4,5-dihydro-1,3-thiazol-2-ylamine
4-(4-pyridylméthyl)-4,5-dihydro-1,3-thiazol-2-ylamine
5-méthyl-4-(4-pyridylméthyl)-4,5-dihydro-1,3-thiazol-2-ylamine
5-éthyl-4-(4-pyridylméthyl)-4,5-dihydro-1,3-thiazol-2-ylamine
4-(4-thiazolylméthyl)-4,5-dihydro-1,3-thiazol-2-ylamine
4-(1-imidazolylméthyl)-4,5-dihydro-1,3-thiazol-2-ylamine
4-(2-pyrazinylméthyl)-4,5-dihydro-1,3-thiazol-2-ylamine
4-(5-thiazolylméthyl)-4,5-dihydro-1,3-thiazol-2-ylamine
4-(4-pyridylsulfanylméthyl)-4,5-dihydro-1,3-thiazol-2-ylamine
4-(1-triazolylméthyl) -4,5-dihydro-1,3-thiazol-2-ylamine
leurs racémiques, énantiomères, diastéréoisomères, tautomères ainsi que leurs sels pharmaceutiquement acceptables,
et notamment les composés suivants :
(+)-(4R)-4-(3-pyridylméthyl)-4,5-dihydro-1,3-thiazol-2-ylamine
(+)-(4R)-4-(3-nitrobenzyl)-4,5-dihydro-1,3-thiazol-2-ylamine
(+)-(4R,5S)-4-benzyl-5-méthyl-4,5-dihydro-1,3-thiazol-2-ylamine
(+)-(4R,5R)-4-benzyl-5-méthyl-4,5-dihydro-1,3-thiazol-2-ylamine
(-)-(4R)-4-(3-thiénylméthyl)-4,5-dihydro-1,3-thiazol-2-ylamine
(4R)-4-(2-thiénylméthyl)-4,5-dihydro-1,3-thiazol-2-ylamine
[3-(2-amino-4,5-dihydro-thiazol-4-yl)-phényl]-(1-imino-éthyl)-amine
(+)-(4R)-4-benzyl-4,5-dihydro-1,3-thiazol-2-ylamine
(+)-(4R)-4-(3-carboxybenzyl)-4,5-dihydro-1,3-thiazol-2-ylamine
(+)-(4R)-4-(4-aminobutyl)-4,5-dihydro-1,3-thiazol-2-ylamine
(-)-(4S)-4-(3-nitrobenzyl)-4,5-dihydro-1,3-thiazol-2-ylamine
(-)-(4S,5S)-4-benzyl-5-méthyl-4,5-dihydro-1,3-thiazol-2-ylamine
(-)-(4S)-4-(4-aminobutyl)-4,5-dihydro-1,3-thiazol-2-ylamine
(4S,5R)-4-benzyl-5-méthyl-4,5-dihydro-1,3-thiazol-2-ylamine
(+)-(4R)-4-(4-pyridylméthyl)-4,5-dihydro-1,3-thiazol-2-ylamine
(+)-(4R,5R)-5-méthyl-4-(4-pyridylméthyl)-4,5-dihydro-1,3-thiazol-2-ylamine
(+)-(4R,5R)-5-éthyl-4-(4-pyridylméthyl)-4,5-dihydro-1,3-thiazol-2-ylamine
(+)-4-(5-thiazolylméthyl)-4,5-dihydro-1,3-thiazol-2-ylamine
(-)-4-(5-thiazolylméthyl)-4,5-dihydro-1,3-thiazol-2-ylamine
(+)-(4R)-4-(2-pyrazinylméthyl)-4,5-dihydro-1,3-thiazol-2-ylamine
(4R)-4-(1-imidazolylméthyl)-4,5-dihydro-1,3-thiazol-2-ylamine
(4S)-4-(1-imidazolylméthyl)-4,5-dihydro-1,3-thiazol-2-ylamine
(+)-(4R)-4-(4-thiazolylméthyl)-4,5-dihydro-1,3-thiazol-2-ylamine
(+)-4-(4-pyridylsulfanylméthyl)-4,5-dihydro-1,3-thiazol-2-ylamine
(+)-4-(1-triazolylméthyl) -4,5-dihydro-1,3-thiazol-2-ylamine
leurs tautomères ainsi que leurs sels pharmaceutiquement acceptables.

Les composés de formule (I) pour lesquels R₁ est méthyle ou hexyle et R₂ est hydrogène ou bien R₁ et R₂ sont méthyle sont connus en tant que produits chimiques (J. Org. Chem., 27, 1049 (1962) ; J. Org. Chem., 37, 4401 (1972) ; Beilstein Registry Numbers 6114855,6114856,6117694,6117695).

Par ailleurs, certains composés de formule (I) sont connus comme radioprotecteurs. Ce sont les composés racémiques pour lesquels
R₁ est un radical méthyle et R₂ est un atome d'hydrogène (Chem. Abst., 1980, 92, 209060 et 209061),
R₁ est hydrogène et R₂ est méthyle (Chem. Abst., 1997, 87, 193910),
R₁ est hydrogène et R₂ est éthyle (Khim. Geterotsikl. Soedin, 1987, 11, 1572),
R₁ est hydrogène et R₂ est n-propyl (Radiobiologiya, 1979, 19 (5), 671).

Les autres composés de formule (I) sont nouveaux et en tant que tels font partie de l'invention ainsi que leurs racémiques, énantiomères, diastéréoisomères et tautomères et leurs sels pharmaceutiquement acceptables.

Ce sont les composés pour lesquels
soit R₁ est un atome d'hydrogène ou un radical alkyle et R₂ est un radical alkyle, -alk-NH₂, -CH₂-R₃, -CH₂-S-R₄ ou phényle substitué par un radical nitro ou -NH-C(=NH)CH₃,
soit R₁ est un radical alkyle et R₂ est un atome d'hydrogène,
R₃ est un radical cycloalkyle (3-6C), pyridyle, thiényle, thiazolyle, imidazolyle, pyrazinyle, triazolyle, phényle ou phényle substitué par un radical nitro, hydroxy ou carboxy,
R₄ représente un radical pyridyle,
alk représente un radical alkylène,
leurs racémiques, énantiomères, diastéréoisomères et leurs mélanges, leurs tautomères et leurs sels pharmaceutiquement acceptables,
à l'exception des composés pour lesquels R₁ est hexyle ou méthyle et R₂ est hydrogène ou bien R₁ et R₂ sont méthyle et les racémiques des composés pour lesquels R₁ est hydrogène et R₂ est méthyle, éthyle ou n-propyle.

Sont particulièrement préférés les composés de formule (I) pour lesquels R₁ est un atome d'hydrogène ou un radical alkyle et R₂ est un radical -alk-NH₂, phényle substitué par un radical -NH-C(=NH)CH₃, -CH₂-R₃ pour lequel R₃ est un radical pyridyle, thiényle, thiazolyle, imidazolyle, triazolyle, pyrazinyle, phényle ou phényle substitué par un radical nitro ou carboxy ou -CH₂-S-R₄ pour lequel R₄ est un radical pyridyle, leurs racémiques, énantiomères, diastéréoisomères et leurs mélanges, leurs tautomères et leurs sels pharmaceutiquement acceptables.

En particulier, R₃ est un radical 3- ou 4-pyridyle, 2- ou 3-thiényle, 4- ou 5-thiazolyle, 1-imidazolyle, 1-triazolyle, 2-pyrazinyle, phényle ou phényle substitué en position -3 par un radical nitro ou carboxy et R₄ est un radical 4-pyridyle.

Parmi les composés nouveaux de formule (I), on peut citer les composés suivants :
4-(3-pyridylméthyl)-4,5-dihydro-1,3-thiazol-2-ylamine
4-(3-nitrobenzyl)-4,5-dihydro-1,3-thiazol-2-ylamine
4-benzyl-5-méthyl-4,5-dihydro-1,3-thiazol-2-ylamine
4-(3-thiénylméthyl)-4,5-dihydro-1,3-thiazol-2-ylamine
4-(2-thiénylméthyl)-4,5-dihydro-1,3-thiazol-2-ylamine
[3-(2-amino-4,5-dihydro-thiazol-4-yl)-phényl]-(1-imino-éthyl)-amine
4-benzyl-4,5-dihydro-1,3-thiazol-2-ylamine
4-(3-carboxybenzyl)-4,5-dihydro-1,3-thiazol-2-ylamine
4-(4-aminobutyl)-4,5-dihydro-1,3-thiazol-2-ylamine
4-(4-pyridylméthyl)-4,5-dihydro-1,3-thiazol-2-ylamine
5-méthyl-4-(4-pyridylméthyl)-4,5-dihydro-1,3-thiazol-2-ylamine
5-éthyl-4-(4-pyridylméthyl)-4,5-dihydro-1,3-thiazol-2-ylamine
4-(4-thiazolylméthyl)-4,5-dihydro-1,3-thiazol-2-ylamine
4-(1-imidazolylméthyl)-4,5-dihydro-1,3-thiazol-2-ylamine
4-(2-pyrazinylméthyl)-4,5-dihydro-1,3-thiazol-2-ylamine
4-(5-thiazolylméthyl)-4,5-dihydro-1,3-thiazol-2-ylamine
4-(1-triazolylméthyl) -4,5-dihydro-1,3-thiazol-2-ylamine
4-(4-pyridylsulfanylméthyl)-4,5-dihydro-1,3-thiazol-2-ylamine
leurs racémiques, énantiomères, diastéréoisomères, tautomères ainsi que leurs sels pharmaceutiquement acceptables,
et notamment les composés suivants :
(+)-(4R)-4-(3-pyridylméthyl)-4,5-dihydro-1,3-thiazol-2-ylamine
(+)-(4R)-4-(3-nitrobenzyl)-4,5-dihydro-1,3-thiazol-2-ylamine
(+)-(4R,5S)-4-benzyl-5-méthyl-4,5-dihydro-1,3-thiazol-2-ylamine
(+)-(4R,5R)-4-benzyl-5-méthyl-4,5-dihydro-1,3-thiazol-2-ylamine
(-)-(4R)-4-(3-thiénylméthyl)-4,5-dihydro-1,3-thiazol-2-ylamine
(4R)-4-(2-thiénylméthyl)-4,5-dihydro-1,3-thiazol-2-ylamine ??
[3-(2-amino-4,5-dihydro-thiazol-4-yl)-phényl]-(1-imino-éthyl)-amine
(+)-(4R)-4-benzyl-4,5-dihydro-1,3-thiazol-2-ylamine
(+)-(4R)-4-(3-carboxybenzyl)-4,5-dihydro-1,3-thiazol-2-ylamine
(+)-(4R)-4-(4-aminobutyl)-4,5-dihydro-1,3-thiazol-2-ylamine
(-)-(4S)-4-(3-nitrobenzyl)-4,5-dihydro-1,3-thiazol-2-ylamine
(-)-(4S,5S)-4-benzyl-5-méthyl-4,5-dihydro-1,3-thiazol-2-ylamine
(-)-(4S)-4-(4-aminobutyl)-4,5-dihydro-1,3-thiazol-2-ylamine
(4S,5R)-4-benzyl-5-méthyl-4,5-dihydro-1,3-thiazol-2-ylamine
(+)-(4R)-4-(4-pyridylméthyl)-4,5-dihydro-1,3-thiazol-2-ylamine
(+)-(4R,5R)-5-méthyl-4-(4-pyridylméthyl)-4,5-dihydro-1,3-thiazol-2-ylamine
(+)-(4R,5R)-5-éthyl-4-(4-pyridylméthyl)-4,5-dihydro-1,3-thiazol-2-ylamine
(+)-4-(5-thiazolylméthyl)-4,5-dihydro-1,3-thiazol-2-ylamine
(-)-4-(5-thiazolylméthyl)-4,5-dihydro-1,3-thiazol-2-ylamine
(+)-(4R)-4-(2-pyrazinylméthyl)-4,5-dihydro-1,3-thiazol-2-ylamine
(4R)-4-(1-imidazolylméthyl)-4,5-dihydro-1,3-thiazol-2-ylamine
(4S)-4-(1-imidazolylméthyl)-4,5-dihydro-1,3-thiazol-2-ylamine
(+)-(4R)-4-(4-thiazolylméthyl)-4,5-dihydro-1,3-thiazol-2-ylamine
(+)-4-(4-pyridylsulfanylméthyl)-4,5-dihydro-1,3-thiazol-2-ylamine
(+)-4-(1-triazolylméthyl) -4,5-dihydro-1,3-thiazol-2-ylamine
leurs tautomères ainsi que leurs sels pharmaceutiquement acceptables.

L'invention concerne également les compositions pharmaceutiques contenant en tant que principe actif un dérivé de formule (I) pour lequel
soit R₁ est un atome d'hydrogène ou un radical alkyle et R₂ est un radical alkyle, -alk-NH₂, -CH₂-R₃, -CH₂-S-R₄ ou phényle substitué par un radical nitro ou -NH-C(=NH)CH₃,
soit R₁ est un radical alkyle et R₂ est un atome d'hydrogène,
R₃ est un radical cycloalkyle (3-6C), pyridyle, thiényle, thiazolyle, imidazolyle, pyrazinyle, triazolyle, phényle ou phényle substitué par un radical nitro, hydroxy ou carboxy,
R₄ représente un radical pyridyle, alk représente un radical alkylène,
ainsi que ses racémiques, énantiomères, diastéréoisomères et leurs mélanges, son tautomère et ses sels pharmaceutiquement acceptables, à l'exception des composés racémiques pour lesquels R₁ est un radical méthyle et R₂ est un atome d'hydrogène ou bien R₁ est hydrogène et R₂ est méthyle, éthyle ou n-propyle.

Les composés de formule (I) peuvent être préparés par cyclisation d'un dérivé de formule : 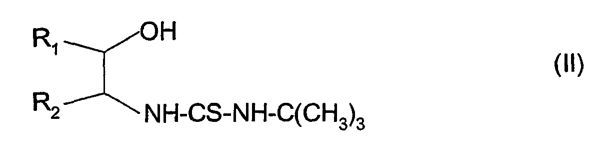 dans laquelle R₁ et R₂ ont les mêmes significations que dans la formule (I).

Cette cyclisation s'effectue généralement au moyen d'un acide tel que l'acide chlorhydrique, en milieu aqueux, à une température de 100°C. De préférence, on utilise l'acide chlorhydrique 6N.

Les dérivés de formule (II) peuvent être obtenus selon les schémas réactionnels suivants :

Schéma 1 pour les composés pour lesquels R₁ est hydrogène 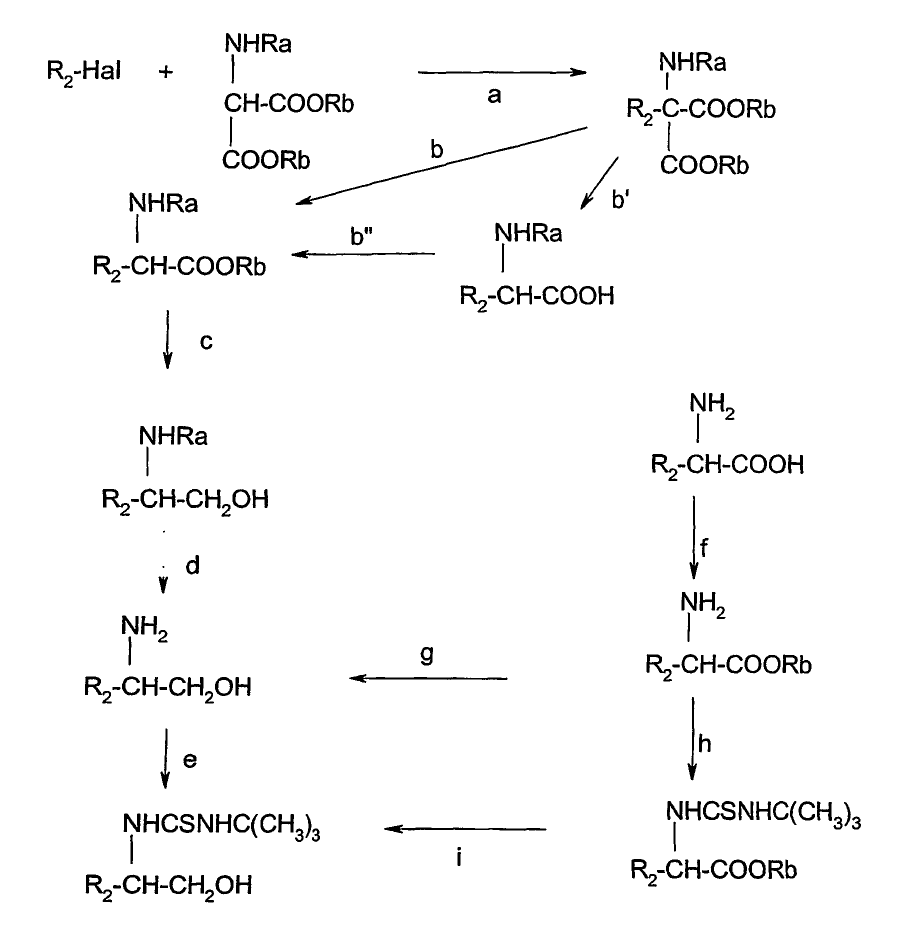 dans ces formules R₂ a les mêmes significations que dans la formule (I), Ra est un atome d'hydrogène ou un groupe protecteur de la fonction amine tel que ceux décrits par T.W. GREENE, Protective groups in Organic Synthesis, J. Wiley-Interscience Publication (1991) et Rb est un radical alkyle (1-4C) ou alcoxycarbonyle, de préférence, méthyle, éthyle ou isobutyloxycarbonyle. De préférence le groupe protecteur de la fonction amine est un radical acétyle ou tert-butoxycarbonyle.

La réaction a s'effectue généralement en présence d'alcoolate de sodium (1-4C) (éthylate de sodium de préférence), au sein de l'alcool correspondant, à une température comprise entre 10°C et la température d'ébullition du milieu réactionnel.

La réaction b s'effectue généralement au sein d'un solvant inerte tel que le diméthylformamide en présence d'iodure de lithium, à une température comprise entre 100°C et la température d'ébullition du milieu réactionnel ou bien au sein d'un alcool aliphatique (1-4C), en présence de soude, à une température de 10 à 30°C suivie d'une neutralisation par HCl 6N puis d'un chauffage au sein d'un solvant tel que le dioxanne à une température voisine de 100°C.

La réaction b' s'effectue de préférence au moyen d'acide chlorhydrique, à une température de 100°C.

La réaction b" pour les dérivés pour lesquels Rb est un radical alkyle s'effectue généralement par action d'un alcool aliphatique (1-4C) (méthanol, éthanol de préférence), en présence d'un acide minéral tel que l'acide sulfurique, à une température comprise entre 50°C et la température d'ébullition du milieu réactionnel. Pour les dérivés pour lesquels Rb est un radical isobutyloxycarbonyle, cette réaction s'effectue généralement par action de choroformiate d'isobutyle en présence d'une base telle que la triéthylamine, au sein d'un solvant inerte tel que le tétrahydrofuranne, à une température comprise entre -20°C et 0°C.

Les réactions de réduction c, g et i s'effectuent de préférence au moyen d'un hydrure tel que le borohydrure de sodium ou l'aluminohydrure de lithium, au sein d'un alcool aliphatique (1-4C) ou du tétrahydrofuranne, à une température comprise entre 10°C et 30°C, ou bien au moyen d'un dérivé du borane tel que le complexe BH₃-THF, au sein d'un solvant tel que le tétrahydrofuranne, à une température comprise entre 0°C et 30°C.

La réaction de déprotection d pour les composés pour lesquels Ra est un groupe protecteur de la fonction amine s'effectue par toute méthode de déprotection connue de l'homme de l'art et notamment celles décrites par T.W. GREENE, Protective groups in Organic Synthesis, J. Wiley-Interscience Publication (1991). De préférence lorsque le groupe protecteur est un radical acétyle cette réaction s'effectue au moyen d'acide chlorhydrique aqueux, à une température de 100°C. Lorsque le groupe protecteur est un radical tert-butoxycarbonyle cette réaction s'effectue au moyen d'acide chlorhydrique au sein du dioxanne, à une température voisine de 20°C.

Les réactions e et h s'effectuent par action du tert-butyl isothiocyanate, au sein d'un solvant inerte tel qu'un alcool aliphatique (1-4C) (méthanol, éthanol de préférence), éventuellement en présence d'une amine tertiaire telle que la triéthylamine, à une température comprise entre 20°C et la température d'ébullition du milieu réactionnel.

La réaction f s'effectue par action d'un alcool aliphatique (1-4C) (méthanol ou éthanol de préférence), en présence d'un acide minéral tel que l'acide chlorhydrique, à une température comprise entre 20°C et la température d'ébullition du milieu réactionnel ou bien par action du chlorure de thionyle, au sein d'un alcool aliphatique (1-4C) (méthanol ou éthanol de préférence), à une température comprise entre -25°C et 30°C.

Schéma 2 pour les composés pour lesquels R₁ est alkyle 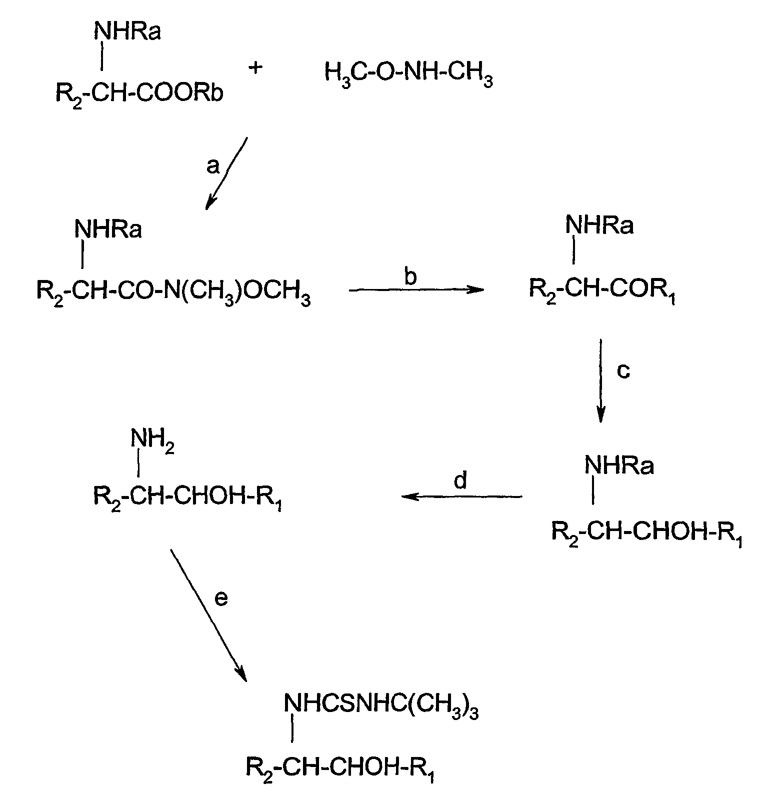 dans ces formules R₁ et R₂ ont les mêmes significations que dans la formule (I), Ra est un atome d'hydrogène ou un groupe protecteur de la fonction amine tel que ceux décrits par T.W. GREENE, Protective groups in Organic Synthesis, J. Wiley-Interscience Publication (1991) et, de préférence, un radical acétyle ou tert-butoxycarbonyle et Rb est un radical alkyle ou alcoxycarbonyle et, de préférence, méthyle, éthyle ou isobutyloxycarbonyle.

La réaction a s'effectue de préférence, au sein d'un solvant inerte tel qu'un solvant chloré (chloroforme ou dichlorométhane par exemple), éventuellement en présence d'une base telle que la N-méthylmorpholine ou la triéthylamine, à une température comprise entre -15°C et 30°C.

La réaction b s'effectue par action d'un halogénure d'alkylmagnésium tel qu'un bromure d'alkylmagnésium, au sein d'un éther tel que le tétrahydrofuranne ou l'éther éthylique, à une température comprise entre 0°C et 30°C.

La réaction de réduction c s'effectue de préférence au moyen d'un hydrure tel que le borohydrure de sodium ou l'aluminohydrure de lithium, au sein d'un alcool aliphatique (1-4C) ou du tétrahydrofuranne, à une température comprise entre 10°C et 30°C, ou bien au moyen d'un dérivé du borane tel que le complexe BH₃-THF, au sein d'un solvant tel que le tétrahydrofuranne, à une température comprise entre 0°C et 30°C.

La réaction de déprotection d s'effectue par toute méthode de déprotection connue de l'homme de l'art d'une fonction amine et notamment celles décrites par T.W. GREENE, Protective groups in Organic Synthesis, J. Wiley-Interscience Publication (1991). De préférence lorsque le groupe protecteur est un radical acétyle cette réaction s'effectue au moyen d'acide chlorhydrique aqueux, à une température de 100°C. Lorsque le groupe protecteur est un radical tert-butoxycarbonyle cette réaction s'effectue au moyen d'acide chlorhydrique au sein du dioxanne, à une température voisine de 20°C.

La réaction e s'effectue par action du tert-butyl isothiocyanate, au sein d'un solvant inerte tel qu'un alcool aliphatique (1-4C) (méthanol, éthanol de préférence), en présence d'une amine tertiaire telle que la triéthylamine, à une température comprise entre 20°C et la température d'ébullition du milieu réactionnel.

Les composés de formule (I) pour lesquels R₂ est un radical phényle substitué par un radical -NH-C(=NH)CH₃ peuvent également être préparés à partir des dérivés aminés correspondants eux-mêmes obtenus par réduction des dérivés nitrés de formule (I) selon le schéma suivant :

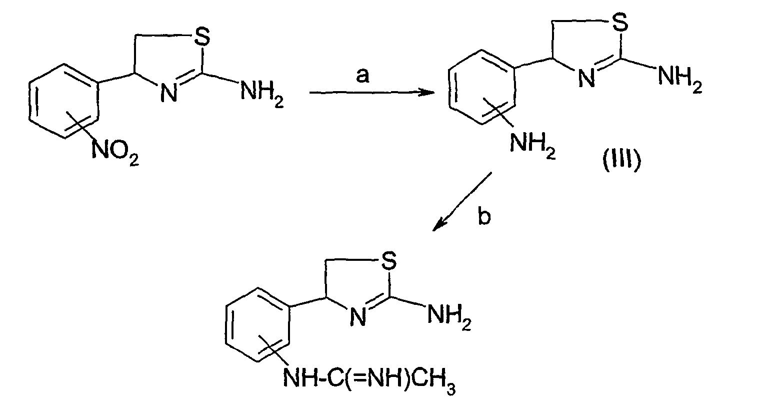

La réaction a de réduction s'effectue par toute méthode de réduction connue de l'homme de l'art permettant de passer d'un nitro à un amino sans toucher au reste de la molécule. De préférence, on opère au moyen du zinc, au sein de l'acide acétique, à une température voisine de 20°C.

La réaction b s'effectue par réaction du chlorhydrate de benzyléthanimidothioate, au sein d'un solvant inerte tel qu'un alcool aliphatique (1-4C) et de préférence le méthanol ou l'éthanol, à une température comprise entre 0°C et 45°C.

Les intermédiaires de formule (III) sont nouveaux et font partie de l'invention.

Les composés de formule (I) pour lesquels R₂ est un radical -CH₂-R₃ dans lequel R₃ est un radical 1-imidazolyle ou 1-(1,2,4-triazolyle) peuvent également être préparés par action d'imidazole ou de 1,2,4-triazole sur un dérivé de formule : 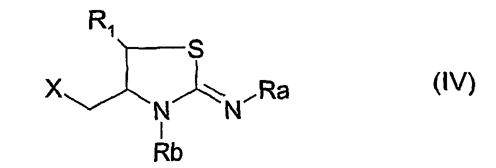 dans laquelle R₁ a les mêmes significations que dans la formule (I), X est un atome d'halogène et, en particulier, un atome d'iode, ou un radical tosyle, Ra et Rb sont des atomes d'hydrogène ou des groupes protecteurs de la fonction amine tel que ceux décrits par T.W. GREENE, Protective groups in Organic Synthesis, J. Wiley-Interscience Publication (1991), de préférence alcoxycarbonyle ou acétyle et plus particulièrement tert-butyloxycarbonyle, suivie éventuellement d'une déprotection.

Cette réaction s'effectue généralement en présence d'une base telle qu'un hydrure de métal alcalin, de préférence l'hydrure de sodium, au sein d'un solvant tel que le diméthylsulfoxyde, à une température comprise entre 20°C et la température d'ébullition du milieu réactionnel.

La réaction de déprotection pour les composés pour lesquels Ra ou Rb est un groupe protecteur de la fonction amine s'effectue par toute méthode de déprotection connue de l'homme de l'art et notamment celles décrites par T.W. GREENE, Protective groups in Organic Synthesis, J. Wiley-Interscience Publication (1991). De préférence lorsque le groupe protecteur est un radical acétyle cette réaction s'effectue au moyen d'acide chlorhydrique aqueux, à une température de 100°C. Lorsque le groupe protecteur est un radical tert-butyloxycarbonyle cette réaction s'effectue au moyen d'acide chlorhydrique au sein du dioxanne, à une température voisine de 20°C.

Les composés de formule (IV) peuvent eux-mêmes être obtenus selon le schéma réactionnel suivant :

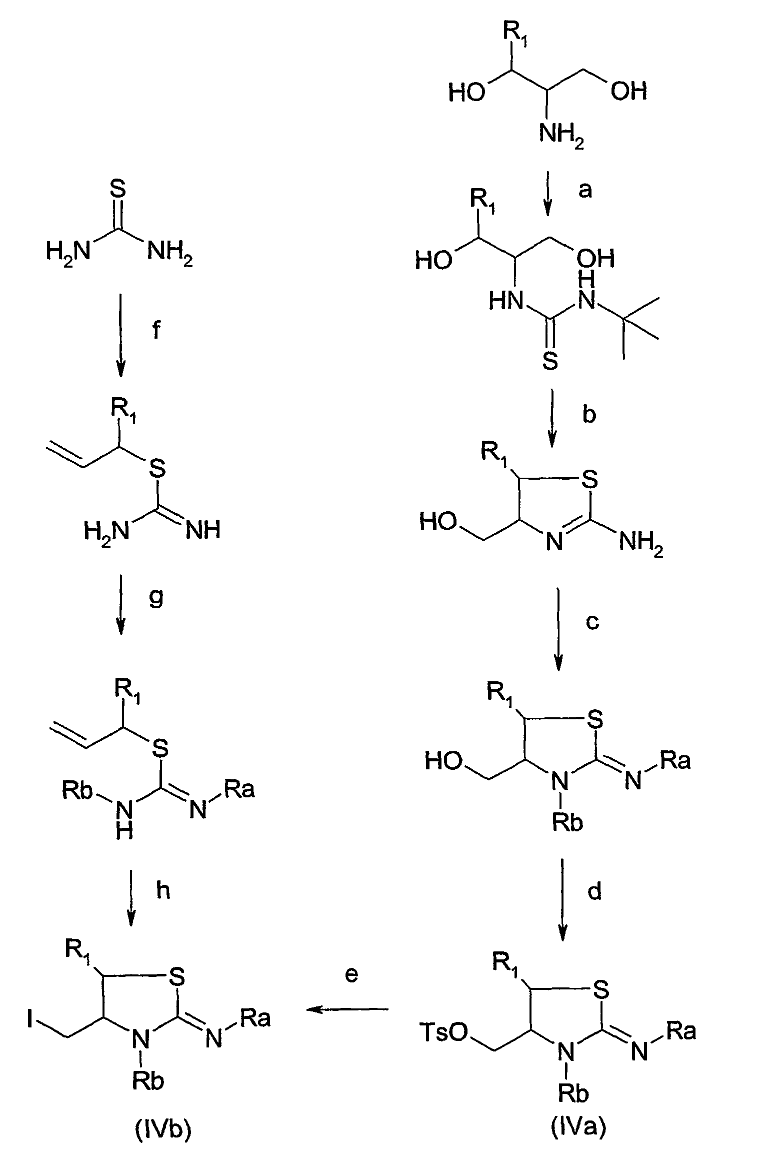

Dans ces formules, R₁ a la même signification que dans la formule (I), Ts est un radical tosylate, Ra et Rb sont un atome d'hydrogène ou un groupe protecteur de la fonction amine tel que ceux décrits par T.W. GREENE, Protective groups in Organic Synthesis, J. Wiley-Interscience Publication (1991), de préférence alcoxycarbonyle ou acétyle et plus particulièrement tert-butyloxycarbonyle.

La réaction a s'effectue généralement par action du tert-butyl isothiocyanate, au sein d'un solvant inerte tel qu'un alcool aliphatique (1-4C) (méthanol, éthanol de préférence), éventuellement en présence d'une amine tertiaire telle que la triéthylamine, à une température comprise entre 20°C et la température d'ébullition du milieu réactionnel.

La réaction de cyclisation b s'effectue généralement au moyen d'un acide tel que l'acide chlorhydrique, en milieu aqueux, à une température voisine de 100°C. De préférence, on utilise l'acide chlorhydrique 6N.

Les réactions c et g lorsque Ra ou Rb est un groupe tert-butoxycarbonyle s'effectuent par toute méthode de protection connue de l'homme de l'art et notamment celles décrites par T.W. GREENE, Protective groups in Organic Synthesis, J. Wiley-Interscience Publication (1991). De préférence cette réaction s'effectue au moyen de dicarbonate de bis-tert-butyle, en présence d'une base telle que la triéthylamine et éventuellement en présence de 4-(diméthylamino)pyridine, au sein d'un solvant tel que le dichlorométhane et à une température voisine de 20°C, ou bien en présence d'une base telle que le carbonate de potassium, au sein d'un solvant tel que l'eau et à une température voisine de 20°C.

La réaction d s'effectue généralement par action du chlorure de p-toluène sulfonyle, en présence d'une amine tertiaire telle que la triéthylamine, au sein d'un solvant inerte tel que le dichlorométhane, à une température comprise entre 20°C et la température d'ébullition du milieu réactionnel.

La réaction e s'effectue généralement par action de l'iodure de sodium, au sein d'un solvant inerte tel que l'acétone, à une température comprise entre 20°C et la température d'ébullition du milieu réactionnel.

La réaction f s'effectue généralement par action d'un halogénure d'allyle, par exemple le chlorure d'allyle, au sein d'un alcool aliphatique (1-4C), de préférence l'éthanol, à une température comprise entre 20°C et la température d'ébullition du milieu réactionnel.

La réaction h s'effectue généralement par action d'iode, en présence d'une base telle que le bicarbonate de sodium, au sein d'un solvant tel que le dichlorométhane, à une température comprise entre 20°C et la température d'ébullition du milieu réactionnel.

Les composés de formule (I) pour lesquels R₂ est un radical -CH₂-S-R₄ peuvent être préparés par action d'un composé de formule (IVa) ou (IVb) correspondant avec un dérivé de formule HS-R₄ dans laquelle R₄ a la même signification que dans la formule (I), Ra et Rb sont des atomes d'hydrogène ou des groupes protecteurs de la fonction amine tels que ceux décrits par T.W. GREENE, Protective groups in Organic Synthesis, J. Wiley-Interscience Publication (1991), de préférence alcoxycarbonyle ou acétyle et plus particulièrement tert-butyloxycarbonyle suivie éventuellement d'une déprotection de la fonction amine.

Cette réaction s'effectue généralement en présence d'une base telle que le carbonate de potassium, au sein d'un solvant tel que l'acétonitrile ou le diméthylformamide (acétonitrile de préférence), à une température comprise entre 20°C et la température d'ébullition du milieu réactionnel.

La réaction de déprotection pour les composés pour lesquels Ra ou Rb est un groupe protecteur de la fonction amine s'effectue par toute méthode de déprotection connue de l'homme de l'art et notamment celles décrites par T.W. GREENE, Protective groups in Organic Synthesis, J. Wiley-Interscience Publication (1991). De préférence lorsque le groupe protecteur est un radical acétyle cette réaction s'effectue au moyen d'acide chlorhydrique aqueux, à une température de 100°C. Lorsque le groupe protecteur est un radical tert-butoxycarbonyle cette réaction s'effectue au moyen d'acide chlorhydrique au sein du dioxanne, à une température voisine de 20°C. Les composés de formule (I) sont isolés et peuvent être purifiés par les méthodes connues habituelles, par exemple par cristallisation, chromatographie ou extraction.

Les énantiomères des composés de formule (I) peuvent être obtenus par dédoublement des racémiques par exemple par chromatographie sur colonne chirale selon PIRCKLE W.H. et coll., asymmetric synthesis, vol. 1, Academic Press (1983) ou par formation de sels ou par synthèse à partir des précurseurs chiraux. Les diastéréoisomères peuvent être préparés selon les méthodes classiques connues (cristallisation, chromatographie ou à partir des précurseurs chiraux).

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, iséthionate, maléate, méthanesulfonate, méthylène-bis-b-oxynaphtoate, nitrate, oxalate, pamoate, phosphate, salicylate, succinate, sulfate, tartrate, théophyllinacétate et p-toluènesulfonate.

Les composés de formule (I) sont des inhibiteurs de NO-synthase inductible ou NO-synthase de type 2 (NOS-2) et sont ainsi utiles pour la prévention et le traitement des désordres liés à une production excessive de NO telles que la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie, le diabète, l'athérosclérose, la myocardite, l'arthrite, l'arthrose, l'asthme, le syndrome du colon irritable, la maladie de Crohn, la péritonite, le reflux gastro-oesophagien, l'uveite, le syndrome de Guillain-Barré, la glomerulo-néphrite, le lupus erythémateux, le psoriasis, la croissance de certaines formes de tumeurs comme par exemple les épithéliomes, les adénocarcinomes ou les sarcomes, et les infections par des bactéries intracellulaires ou extracellulaires, gram-plus ou gram-moins.

Leurs activités en tant qu'inhibiteurs de NOS-2 et NOS-3 ont été déterminées par la mesure de la conversion de [³H]-L-arginine en [³H]-L-citrulline par, respectivement, une fraction enzymatique NOS-2 extraite de poumons de rats ou de souris préalablement traités par des lipopolysaccharides (10 mg/kg i.p. 6 heures avant le recueil de tissu) et par une préparation commerciale de NOS-3 recombinante de boeuf. Les composés ont été incubés pendant 20 à 30 minutes à 37°C en présence de 5 µM (pour activité NOS-2) ou 10 µM (pour activité NOS-3) de [³H]-L-arginine, 1 mM de NADPH, 15 µM de tétrabiopterine, 1µM de FAD, 0.1 mM de DTT dans un tampon HEPES (50 mM, pH 6,7) contenant 10 µg/ml de calmoduline et 1.25 mM de CaCl₂ lorsque l'activité NOS-3 a été mesurée. L'incubation a été arrêtée par addition de tampon HEPES froid (100 mM, pH 5,5) contenant 10 mM d'EGTA et 500 mg d'une résine cationique échangeuse d'ion (AG50W-X8, contre-ion : Na⁺) pour séparer la [³H]-L-arginine de la [³H]-L-citrulline. Après 5 min de décantation, la radioactivité restant dans la phase liquide a été mesurée dans un compteur à scintillation en présence d'un liquide scintillant approprié. Le rendement de la récupération de la L-[³H]citrulline formée a pu être estimée en utilisant de la L-[ureido-¹⁴C]-citrulline comme standard externe.
L'activité NOS-2 ou NOS-3 a été exprimée en picomole(s) de [³H]-L-citrulline formée par minute et par milligramme de protéine contenu dans le milieu réactionel.

Dans ce test sur l'enzyme NOS-2, la CI₅₀ des composés de formule (I) est inférieure ou égale à 10 µM.

La sélectivité est mesurée par le rapport CI₅₀ NOS-3 / CI₅₀ NOS-2. Cette sélectivité est supérieure à 20.

Les composés de formule (I) présentent une toxicité faible. Leur DL₅₀ est supérieure à 40 mg/kg par voie sous cutanée chez la souris.

Les exemples suivants illustrent l'invention.

### Exemple 1

### Dichlorhydrate de (+)-(4R)-4-(3-pyridylméthyl)-4,5-dihydro-1,3-thiazol-2-ylamine

Une suspension de 3,35 g de N-(*tert*-butyl)-N'-[(1R)-2-hydroxy-1-(3-pyridylméthyl) éthyl]thiourée dans 32 cm³ d'acide chlorhydrique 6N est chauffée à une température voisine de 100°C pendant 5 heures puis on arrête le chauffage. La solution est ensuite évaporée sous pression réduite (2 kPa) à une température voisine de 50°C. On obtient 4.42g d'une huile jaune qui devient pâteuse. Cette pâte est reprise dans de l'acétone puis évaporée de nouveau sous pression réduite (2 kPa) à une température voisine de 40°C. Le résidu est repris dans de l'éther diéthylique. On obtient des cristaux de couleur beige clair qui sont filtrés sur verre fritté, puis lavés avec de l'éther diéthylique et séchés dans l'étuve sous pression réduite (0,1 kPa) à une température voisine de 50°C. On obtient 3.28 g de dichlorhydrate de (+)-(4R)-4-(3-pyridylméthyl)-4,5-dihydro-1,3-thiazol-2-ylamine, sous forme d'un solide sableux de couleur beige clair, fondant à 124°C [Spectre de R.M.N. ¹H (300 MHz, (CD₃)₂SO d6, δ en ppm) : 3,20 (mt : 2H); 3,40 (dd, J = 11 et 5 Hz : 1H); 3,70 (dd, J = 11 et 8 Hz : 1H); 4,67 (mt : 1H); 7,97 (dd, J = 8 et 5,5 Hz : 1H); 8,46 (d large, J = 8 Hz : 1H); 8,83 (d large, J = 5,5 Hz : 1H); 8,90 (d, J = 2 Hz : 1H); 9,28 (mf : 1H); 9,75 (mf: 1H); 10,30 (mf : 1H); (α_{D}²⁰ = +18,3 +/- 0,7 dans le méthanol à 0,5%].

N-(*tert*-butyl)-N'-[(1R)-2-hydroxy-1-(3-pyridylméthyl) éthyl]thiourée : A une suspension sous atmosphère inerte de 16,51 g de dichlorhydrate de (2R)-2-amino-3-(3-pyridyl)-1-propanol dans 200 cm³ d'éthanol, on ajoute goutte à goutte 22,7 cm³ de triéthylamine, puis 14 cm³ d'isothiocyanate de *tert*-butyle. On chauffe ensuite le mélange à une température voisine de 60°C pendant 1 heure 30 min.. Après refroidissement à une température voisine de 20°C, on filtre l'insoluble sur verre fritté, puis le filtrat est évaporé sous pression réduite (2 kPa) à une température voisine de 40°C. On obtient une pâte jaune qui est reprise dans 200 cm³ d'acétate d'éthyle et 100 cm³ d'eau. La phase aqueuse est extraite puis lavée par 2 fois 200 cm³ d'acétate d'éthyle. Les phases organiques sont réunies, lavées par 3 fois 50 cm³ d'une solution saturée de chlorure de sodium, et séchées sur sulfate de magnésium. Après filtration puis évaporation sous pression réduite (2 kPa) à une température voisine de 40°C, on obtient une huile jaune qui est reprise dans le dichlorométhane et évaporée à nouveau pour donner 3,25 g de N-(*tert*-butyl)-N'-[(1R)-2-hydroxy-1-(3-pyridylméthyl) éthyl]thiourée sous forme d'une meringue jaune pâle [Spectre de R.M.N. ¹H (400 MHz, (CD₃)₂SO d6, δ en ppm) : 1,41 (s : 9H); 2,84 (mt : 2H); 3,37 (mt : 2H); 4,43 (mf : 1H); 4,96 (t, J = 5 Hz : 1H); 7,24 (d, J = 8 Hz : 1H); 7,27 (s large : 1H); 7,33 (dd, J = 8 et 5 Hz : 1H); 7,67 (dt, J = 8 et 2 Hz : 1H); 8,42 (dd, J = 5 et 2 Hz : 1H); 8,46 (d, J = 2 Hz : 1H)].

Dichlorhydrate de (2R)-2-amino-3-(3-pyridyl)-1-propanol : Une suspension de 13,55 g de N-[(1R)-2-hydroxy-1-(3-pyridylméthyl)éthyl] acétamide dans 110 cm³ d'acide chlorhydrique aqueux 6N est chauffée à une température voisine de 100°C pendant 3 heures. On évapore l'eau sous pression réduite (3 kPa) à une température voisine de 50°C. On obtient 16,51 g de dichlorhydrate de (2R)-2-amino-3-(3-pyridyl)-1-propanol, sous forme d'un solide blanc cassé.[Spectre de R.M.N. ¹H (400 MHz, (CD₃)₂SO d6, δ en ppm) : 3,13 (d, J = 7,5 Hz : 2H); 3,50 (mt : 2H); 3,62 (mt : 1H); 7,94 (dd, J = 8 et 5,5 Hz : 1H); 8,27 (mf : 3H); 8,43 (d large, J = 8 Hz : 1H); 8,79 (d large, J = 5,5 Hz : 1H); 8,86 (s large : 1H].

N-[(1R)-2-hydroxy-1-(3-pyridylméthyl)éthyl]acétamide : Une solution sous atmosphère inerte de 20,77 g (2R)-2-(acétylamino)-3-(3-pyridyl)propanoate d'éthyle dans 208 cm3 d'éthanol anhydre est refroidie à une température voisine de 10°C, puis on ajoute par fractions 8,31 g de borohydrure de sodium. Le mélange réactionnel est agité pendant 16 heures à température ambiante. On évapore l'éthanol sous pression réduite (2 kPa) à une température voisine de 40°C, et l'on obtient 43,56 g d'une pâte jaune pâle qui est reprise et agitée dans 50 cm³ d'eau. La solution, qui a un pH voisin de 12, est refroidie à une température voisine de 0°C puis neutralisée par ajout au goutte à goutte d'acide chlorhydrique concentré. On agite 1 heure à cette température puis le précipité blanc formé est filtré sur verre fritté puis séché dans l'étuve sous vide (0,1 kPa) à une température voisine de 55°C. On obtient 11,15 g de N-[(1R)-2-hydroxy-1-(3-pyridylméthyl)éthyl] acétamide sous forme d'un solide blanc fondant à une température supérieure à 260°C. Le filtrat est extrait par 3 fois 100 cm³ d'acétate d'éthyle puis les phases organiques sont réunies, séchées sur sulfate de magnésium, filtrées et évaporées sous pression réduite (2 kPa) à une température voisine de 40°C. On obtient 2.4 g de N-[(1R)-2-hydroxy-1-(3-pyridylméthyl)éthyl] acétamide partiellement complexé avec le bore sous forme de pâte blanche [Spectre de R.M.N. ¹H (300 MHz, (CD₃)₂SO d6, δ en ppm) : 1,75 (s : 3H); 2,61 (dd, J = 14 et 9 Hz : 1H); 2,88 (dd, J = 14 et 5,5 Hz : 1H); de 3,20 à 3,45 (mt : 2H); 3,91 (mt : 1H); 4,82 (t large, J = 5 Hz : 1H); 7,30 (dd, J = 7,5 et 5 Hz : 1H); 7,62 (dt, J = 7,5 et 2 Hz : 1H); 7,72 (d, J = 9 Hz : 1H); de 8,35 à 8,45 (mt : 2H)].

(2R)-2-(Acétylamino)-3-(3-pyridyl)propanoate d'éthyle : Une suspension de 54,7 g de 2-(acétylamino)-3-(3-pyridyl)propanoate d'éthyle dans 420 cm³ d'eau, à laquelle on additionne 18,5 g de chlorure de potassium, est solubilisée par ajout de 100 cm³ d'acétonitrile. On obtient une solution orange de pH neutre puis on ajoute 0,168 g d'α -chymotrypsine et le pH diminue. On additionne au goutte à goutte, en agitant, une solution aqueuse d'hydroxyde de potassium 2N pour rester à un pH constant de 7,2. Après 1 heure 30 min., le pH variant peu, le mélange est agité 48 heures à température ambiante. Le pH de la solution est alors approximativement de 5.6 puis par ajout de solution aqueuse d'hydroxyde de potassium 2N, il est ajusté à environ 7. On introduit 0,084 g d' α-chymotrypsine, et l'on ajuste le pH à 7,2 par une addition complémentaire de solution aqueuse d'hydroxyde de potassium 2N et on laisse agiter 1 heure 30 min.. Le pH du milieu réactionnel n'a pas varié. On ajoute 400 cm³ d'acétate d'éthyle, agite 30 min., puis on filtre sur célite. Le filtrat est décanté et la phase aqueuse est extraite par 3 fois 400 cm³ d'acétate d'éthyle. Les phases organiques sont réunies, lavées par 400 cm³ d'une solution saturée de chlorure de sodium, et séchées sur sulfate de magnésium. Après filtration et concentration sous pression réduite (2 kPa) à une température voisine de 40°C, on obtient 20,77 g de (2R)-2-(acétylamino)-3-(3-pyridyl)propanoate d'éthyle sous forme d'un solide beige fondant à 80°C [Spectre de R.M.N. ¹H (400 MHz, (CD₃)₂SO d6, δ en ppm) : 1,13 (t, J = 7 Hz : 3H); 1,81 (s : 3H); 2,92 (dd, J = 14 et 9,5 Hz : 1H); 3,05 (dd, J = 14 et 5,5 Hz : 1H); 4,07 (q, J = 7 Hz : 2H); 4,48 (mt : 1H); 7,33 (dd, J = 8 et 5 Hz : 1H); 7,66 (dt, J = 8 et 2 Hz : 1H); 8,36 (d, J = 8 Hz : 1H); 8,44 (mt : 2H)].

2-(Acétylamino)-3-(3-pyridyl)propanoate d'éthyle : A une solution de 51,4 g de 2-(acétylamino)-2-(3-pyridylméthyl)malonate de diéthyle dans 857 cm³ d'éthanol est additionnée au goutte à goutte 44,45 cm³ de soude aqueuse 6N. Le mélange est agité 1 heure 30 minutes. à une température voisine de 20°C, puis refroidi à une température voisine de 0°C. On coule au goutte à goutte 22,2 cm³ d'acide chlorhydrique 12N; un précipité se forme. On revient à température ambiante puis après 2 heures d'agitation le pH du milieu réactionnel est de 5-6. On concentre sous pression réduite (4 kPa) à une température voisine de 50°C pour obtenir un solide collant beige foncé que l'on sèche pendant 16 heures dans l'étuve sous vide (0,1kPa) à une température voisine de 50°C. Ce résidu est repris avec 1000 cm³ de dioxanne puis chauffé à une température voisine de 100°C pendant 1 heure. On évapore le dioxanne sous pression réduite (2 kPa) à une température voisine de 40°C et le résidu est dissout dans 250 cm³ d'eau. On neutralise par addition de 4 cm³ de soude aqueuse 10N puis on ajoute 750 cm³ d'acétate d'éthyle. Après décantation, la phase organique est extraite et la phase aqueuse est lavée par 2 fois 300 cm³ d'acétate d'éthyle. Les phases organiques sont réunies, séchées sur sulfate de magnésium, filtrées et concentrées sous pression réduite (2 kPa) à une température voisine de 40°C. Le résidu solide obtenu est repris dans 70 cm³ d'acétate d'éthyle et 20 cm³ d'heptane puis filtré sur verre fritté. On obtient 27,76 g de 2-(acétylamino)-3-(3-pyridyl)propanoate d'éthyle sous forme d'un solide beige fondant à 118°C [Spectre de R.M.N. ¹H (400 MHz, (CD₃)₂SO d6, δ en ppm) : 1,13 (t, J = 7 Hz : 3H); 1,81 (s : 3H); 2,92 (dd, J = 14 et 9 Hz : 1H); 3,04 (dd, J = 14 et 5,5 Hz : 1H); 4,07 (q, J = 7 Hz : 2H); 4,48 (mt : 1H); 7,33 (dd, J = 8 et 5 Hz : 1H); 7,66 (dt, J = 8 et 2 Hz : 1H); 8,38 (d, J = 8 Hz : 1H); 8,45 (mt : 2H)]. En extrayant à nouveau la phase aqueuse par 2 fois 500 cm³ d'acétate d'éthyle et après séchage et concentration sous pression réduite des phases organiques réunies, on récupère 4 g de 2-(acétylamino)-3-(3-pyridyl)propanoate d'éthyle sous forme d'un solide de couleur jaune-beige fondant à 121°C.

De la même façon, en partant de 50,2 g de diéthyl 2-(acétylamino)-2-(3-pyridylméthyl) malonate, on obtient 26,94 g d'éthyl 2-(acétylamino)-3-(3-pyridyl)propanoate.

2-(Acétylamino)-2-(3-pyridylméthyl)malonate de diéthyle : A 600 cm³ d'éthanol, on additionne par petites portions, en agitant sous atmosphère inerte, 17,2 g de sodium lavé préalablement 2 fois par du pentane. Après consommation totale du sodium, on ajoute rapidement 73,5 g d'acétamidomalonate de diéthyle et on laisse agiter le mélange 30 min. à une température voisine de 40°C. Une suspension de 56 g de chlorhydrate du chlorure de 3-picolyle, dans 300 cm³ d'éthanol est ensuite ajoutée rapidement et la suspension rosée résultante est agitée heures 44 heures à une température voisine de 20°C. On ajoute 28,1 g d'iodure de potassium, et l'on poursuit l'agitation à température ambiante pendant 4 heures et enfin on chauffe à une température voisine de 50°C pendant 44 heures. Le mélange est refroidi à une température voisine de 0°C, puis filtré sur verre fritté pour éliminer les sels qui sont lavés avec de l'éthanol. Le filtrat est concentré sous pression réduite (2 kPa) à une température voisine de 40°C. On obtient une pâte brune qui est dissoute dans 100 cm³ d'eau et traitée avec 7 cm³ d'une solution aqueuse d'acide chlorhydrique 3N jusqu'à obtention d'un pH voisin de 6. Le mélange est placé 48 heures au réfrigérateur. Le précipité cristallin obtenu est filtré sur verre fritté, lavé avec 50 cm³ d'eau glacée, essoré puis séché dans un dessicateur sous pression réduite (5 kPa) pendant 16 heures. Après séchage dans l'étuve sous pression réduite (0,1 kPa) à une température voisine de 40°C pendant 4 heures, on obtient 50,2 g de 2-(acétylamino)-2-(3-pyridylméthyl)malonate de diéthyle sous forme d'un solide de couleur beige foncé fondant à 96°C [Spectre de R.M.N. ¹H (400 MHz, (CD₃)₂SO d6, δ en ppm) : 1,19 (t, J = 7 Hz : 6H); 1,97 (s : 3H); 3,47 (s : 2H); 4,17 (q, J = 7 Hz : 4H); 7,33 (dd, J = 8 et 5 Hz : 1H); 7,41 (dt, J = 8 et 2 Hz : 1H); 8,21 (d, J = 2 Hz : 1H); 8,24 (s : 1H); 8,47 (dd, J = 5 et 2 Hz : 1H)].

### Exemple 2

### (+)-(4R)-4-(3-Nitrobenzyl)-4,5-dihydro-1,3-thiazol-2-ylamine, chlorhydrate

On chauffe pendant 5 heures à une température voisine de 100°C, 1,1 g de *N*-(*tert*-butyl)-*N*'-[(1R)-2-hydroxy-1-(3-nitrobenzyl)éthyl]thiourée dans 12 cm³ d'acide chlorhydrique 6N. Le milieu réactionnel est ensuite refroidi à une température voisine de 0°C; il y a formation d'un précipité blanc qui est filtré sur verre fritté puis lavé par 3 fois 20 cm³ d'éther diéthylique. Le gâteau est ensuite séché dans un dessicateur sous vide. On obtient 0,68 g de (+)-(4R)-4-(3-nitrobenzyl)-4,5-dihydro-1,3-thiazol-2-ylamine, chlorhydrate sous forme de cristaux de couleur beige fondant à 232°C [Spectre de R.M.N. ¹H (300 MHz, (CD₃)₂SO d6, δ en ppm) : 3,15 (AB limite : 2H); de 3,25 à 3,45 (mt : 1H); 3,63 (dd, J = 13 et 8 Hz : 1H); 4,62 (mt : 1H); 7,68 (t, J = 8 Hz : 1H); 7,81 (d large, J = 8 Hz : 1H); 8,18 (d large, J = 8 Hz : 1H); 8,24 (s large : 1H); de 9,00 à 10,40 (mf: 3H); (α_{D}²⁰ = +18,8 +/- 0,6 dans le méthanol à 0,5%)].

*N*-(*tert*-Butyl)-*N*'-[(1R)-2-hydroxy-1-(3-nitrobenzyl)éthyl]thiourée : Une suspension de 0,92 g de (2R)-2-amino-3-(3-nitrophényl)-1-propanol, chlorhydrate et de 0,6 cm³ de *tert*-butylisothiocyanate dans 10 cm³ d'éthanol est refroidie à une température voisine de 0°C puis est additionnée de 0,56 cm³ de triéthylamine. Le mélange est agité 18 heures à température ambiante puis est chauffé à une température voisine de 60°C pendant 2 heures; il y a alors dissolution de la suspension. On ajoute 0,6 cm³ de *tert*-butylisothiocyanate et on continue le chauffage pendant 3 heures puis on concentre le milieu réactionnel sous pression réduite (2 kPa) à une température voisine de 50°C. L'huile jaune obtenue est dissoute dans 50 cm³ d'acétate d'éthyle puis la solution organique est lavée par 2 fois 50 cm³ d'une solution de chlorure de sodium, séchée sur sulfate de sodium, filtrée et concentrée sous pression réduite (2 kPa) à une température voisine de 40°C. On obtient 1,2 g de *N*-(*tert*-butyl)-*N*'-[(1R)-2-hydroxy-1-(3-nitrobenzyl)éthyl]thiourée sous forme d'une huile jaune [Spectre de R.M.N. ¹H (300 MHz, (CD₃)₂SO d6 avec ajout de quelques gouttes de CD₃COOD d4, δ en ppm) : 1,39 (s : 9H); 2,98 (AB limite : 2H); 3,38 (AB limite : 2H); 4,48 (mt : 1H); 7,60 (t, J = 8 Hz : 1H); 7,74 (d large, J = 8 Hz : 1H); 8,09 (d large, J = 8 Hz : 1H); 8,16 (s large : 1H)].

(2R)-2-Amino-3-(3-nitrophényl)-1-propanol, chlorhydrate : Une suspension de 0,98 g N-[(1R)-2-hydroxy-1-(3-nitrobenzyl)éthyl]acétamide dans 10 cm³ d'acide chlorhydrique 6N est chauffée à une température voisine de 100°C pendant 10 heures (solubilisation). Le milieu réactionnel est concentré sous pression réduite (2 kPa) à une température voisine de 40°C. Le résidu est repris avec 20 cm³ d'éther diéthylique et évaporé à nouveau dans les mêmes conditions pour donner des cristaux beiges qui sont lavés par 3 fois 20 cm³ d'éther diéthylique puis filtrés sur verre fritté et enfin séchés sous hotte ventilée. On obtient 0,92 g de (2R)-2-amino-3-(3-nitrophényl)-1-propanol, chlorhydrate sous forme de cristaux de couleur beige fondant à 192°C [Spectre de R.M.N. ¹H (250 MHz, (CD₃)₂SO d6, δ en ppm) : 3,06 (AB limite : 2H); de 3,25 à 3,65 (mt : 3H); 5,45 (t, J = 4,5 Hz : 1H); 7,65 (t, J = 7 Hz : 1H); 7,79 (d large, J = 7 Hz : 1H); de 8,05 à 8,40 (mt : 5H)].

N-[(1R)-2-Hydroxy-1-(3-nitrobenzyl)éthyl]acétamide : Une légère suspension, sous atmosphère inerte, de 1,4 g de (2R)-2-(acétylamino)-3-(3-nitrophényl)propanoate d'éthyle dans 13 cm³ d'éthanol est refroidie à une température de 20°C puis est additionnée de 0,29 g de borohydrure de sodium. La solution jaune obtenue est agitée à température ambiante pendant 18 heures. On concentre ensuite le milieu réactionnel sous pression réduite (2 kPa) à une température voisine de 50°C, puis on ajoute 10 cm³ d'eau et on extrait par 2 fois 50 cm³ d'acétate d'éthyle. Les phases organiques réunies sont séchées sur sulfate de sodium, filtrées puis concentrées sous pression réduite (2 kPa) à une température voisine de 40°C. On obtient 1 g de N-[(1R)-2-hydroxy-1-(3-nitrobenzyl)éthyl]acétamide sous forme de cristaux de couleur jaune pâle fondant à 135°C [Spectre de R.M.N. ¹H (250 MHz, (CD₃)₂SO d6, δ en ppm) : 1,75 (s : 3H); 2,74 (dd, J = 14 et 9 Hz : 1H); 3,02 (dd, J = 14 et 5 Hz : 1H); de 3,25 à 3,45 (mt : 2H); 3,96 (mt : 1H); 4,90 (s large : 1H); 7,59 (t, J = 8 Hz : 1H); 7,70 (d large, J = 8 Hz : 1H); 7,82 (d, J = 8,5 Hz : 1H); de 8,05 à 8,15 (mt : 2H)].

(2R)-2-(Acétylamino)-3-(3-nitrophényl)propanoate d'éthyle : Un mélange de 2,8 g de 2-(acétylamino)-3-(3-nitrophényl)propanoate d'éthyle dans 17 cm³ d'acétonitrile auquel est ajouté 3,95 g d'hydrogénocarbonate d'ammonium et 35 cm³ d'eau a un pH voisin de 8. On introduit alors 0,017 g d'α-chymotrypsine type II et on laisse agiter à température ambiante pendant 1 heure 15 min. en mesurant régulièrement le pH. Après ce temps de réaction, le pH est inférieur à 8, on ajoute à nouveau 0,017 g d'α-chymotrypsine type II et on poursuit l'agitation du milieu réactionnel pendant 2 heures 45 min.. On extrait la phase aqueuse par 3 fois 50 cm³ d'acétate d'éthyle; les phases organiques réunies sont ensuite séchées sur sulfate de sodium, filtrées et évaporées sous pression réduite (2 kPa) à une température voisine de 40°C. On obtient 1,4 g de (2R)-2-(acétylamino)-3-(3-nitrophényl)propanoate d'éthyle sous forme de cristaux blancs fondant à 110°C.

2-(Acétylamino)-3-(3-nitrophényl)propanoate d'éthyle : A 12 g de 2-amino-3-(3-nitrophényl)propanoate d'éthyle à une température voisine de 0°C on ajoute au goutte à goutte 9,52 cm³ d'anhydride acétique. On laisse agiter à 0°C pendant 1 heure et le mélange prend en masse. On ajoute alors 10 cm³ d'anhydride acétique et on continue l'agitation à 0°C pendant encore 1 heure. Le précipité est filtré sur verre fritté, lavé avec 3 fois 25 cm³ d'eau, séché sous hotte ventilée puis dans l'étuve sous vide (10 Pa) à une température voisine de 50°C. On obtient 12 g de 2-(acétylamino)-3-(3-nitrophényl)propanoate d'éthyle sous forme de cristaux blancs fondant à 91°C [Spectre de R.M.N. ¹H (250 MHz, (CD₃)₂SO d6, δ en ppm) : 1,14 (t, J = 7 Hz : 3H); 1,80 (s : 3H); 3,04 (dd, J = 14 et 9 Hz : 1H); 3,18 (t, J = 14 et 4 Hz : 1H); 4,08 (q, J = 7 Hz : 2H); 4,52 (mt : 1H); 7,61 (t large, J = 8 Hz : 1H); 7,72 (d large, J = 8 Hz : 1H); 8,12 (mt : 2H); 8,42 (d, J = 8,5 Hz : 1H)].

2-Amino-3-(3-nitrophényl)propanoate d'éthyle : Dans un mélange de 23,4 g de 3-nitrophénylalanine, chlorhydrate dans 300 cm³ d'éthanol refroidi à une température voisine de 0°C, on fait buller de l'acide chlorhydrique gazeux jusqu'à saturation. La suspension obtenue est chauffée à une température voisine de 78°C pendant 18 heures : il y a dissolution à chaud de cette suspension. On arrête le chauffage, on fait buller de l'acide chlorhydrique gazeux dans la solution pendant 15 min. et on chauffe à nouveau à une température voisine de 78°C pendant 70 heures. On concentre le milieu réactionnel sous pression réduite (2 kPa) à une température voisine de 50°C puis on alcalinise le résidu à un pH de 10 environ avec une solution de carbonate de sodium. On extrait cette phase aqueuse par 3 fois 100 cm³ de dichlorométhane puis les phases organiques réunies sont séchées sur sulfate de sodium, filtrées et évaporées sous pression réduite (2 kPa) à une température voisine de 40°C pour donner 12,2 g de 2-amino-3-(3-nitrophényl)propanoate d'éthyle sous forme d'une huile orange [Spectre de R.M.N. ¹H (250 MHz, (CD₃)₂SO d6, δ en ppm) : 1,14 (t, J = 7 Hz : 3H); 1,88 (mf: 2H); 2,92 (dd, J = 13 et 7 Hz : 1H); 3,02 (dd, J = 13 et 6 Hz : 1H); 3,62 (dd, J = 7 et 6 Hz : 1H); 4,05 (q, J = 7 Hz : 2H); 7,59 (t dédoublé, J = 8,5 et 1,5 Hz : 1H); 7,71 (d large, J = 8 Hz : 1H); de 8,05 à 8,20 (mt : 2H].

3-Nitrophénylalanine, chlorhydrate : Une suspension de 34 g de 2-(acétylamino)-2-(3-nitrobenzyl)malonate de diéthyle dans 510 cm³ d'acide chlorhydrique concentré est chauffée à une température voisine de 100°C pendant 18 heures : il se produit un fort dégagement gazeux et la suspension se solubilise à chaud. Le mélange réactionnel est ensuite refroidi à une température voisine de 0°C puis agité 1 heure à 0°C et enfin filtré sur verre fritté. Le gâteau est lavé avec 2 fois 50 cm³ de filtrat, séché sous hotte ventilée puis dans l'étuve sous vide (10 Pa) à une température voisine de 60°C. On obtient 23,4 g de 3-nitrophénylalanine, chlorhydrate sous forme de cristaux blancs fondant à 222°C.

2-(Acétylamino)-2-(3-nitrobenzyl)malonate de diéthyle : A 300 cm³ d'éthanol, on additionne, en agitant sous atmosphère inerte, 3,1 g de sodium. Après consommation totale du sodium, on ajoute 29,1 g d'acétamidomalonate de diéthyle puis on chauffe à une température voisine de 78°C pendant 20 minutes. On coule alors une solution de 23 g de chlorure de 3-nitrobenzyle dans 100 cm³ d'éthanol et on poursuit le chauffage à cette même température pendant 18 heures. Le mélange réactionnel est refroidi à une température voisine de 0°C. Après agitation à froid pendant 1 heure, on filtre le précipité formé sur verre fritté, on lave le gâteau avec 2 fois 50 cm³ de filtrat puis 2 fois 50 cm³ d'eau. Après séchage sous hotte ventilée puis dans l'étuve sous vide (10 Pa) à une température voisine de 50°C, on obtient 34 g de 2-(acétylamino)-2-(3-nitrobenzyl)malonate de diéthyle sous forme de cristaux blancs fondant à 156°C.

### Exemple 3

### (+)-(4R,5S)-4-Benzyl-5-méthyl-4,5-dihydro-1,3-thiazol-2-ylamine

Une suspension de 1,8 g de la *N*-[(1R,2S)-1-benzyl-2-hydroxypropyl]-*N*'-*tert*-butylthiourée (diastéréoisomère A) dans 21 cm³ d'acide chlorhydrique 6N est chauffée à une température voisine de 100°C pendant 6 heures. On ajoute à nouveau 10 cm³ d'acide chlorhydrique 6N et on poursuit le chauffage pendant 6 heures. Le milieu réactionnel est filtré à chaud sur verre fritté, l'insoluble est lavé avec 2 fois 5 cm³ d'acide chlorhydrique 6N chaud. Le filtrat est refroidi à une température voisine de 0°C puis est additionné de 20 cm³ d'eau et enfin alcalinisé par 30 cm³ de soude à 30%. On l'extrait par 3 fois 50 cm³ de dichlorométhane. Les phases organiques réunies sont séchées sur sulfate de sodium, filtrées et concentrées sous pression réduite (2 kPa) à une température voisine de 40°C. On obtient 1 g d'huile rose que l'on purifie par chromatographie sous pression d'argon (50 kPa), sur une colonne de gel de silice (granulométrie 40-63 µm; diamètre 3 cm; hauteur 35 cm), en éluant par un mélange de dichlorométhane 90%/méthanol 10%. Les fractions contenant le produit attendu sont réunies et concentrées sous pression réduite (2 kPa) à une température voisine de 40°C. On obtient 0,31 g de (+)-(4R,5S)-4-benzyl-5-méthyl-4,5-dihydro-1,3-thiazol-2-ylamine sous forme de cristaux blancs fondant à 90°C [Spectre de R.M.N. ¹H (300 MHz, (CD₃)₂SO d6 avec ajout de quelques gouttes de CD₃COOD d4, δ en ppm) : 1,31 (d, J = 7 Hz : 3H); 2,89 (dd, J = 14 et 7,5 Hz : 1H); 3,01 (dd, J = 14 et 7,5 Hz : 1H); 3,91 (mt : 1H); 4,41 (mt : 1H); de 7,15 à 7,40 (mt : 5H). La stéréochimie relative en position 4 et 5 a été validée par effet NOE. On note une forte réponse du méthylène du benzyle après irradiation du méthyle; (α_{D}²⁰ = +29,6 +/- 0,8 dans le méthanol à 0,5%)].

*N*-[(1R,2S)-1-Benzyl-2-hydroxypropyl]-*N*'-*tert*-butylthiourée : Une solution de 12,9 g d'un mélange 75%/25% des 2 diastéréoisomères du (3R)-3-amino-4-phényl-2-butanol dans 160 cm³ d'éthanol, additionnée de 9,50 cm³ de *tert*-butylisothiocyanate est agitée à température ambiante pendant 36 heures puis chauffée 2 heures à une température voisine de 60°C. Le milieu réactionnel est concentré sous pression réduite (2 kPa) à une température voisine de 40°C pour donner 20,8 g d'une huile jaune. Cette huile est purifiée par chromatographie sous pression d'argon (50 kPa), sur une colonne de gel de silice (granulométrie 40-63 µm; diamètre 5 cm; hauteur 50 cm), en éluant par un mélange de dichlorométhane 95%/acétate d'éthyle 5%. Les fractions qui contiennent le diastéréoisomère A, sont évaporées sous pression réduite (2 kPa) à une température voisine de 40°C. On obtient 1,95 g de *N*-[(1R,2S)-1-benzyl-2-hydroxypropyl]-*N*'-*tert*-butylthiourée sous forme de cristaux jaunes pâles fondant à 128°C [Spectre de R.M.N. ¹H (250 MHz, (CD₃)₂SO d6, δ en ppm) : 0,96 (d, J = 7 Hz : 3H); 1,42 (s : 9H); 2,78 (AB limite : 2H); 3,64 (mt : 1H); 4,33 (mt : 1H); 5,02 (d, J = 4 Hz : 1H); de 7,10 à 7,40 (mt, 6H); 7,44 (s large : 1H)]. En concentrant dans les mêmes conditions les fractions correspondant au diastéréoisomère B, on obtient 6,5 g de *N*-[(1R,2R)-1-benzyl-2-hydroxypropyl]-*N*'-*tert*-butylthiourée sous forme de cristaux blancs fondant à 155°C [Spectre de R.M.N. ¹H (250 MHz, (CD₃)₂SO d6, δ en ppm) : 1,08 (d, J = 7 Hz : 3H); 1,39 (s : 9H); 2,80 (AB limite : 2H); 3,68 (mt : 1H); 4,42 (mf : 1H); 4,78 (mf : 1H); 7,08 (d, J = 8,5 Hz : 1H); de 7,10 à 7,40 (mt : 6H].

(3R)-3-Amino-4-phényl-2-butanol : Une solution de 19,3 g de *tert*-butyl (1R)-1-benzyl-2-hydroxypropylcarbamate dans 160 cm³ de dioxanne et 67 cm³ de dioxanne chlorhydrique 6,5 N est agitée à température ambiante pendant 5 heures puis est concentrée sous pression réduite (2 kPa) à une température voisine de 40°C. On obtient une huile jaune qui est reprise dans 50 cm³ d'eau et alcalinisée à un pH voisin de 10 avec du carbonate de potassium. On extrait par 3 fois 100 cm³ d'acétate d'éthyle puis les phases organiques réunies sont séchées sur sulfate de sodium, filtrées et évaporées sous pression réduite (2 kPa) à une température voisine de 40°C. On obtient 12,9 g d'un mélange 75%/25% des deux diastéréoisomères A et B du (3R)-3-amino-4-phényl-2-butanol sous forme d'une huile de couleur orange [Spectre de R.M.N. ¹H (300 MHz, (CD₃)₂SO d6, δ en ppm). On observe un mélange de diastéréoisomères: de 0,70 à 1,50 (mf étalé : 2H); 1,08 (d, J = 6 Hz : 3H); de 2,25 à 2,45 (mt : 1H); de 2,70 à 2,85 (mt : 2H); 3,44 (mt : 1H); de 4,25 à 4,75 (mf : 1H); de 7,10 à 7,35 (mt : 5H)].

*tert*-Butyl (1R)-1-benzyl-2-hydroxypropylcarbamate : Sous atmosphère inerte, une solution de 21 g de tert-butyl (1R)-1-benzyl-2-oxopropylcarbamate dans 200 cm³ d'éthanol est refroidie à une température voisine de 10°C puis est additionnée par petites fractions de 4,55 g de borohydrure de sodium. On laisse remonter la température du milieu réactionnel à l'ambiante sous agitation, puis on poursuit l'agitation pendant 18 heures. On concentre le milieu réactionnel sous pression réduite (2 kPa) à une température voisine de 40°C pour obtenir des cristaux blancs qui sont repris dans 150 cm³ d'eau. On laisse agiter 2 heures à température ambiante puis on filtre sur verre fritté. Le gâteau est lavé avec 2 fois 150cm³ d'eau. Après séchage toute une nuit sous hotte ventilée, on obtient 19,37 g de *tert*-butyl (1R)-1-benzyl-2-hydroxypropylcarbamate sous forme de cristaux de couleur blanche fondant à 127°C [Spectre de R.M.N.¹H (300 MHz, (CD₃)₂SO d6 avec ajout de quelques gouttes de CD₃COOD d4, δ en ppm). On observe un mélange de deux diastéréoisomères dans les proportions 70/30: 1,04 et 1,10 (2d, J = 6 Hz : 3H); 1,29 et 1,32 (2s : 9H); de 2,40 à 3,10 (mt : 2H); de 3,35 à 3,70 (mt : 2H); 6,40 et 6,54 (2d, J = 9 Hz : 1H); de 7,10 à 7,35 (mt : 5H)].

*tert*-Butyl (1R)-1-benzyl-2-oxopropylcarbamate : Un mélange, sous atmosphère inerte, de 26,7 g de *tert*-butyl (1R)-1-benzyl-2-[méthoxy(méthyl)amino]-2-oxoéthylcarbamate dans 534 cm³ de tétrahydrofuranne séché sur tamis 4Å est refroidi à une température voisine de 0°C. On ajoute alors, en 45 minutes, 87 cm³ d'une solution de bromure de méthylmagnésium à 3 M dans l'éther diéthylique, puis on laisse agiter 1 heure à 0°C et 18 heures à température ambiante. On refroidit à nouveau le milieu réactionnel à une température voisine de 0°C, puis on ajoute goutte à goutte 55 cm³ d'acide chlorhydrique 1N et on laisse agiter 30 minutes à cette température. Après filtration sur célite, on ajoute 200 cm³ d'eau au filtrat qui est ensuite extrait par 2 fois 200 cm³ d'acétate d'éthyle. Les phases organiques réunies sont séchées sur sulfate de sodium, filtrées puis concentrées sous pression réduite (2 kPa) à une température voisine de 40°C. On obtient 21,2 g de *tert*-butyl (1R)-1-benzyl-2-oxopropylcarbamate sous forme de cristaux de couleur jaune fondant à 59°C [Spectre de R.M.N. ¹H (300 MHz, (CD₃)₂SO d6, δ en ppm) : 1,34 (s : 9H); 2,13 (s : 3H); 2,71 (dd, J = 14 et 10 Hz : 1H); 3,01 (dd, J = 14 et 5 Hz : 1H); 4,13 (mt : 1H); de 7,10 à 7,35 (mt : 6H)].

*tert*-Butyl (1R)-1-benzyl-2-[méthoxy(méthyl)amino]-2-oxoéthylcarbamate : Une solution, sous atmosphère inerte, de 26,5 g de D-N-Boc-phénylalanine et de 22 cm³ de N-méthylmorpholine dans 300 cm³ de dichlorométhane est refroidie à une température voisine de -15°C. On coule alors 13 cm³ d'isobutylchloroformiate et on laisse agiter 15 min. à cette température. Après addition de 10,14 g de chlorhydrate de N,O-diméthylhydroxylamine, l'agitation est poursuivie pendant 1 heure à une température voisine de -15°C puis 3 heures à température ambiante. Le milieu réactionnel est additionné de 300 cm³ d'eau puis extrait par 2 fois 200 cm³ de dichlorométhane. Les phases organiques réunies sont séchées sur sulfate de sodium, filtrées et concentrées sous pression réduite (2kPa) à une température voisine de 50°C. On obtient 32,2 g d'une huile orange qui est purifiée sur une colonne de gel de silice (granulométrie 40-63 µm; hauteur 032 cm), en éluant par un mélange de dichlorométhane 97%/méthanol 3%. Après concentrat.lion des fractions contenant le produit attendu sous pression réduite (2kPa) à une température voisine de 40°C, on obtient 26,7 g de *tert*-butyl (1R)-1-benzyl-2-[méthoxy(méthyl)amino]-2-oxoéthylcarbamate sous forme d'une huile de couleur jaune [Spectre de R.M.N. ¹H (250 MHz, (CD₃)₂SO d6, δ en ppm) : 1,32 (s : 9M; 2,72 (dd, J = 13,5 et 10 Hz : 1H); 2,86 (dd, J = 13,5 et 5 Hz : 1H); 3,12 (s : 3H); 3,74 (s large : 3H); 4,55 (mt : 1H); de 7,10 à 7,35 (mt : 6H)].

### Exemple 4

### (+)-(4R,5R)-4-Benzyl-5-méthyl-4,5-dihydro-1,3-thiazol-2-ylamine

Une suspension de 6,5 g, de la *N*-[(1R,2R)-1-benzyl-2-hydroxypropyl]-*N*'-*tert*-butylthiourée (diastéréoisomère B) dans 77 cm³ d'acide chlorhydrique 6N est chauffée à une température voisine de 100°C pendant 10 heures. Le milieu réactionnel est ensuite concentré sous pression réduite (2 kPa) à une température voisine de 40°C pour donner une huile jaune qui est reprise dans 100 cm³ d'eau. Cette phase aqueuse est lavée par 2 fois 50 cm³ de dichlorométhane puis est alcalinisée à un pH voisin de 10 par ajout de 5 cm³ de soude concentrée. On l'extrait alors avec 3 fois 50 cm³ de dichlorométhane. Les phases organiques réunies sont séchées sur sulfate de sodium, filtrées puis concentrées sous pression réduite (2 kPa) à une température voisine de 40°C. On obtient 4,09 g de (+)-(4R,5R)-4-benzyl-5-méthyl-4,5-dihydro-1,3-thiazol-2-ylamine sous forme de cristaux blancs fondant à 89°C [Spectre de R.M.N. ¹H (300 MHz, (CD₃)₂SO d6, δ en ppm) : 1,22 (d, J = 7 Hz : 3H); 2,66 (dd, J = 13 et 7 Hz : 1H); 2,76 (dd, J = 13 et 7 Hz : 1H); 3,54 (mt : 1H); 3,95 (mt : 1H); de 5,60 à 6,45 (mf : 2H); de 7,10 à 7,40 (mt : 5H). La stéréochimie relative en position 4 et 5 a été validée par effet NOE. On note une forte réponse du proton H4 après irradiation du méthyle; (α_{D}²⁰ = +83,9 +/- 1,3 dans le méthanol à 0,5%)].

### Exemple 5

### (-)-(4R)-4-(3-Thiénylméthyl)-4,5-dihydro-1,3-thiazol-2-ylamine, chlorhydrate

Un mélange de 0,94 g de *N*-(*tert*-butyl)-*N*'-[(1R)-2-hydroxy-1-(3-thiénylméthyl)éthyl]thiourée dans 9,2 cm³ d'acide chlorhydrique 6N est chauffé à une température voisine de 100°C sous agitation pendant 3 heures puis est concentré sous pression réduite (2 kPa) à une température voisine de 40°C. On obtient une huile verdâtre qui est reprise dans 10 cm³ d'acétone pour donner une gomme collante. On ajoute alors 1,5 cm³ d'éthanol, puis on ajoute au goutte à goutte, en agitant, 6 cm³ d'éther diéthylique. Les cristaux obtenus sont filtrés sur verre fritté, lavés avec de l'éther diéthylique puis séchés dans l'étuve sous vide (10 Pa) à une température voisine de 50°C. On obtient 0,5 g de (-)-(4R)-4-(3-thiénylméthyl)-4,5-dihydro-1,3-thiazol-2-ylamine, chlorhydrate sous forme de cristaux de couleur grise fondant à 152°C [Spectre de R.M.N. ¹H (400 MHz, (CD₃)₂SO d6, δ en ppm) : 2,99 (mt : 2H); 3,26 (dd, J = 11 et 5,5 Hz : 1H); 3,59 (dd, J = 11 et 8 Hz : 1H); 4,54 (mt : 1H); 7,10 (dd, J = 5,5 et 1 Hz : 1H); 7,38 (mt : 1H); 7,54 (dd, J = 5 et 3 Hz : 1H); de 9,15 à 10,55 (mf étalé : 3H) ; (α_{D}²⁰ = -4,3 +/- 0,5 dans le méthanol à 0,5%].

*N*-(*tert*-butyl)-*N*'-[(1R)-2-hydroxy-1-(3-thiénylméthyl)éthyl]thiourée : Une suspension de 0,9 g de (2R)-2-amino-3-(3-thiényl)-1-propanol, chlorhydrate dans 9 cm³ d'éthanol absolu agitée sous atmosphère inerte est additionnée de 0,9 cm³ de *tert*-butylisothiocyanate puis de 0,82 cm³ de triéthylamine. Après 66 heures d'agitation à température ambiante, on évapore le milieu réactionnel sous pression réduite (2 kPa) à une température voisine de 40°C. L'huile épaisse brune obtenue est reprise dans 6 cm³ d'eau puis extraite par 2 fois 12 cm³ d'acétate d'éthyle. La phase organique est filtrée sur célite puis lavée par 2 fois 4 cm³ d'une solution aqueuse saturée de chlorure de sodium, séchée sur sulfate de magnésium et évaporée sous pression réduite (2 kPa) à une température voisine de 40°C. On obtient une huile brune qui cristallise rapidement à froid et qui est séchée dans l'étuve sous vide (10 Pa) à une température voisine de 48°C pour donner 0,97 g de *N*-(*tert*-butyl)-*N*'-[(1R)-2-hydroxy-1-(3-thiénylméthyl)éthyl]thiourée sous forme de solide de couleur beige fondant à 109°C [Spectre de R.M.N. ¹H (400 MHz, (CD₃)₂SO d6, δ en ppm) : 1,43 (s : 9H); 2,84 (mt : 2H); 3,38 (mt : 2H); 4,42 (mf : 1H); 4,86 (t large, J = 4,5 Hz : 1H); 7,04 (d large, J = 5 Hz : 1H); de 7,10 à 7,20 (mt : 2H); 7,25 (s large : 1H); 7,46 (dd, J = 5 et 3 Hz : 1H)].

(2R)-2-Amino-3-(3-thiényl)-1-propanol, chlorhydrate : Une solution de 1,4 g de *N*-[(1R)-2-hydroxy-1-(3-thiénylméthyl)éthyl]acétamide dans 17,6 cm³ d'acide chlorhydrique aqueux 6N est chauffée sous agitation à une température voisine de 100°C pendant 2 heures. Le mélange réactionnel est ensuite concentré sous pression réduite (2 kPa) à une température voisine de 40°C pour donner une huile verdâtre qui cristallise dans 60 cm³ d'acétone. Les cristaux sont filtrés sur verre fritté, lavés à l'acétone puis séchés dans l'étuve sous vide (10 Pa) à une température voisine de 50°C. On obtient 0,92 g de (2R)-2-amino-3-(3-thiényl)-1-propanol, chlorhydrate sous forme d'un solide blanc cassé [Spectre de R.M.N. ¹H (400 MHz, (CD₃)₂SO d6, δ en ppm) : 2,93 (mt : 2H); de 3,25 à 3,50 (mt : 2H); 3,53 (d large, J = 11 Hz : 1H); 5,37 (mf : 1H); 7,07 (d large, J = 5 Hz : 1H); 7,35 (mt : 1H); 7,53 (dd, J = 5 et 3 Hz : 1H); 8,18 (mf : 3H].

*N*-[(1R)-2-hydroxy-1-(3-thiénylméthyl)éthyl]acétamide : Une solution de 2,15 g de (2R)-2-(acétylamino)-3-(3-thiényl)propanoate d'éthyle dans 24 cm³ d'éthanol, agitée sous atmosphère inerte, est amenée à une température de 20°C. On ajoute 0,5 g de borohydrure de sodium puis on laisse agiter le milieu réactionnel, qui est légèrement brun, pendant 24 heures à température ambiante. On rajoute 0,2 g de borohydrure de sodium et on poursuit l'agitation pendant 24 heures. Après évaporation du mélange réactionnel sous pression réduite (2kPa) à une température voisine de 40°C, on obtient un résidu solide qui est repris dans 30 cm³ d'eau. On extrait par 2 fois 100 cm³ d'acétate d'éthyle puis la phase organique est séchée sur sulfate de magnésium et évaporée sous pression réduite (2kPa) à une température voisine de 40°C pour donner un solide blanc cassé qui est séché dans l'étuve sous vide (10Pa) à une température voisine de 48°C. On obtient 1,4 g de *N*-[(1R)-2-hydroxy-1-(3-thiénylméthyl)éthyl]acétamide sous forme d'un solide blanc cassé fondant aux environs de 105°C en devenant pâteux [Spectre de R.M.N. ¹H (400 MHz, (CD₃)₂SO d6, δ en ppm) : 1,78 (s : 3H); 2,64 (dd, J = 14 et 8 Hz : 1H); 2,87 (dd, J =14 et 5,5 Hz : 1H); de 3,20 à 3,40 (mt : 2H); 3,90 (mt : 1H); 4,76 (t, J = 5,5 Hz : 1H); 6,99 (dd, J = 5 et 1 Hz : 1H); 7,16 (dd, J = 3 et 1 Hz : 1H); 7,43 (dd, J = 5 et 3 Hz : 1H); 7,70 (d, J = 8,5 Hz : 1H].

(2R)-2-(Acétylamino)-3-(3-thiényl)propanoate d'éthyle : Une solution de 2,41 g de 2-(acétylamino)-3-(3-thiényl)propanoate d'éthyle dans 17 cm³ d'acétonitrile agitée sous atmosphère inerte est additionnée de 3,95 g d'hydrogénocarbonate d'ammonium et de 35 cm³ d'eau permutée. On introduit 0,017 g d'α-chymotrypsine type II, le pH du milieu réactionnel est alors voisin de 8. On laisse agiter 1 heure à température ambiante puis on rajoute, après avoir constaté que le pH se maintenait à 8, 0,017 g d'α -chymotrypsine type II. Au bout d'une heure d'agitation, le pH est constant, on introduit à nouveau 0,017 g d'α-chymotrypsine type II et on poursuit l'agitation du mélange réactionnel pendant 19 heures à température ambiante. On évapore l'acétonitrile sous pression réduite (2 kPa) à une température voisine de 33°C puis on ajoute à la phase aqueuse résiduelle 25 cm³ d'acétate d'éthyle. On filtre l'émulsion obtenue sur célite puis on extrait la phase aqueuse par 2 fois 25 cm³ d'acétate d'éthyle. Les phases organiques sont réunies, lavées par 2 fois 15 cm³ d'une solution de carbonate de sodium puis évaporées sous pression réduite (2kPa) à une température voisine de 40°C. Le résidu solide est ensuite séché dans l'étuve sous vide (10Pa) à température ambiante. On obtient 1,1 g de (2R)-2-(acétylamino)-3-(3-thiényl)propanoate d'éthyle sous forme d'un solide de couleur beige fondant aux environs de 75 °C.

2-(Acétylamino)-3-(3-thiényl)propanoate d'éthyle : Une solution jaune pâle de 7,7 g de 2-(acétylamino)-2-(3-thiénylméthyl)malonate de diéthyle dans 62 cm³ de diméthylformamide, agitée sous atmosphère inerte, est additionnée en une seule fois de 9,83 g d'iodure de lithium puis est chauffée dans un bain d'huile de température voisine de 128°C pendant 19 heures. On ajoute 6,6 g de iodure de lithium et on poursuit le chauffage pendant 5 heures. Le milieu réactionnel est alors évaporé sous pression réduite (2,4kPa) à une température voisine de 55°C. L'huile brune résiduelle est reprise dans 180 cm³ d'eau et est extraite par 4 fois 60 cm³ d'acétate d'éthyle. Les phases organiques sont réunies, lavées avec 30 cm³ d'une solution aqueuse saturée de chlorure de sodium, séchées sur sulfate de magnésium puis évaporées sous pression réduite (2kPa) à une température voisine de 62°C pour donner 5,34 g d'une huile brune qui cristallise à froid. Les cristaux sont repris dans 40 cm³ d'éther de pétrole, broyés dans un mortier puis filtrés sur verre fritté et séchés dans l'étuve sous vide (10Pa) à température ambiante. On obtient 4,9 g de 2-(acétylamino)-3-(3-thiényl)propanoate d'éthyle sous forme d'un solide blanc cassé fondant aux environs de 60°C [Spectre de R.M.N. ¹H (400 MHz, (CD₃)₂SO d6, δ en ppm) : 1,14 (t, J = 7 Hz : 3H); 1,84 (s : 3H); 2,93 (dd, J = 14 et 9 Hz : 1H); 3,03 (dd, J = 14 et 5,5 Hz : 1H); 4,06 (mt : 2H); 4,44 (mt : 1H); 7,02 (dd, J = 5 et 1 Hz : 1H); 7,24 (mt : 1H); 7,45 (dd, J = 5 et 3 Hz : 1H); 8,31 (d, J = 8 Hz : 1H].

2-(Acétylamino)-2-(3-thiénylméthyl)malonate de diéthyle : A 115 cm³ d'éthanol absolu, on additionne, en agitant sous atmosphère inerte, 1,54 g de sodium. Après consommation totale du sodium, on ajoute 14,55 g d'acétamidomalonate de diéthyle puis on chauffe à une température voisine de 78°C pendant 20 minutes. On coule alors une solution de 16,5 g de 3-(bromométhyl)-thiophène, fraîchement préparé, dans 50 cm³ d'éthanol et on continue le chauffage à cette même température pendant 19 heures. Le milieu réactionnel qui contient un insoluble blanc est refroidi à une température voisine de 0°C puis est filtré sur verre fritté. Le filtrat est concentré sous pression réduite (2kPa) à une température voisine de 40°C puis est filtré sur verre fritté. Les cristaux sont repris dans 100 cm³ d'eau, filtrés à nouveau, lavés avec de l'éther de pétrole et séchés sous hotte à température ambiante. On obtient 1,45 g de 2-(acétylamino)-2-(3-thiénylméthyl)malonate de diéthyle sous forme d'un solide blanc fondant aux environs de 92°C. Après évaporation à l'air ambiant du solvant de filtration, le résidu est repris dans 150 cm³ d'eau puis est filtré sur verre fritté. Le solide jaunâtre obtenu est lavé par 50 cm³ d'eau puis est dissout dans 25 cm³ d'éthanol absolu pour ensuite être cristallisé par ajout de 25 cm³ d'eau. Après filtration, lavage par 3 fois 30 cm³ d'éther de pétrole puis séchage sous hotte à température ambiante, on obtient 6,57 g de 2-(acétylamino)-2-(3-thiénylméthyl) malonate de diéthyle sous forme d'un solide de couleur blanche fondant aux environs de 92 C [Spectre de R.M.N. ¹H (300 MHz, (CD₃)₂SO d6, δ en ppm) : 1,18 (t, J = 7 Hz : 6H); 1,98 (s : 3H); 3,50 (s : 2H); 4,16 (q, J = 7 Hz : 4H); 6,80 (d large, J = 5 Hz : 1H); 7,14 (s large : 1H); 7,46 (dd, J = 5 et 3 Hz : 1H); 8,20 (s large : 1H)].

Le 3-(bromométhyl)-thiophène peut être préparé selon J. GOURIER et P. CANNONE; Bull. Soc. Chim. Fr. **1971,** 3299-3306.

### Exemple 6

### [3-(2-Amino-4,5-dihydro-thiazol-4-yl)-phényl]-(1-imino-éthyl)-amine

A une solution agitée de 0,14 g de 4-(3-aminophényl))-4,5-dihydro-1,3-thiazol-2-ylamine dans 15 cm³ d'éthanol on ajoute sous atmosphère inerte et à une température voisine de 0°C 0,15 g de chlorhydrate de benzyléthanimidothioate, puis 5 cm³ d'éthanol. Le mélange est agité pendant 1 heure à environ 0°C, puis 2 heures à une température voisine de 20°C. On rajoute 0,030 g de benzyl éthanimidothioate pour terminer la réaction, puis l'on porte le mélange à une température voisine de 45°C pendant 24 heures. Le milieu réactionnel est évaporé sous pression réduite (5 kPa) à une température voisine de 40°C. Le résidu obtenu est repris dans 30 cm³ d'eau, la solution lavée par 30 cm³ de dichlorométhane. La solution aqueuse est concentrée dans les conditions précédentes, puis le solide obtenu est trituré dans de l'éther diéthylique, filtré, séché à l'air, purifié par chromatographie à pression atmosphérique sur une colonne de gel de silice (granulométrie 40-63µ; masse 63 g) en éluant par un mélange de dichlorométhane-méthanol-ammoniaque (12/3/0,5 en volumes). On recueille les fractions contenant le produit attendu. Celles-ci sont réunies puis évaporées sous pression réduite (5 kPa) à une température voisine de 40°C. On obtient 0,100 g de [3-(2-amino-4,5-dihydro-thiazol-4-yl)-phényl]-(1-imino-éthyl)-amine, sous forme d'un solide blanc fondant à 180°C [Spectre de R.M.N. ¹H (250 MHz, (CD₃)₂SO d6 avec ajout de queques gouttes de CD₃COOD d4, δ en ppm) : 2,28 (mf: 3H); 3,18 (dd, J = 12,5 et 8,5 Hz : 1H); 3,80 (dd, J = 12,5 et 8,5 Hz : 1H); 5,30 (t, J = 8,5 Hz : 1H); 7,21 (d large, J = 8,5 Hz : 1H); 7,27 (s large : 1H); 7,36 (d large, J = 8,5 Hz : 1H); 7,49 (t large, J = 8,5 : Hz : 1H].

Le chlorhydrate de benzyléthanimidothioate peut être obtenu par application de la méthode décrite dans le brevet WO 96/19440.

4-(3-Aminophényl))-4,5-dihydro-1,3-thiazol-2-ylamine : A une suspension de 2,4 g de chlorhydrate de 4-(3-nitrophényl))-4,5-dihydro-1,3-thiazol-2-ylamine dans 50 cm³ d'acide acétique à 85% en volumes, on ajoute à une température voisine de 20°C, sous agitation, 3 g de zinc en poudre. Après 20 minutes d'agitation à température ambiante, le milieu réactionnel est filtré; le gâteau est lavé par 10 cm³ d'eau, puis le filtrat est concentré sous pression réduite (5 kPa) à une température voisine de 50°C. Le résidu obtenu est repris par du méthanol, puis de l'éther diéthylique, concentré dans les mêmes conditions que précédemment. Cette opération est répétée une seconde fois. Le produit obtenu est repris dans 100 cm³ d'acétonitrile, filtré. Le filtrat est concentré comme ci-dessus, repris par 60 cm³ d'eau. L'insoluble est filtré, tandis que le filtrat est rendu alcalin par addition de 7 cm³ d'une solution aqueuse de soude 1N.. L'insoluble est séparé par filtration, et le filtrat est à nouveau concentré comme ci-dessus, repris dans 40 cm³ d'un mélange de dichlorométhane-méthanol-ammoniaque (40/5/0,5 en volumes). L'insoluble est filtré, lefiltrat concentré comme précédemment. On obtient 4,2 g d'une huile jaune que l'on purifie par chromatographie à pression atmosphérique sur une colonne de gel de silice (granulométrie 40-63µ; 100 g de silice), en éluant par un mélange de dichlorométhane-méthanol-ammoniaque(40/5/0,5 en volumes). Les fractions contenant le produit sont réunies puis concentrées (5 kPa) à une température voisine de 40°C. On obtient 0,73 g de 4-(3-aminophényl))-4,5-dihydro-1,3-thiazol-2-ylamine, sous forme d'un solide de couleur jaune fondant à 152°C.

Chlorhydrate de 4-(3-nitrophényl))-4,5-dihydro-1,3-thiazol-2-ylamine : On procède comme dans l'exemple 2, à partir de 4,77 g de N-(*tert*-butyl)-N'-[2-hydroxy-1-(3-nitrophényl)éthyl]thiourée, 44 cm³ d'acide chlorhydrique aqueux 6N que l'on porte à une température voisine de 100°C pendant 2h30. Après un traitement identique, on obtient 3,1 g de chlorhydrate de 4-(3-nitrophényl))-4,5-dihydro-1,3-thiazol-2-ylamine, sous forme d'un solide de couleur crème fondant à 232°C.

N-(*tert*-Butyl)-N'-[2-hydroxy-1-(3-nitrophényl)éthyl]thiourée : On procède comme dans l'exemple 2 à partir de 5,1 g de 2-amino-2-(3-nitrophényl)-1-éthanol dans 170 cm³ d'éthanol additionnés de 85 cm³ de dichlorométhane et 4,6 cm³ d'isothiocyanate de *tert*-butyle à une température voisine de 20°C pendant 3 jours, puis à une température voisine de 60°C pendant 7 heures. Après une addition supplémentaire de 0,35 cm³ d'isothiocyanate de *tert*-butyle et 16 heures à une température voisine de 60°C, par un traitement identique on obtient 5,7 g de *N*-(*tert*-butyl)-*N*'-[2-hydroxy-1-(3-nitrophényl)éthyl]thiourée, sous forme d'un solide de couleur ocre fondant à 162°C.

2-Amino-2-(3-nitrophényl)-1-éthanol : On procède comme dans l'exemple 2 à partir de 1 g de 3-nitrophénylglycinate d'éthyle dans 20 cm³ d'éthanol avec 0,255 g de borohydrure de sodium et 20 heures à une température voisine de 20°C. Après un traitement identique, on obtient 0,200 g de 2-amino-2-(3-nitrophényl)-1-éthanol, sous forme d'une pâte de couleur jaune ocré (R^{f}=0,18 dans un mélange dichlorométhane-méthanol, 90/10 en volumes, sur plaque de silice Merck 60F_{254^{R}}).

3-Nitrophénylglycinate d'éthyle : 2 g de 3-nitrophénylglycine sont chauffés pendant 20 heures sous agitation à une température voisine de 100°C dans 100 cm³ d'éthanol absolu additionnés de 20 cm³ d'éthanol chlorhydrique 6,5N. La suspension est filtrée à une température voisine de 50°C; le filtrat est concentré sous pression réduite (5 kPa) à une température voisine de 40°C. Le résidu obtenu est repris par de l'eau puis extrait par environ 100 cm³ d'acétate d'éthyle. La phase aqueuse est rendue alcaline par addition de carbonate de sodium jusqu'à l'obtention d'un pH voisin de 10. Le milieu est extrait par l'acétate d'éthyle; les extraits réunis sont lavés par une solution aqueuse de chlorure de sodium, séchés sur sulfate de magnésium, puis concentrés sous pression réduite (5 kPa) à 40°C. On obtient 1 g de 3-nitrophénylglycinate d'éthyle, sous forme d'une huile de couleur jaune (R^{f}=0,38 dans un mélange dichlorométhane-méthanol, 95/5 en volumes, sur plaque de silice Merck 60F_{254^{R}}).

3-Nitrophénylglycine : A un mélange de 1,51 g de D-(-)-alpha phénylglycine dans 6 cm³ d'acide sulfurique à 95%, on ajoute lentement à une température voisine de 0°C, sous agitation, 1,01 g de nitrate de potassium, puis on laisse remonter la température aux environs de 20°C. La solution obtenue est versée sur 30 cm³ d'eau glacée puis ramenée à pH 7 par addition de 18,5 cm³ de soude 10N, à une température inférieure à 5°C. Le mélange est agité pendant 2 heures, puis filtré. Le gâteau obtenu est lavé par 2 fois 50 cm³ d'eau, puis séché. On obtient 0,60 g de 3-nitrophénylglycine, sous forme d'un solide de couleur crème et fondant à 230°C.

### Exemple 7

### Chlorhydrate de (+)-(4R)-4-benzyl-4,5-dihydro-1,3-thiazol-2-ylamine

On procède comme dans l'exemple 2 à partir de 2 g de N-(*tert*-butyl)-N'-[(1R)-2-hydroxy-1-(phénylméthyl) éthyl]thiourée, 20 cm³ d'acide chlorhydrique aqueux 6N. La réaction dure 1h30. Après refroidissement de la masse réactionnelle à une température voisine de 0°C, la solution est concentrée sous pression réduite (5 kPa) à une température voisine de 40°C. A l'huile obtenue on amorce la cristallisation par addition d'éther diéthylique. Le précipité résultant est essoré, lavé par 2 fois 10 cm³ d'éther diéthylique, puis séché sous pression réduite (10 Pa) à une température voisine de 60°C. On purifie le produit en le reprenant dans 100 cm³ de dichlorométhane sous agitation pendant 30 minutes. Après filtration de la suspension, on lave le gâteau par 2 fois 10 cm³ de dichlorométhane, puis on le sèche dans les mêmes conditions que précédemment. On obtient 1,1 g de chlorhydrate de (+)-(4R)-4-phénylméthyl-4,5-dihydro-1,3-thiazol-2-ylamine, sous forme d'un solide de couleur blanche fondant à 168°C,. (α_{D}²⁰ = +8,7 +/- 0,3 dans le méthanol à 1%).

N-(*tert*-Butyl)-N'-[(1R)-2-hydroxy-1-(benzyl) éthyl]thiourée : On procède comme dans l'exemple 2 à partir de 5 g de (R)-(+) 2-amino-3-phényl-1-propanol dans 50 cm³ d'éthanol et 5,7 g d'isothiocyanate de *tert*-butyle à une température voisine de 20°C pendant 16 heures. Après un traitement identique, on obtient 8,7 g de N-(*tert*-butyl)-N'-[(1R)-2-hydroxy-1-(phénylméthyl) éthyl]thiourée, sous forme d'un solide blanc fondant à 107°C.

### Exemple 8

### Chlorhydrate de (+)-(4R)-4-(3-carboxybenzyl)-4,5-dihydro-1,3-thiazol-2-ylamine

On procède comme dans l'exemple 2 à partir de 2,31 g de N-(*tert*-butyl)-N'-[(1R)-2-hydroxy-1-(3-carboxybenzyl) éthyl]thiourée dans 25 cm³ d'acide chlorhydrique 6N pendant 18 heures Après concentration du mélange réactionnel sous pression réduite (5 kPa) à une température voisine de 55°C, le résidu est repris par 20 cm³ d'acétone, puis à nouveau concentré dans les conditions de ci-dessus. On purifie le résidu obtenu par chromatographie sous une pression d'argon de 50 kPa, sur une colonne de gel de silice (granulométrie 40-63µ; masse silice 100 g) en éluant par un mélange chloroforme-méthanol-ammoniaque à 20% (12/6/1 en volumes) et en recueillant des fractions de 30 cm³. Les fractions 16 à 38 sont réunies puis concentrées sous pression réduite (5 kPa) à une température voisine de 40°C. Le solide obtenu est solubilisé dans 10 cm³ d'acide chlorhydrique aqueux 6N, puis concentré comme précédemment Après reprise du résidu obtenu par 10 cm³ d'acétone, l'insoluble résultant est filtré; le filtrat est concentré comme ci-dessus. On obtient un solide que l'on sèche pendant 5 heures à l'étuve à 40°C,sous pression réduite (8 Pa). On obtient 0,650 g de chlorhydrate de (+)-(4R)-4-(3-carboxybenzyl)-4,5-dihydro-1,3-thiazol-2-ylamine, sous forme d'un solide de couleur beige et fondant à 151°C, (α_{D}²⁰ = +13,4 +/- 0,6 dans le méthanol à 0,5%).

N-(*tert*-Butyl)-N'-[(1R)-2-hydroxy-1-(3-carboxybenzyl) éthyl]thiourée : On procède comme dans l'exemple 2 à partir de 1,82 g de chlorhydrate de (2R)-2-amino-3-(3-carboxyphényl)-1-propanol, dans 20 cm³ d'éthanol, 4,2 cm³ de triéthylamine et 1,7 cm³ d'isothiocyanate de *tert*-butyle. Après 21 heures de chauffage à une température voisine de 60°C et un traitement identique, on obtient 2,31 g de N-(*tert*-butyl)-N'-[(1R)-2-hydroxy-1-(3-carboxybenzyl) éthyl]thiourée sous forme d'une meringue de couleur brun clair (R^{f}=0,33 dans un mélange de chloroforme-méthanol-ammoniaque 12/6/1 en volumes, sur plaque de silice Merck 60F_{254^{R}}).

Chlorhydrate de (2R)-2-amino-3-(3-carboxyphényl)-1-propanol : On procède comme dans l'exemple 2 à partir de 1,96 g de N-[(1R)-2-hydroxy-1-(3-carboxybenzyl)éthyl]acétamide dans 14 cm3 d'acide chlorhydrique aqueux 6N chauffés à une température voisine de 100°C pendant 17 heures. Après un traitement identique on obtient 2,16 g de chlorhydrate de (2R)-2-amino-3-(3-carboxyphényl)-1-propanol, sous forme d'un solide de couleur blanche et fondant à 194°C.

N-[(1R)-2-Hydroxy-1-(3-cyanobenzyl)éthyl]acétamide : On procède comme dans l'exemple 2 à partir de 3,3 g de (2R)-2-acétylamino-3-(3-cyanophényl)propanoate d'éthyle et 1,23 g de borohydrure de sodium dans 50 cm³ d'éthanol en opérant à une température voisine de -30°C. Après 16 heures d'agitation à une température voisine de 20°C, le milieu réactionnel est évaporé sous pression réduite (5 kPa) à une température voisine de 45°C, puis le résidu obtenu est agité dans un mélange de 100 cm³ d'eau et 100 cm³ d'acétate d'éthyle. La phase organique est décantée tandis que la phase aqueuse est extraite par 2 fois 100 cm³ d'acétate d'éthyle. Les extraits organiques sont réunis puis lavés par 2 fois 50 cm³ d'eau, séchés sur sulfate de magnésium, filtrés, évaporés sous pression réduite (5 kPa) à une température voisine de 45°C. On obtient 2,13 g de N-[(1R)-2-hydroxy-1-(3-cyanobenzyl)éthyl]acétamide, sous forme d'un solide blanc, (α_{D}²⁰=+7,5 +/- 0,6 dans le méthanol à 0,5%).

(2R)-2-Acétylamino-3-(3-cyanophényl)propanoate d'éthyle : On procède comme dans l'exemple 2 à partir de 11,39 g de (2RS)-2-acétylamino-3-(3-cyanophényl)propanoate d'éthyle, 17,39 g d'hydrogénocarbonate d'ammonium, 0,1 g d'α-chymotrypsine dans 200 cm³ d'eau et 70 cm³ d'acétonitrile. Après 24 heures d'agitation à une température voisine de 20°C, et un traitement identique, on obtient 5,40 g de (2R)-2-acétylamino-3-(3-cyanophényl)propanoate d'éthyle, sous forme d'un solide de couleur beige, (R^{f}=0,76 dans un mélange de n-butanol-acide acétique-eau, 40/10/20 en volumes, sur plaque de silice Merck 60F_{254^{R}}).

(2RS)-2-Acétylamino-3-(3-cyanophényl)propanoate d'éthyle : On procède comme dans l'exemple 5 à partir de 14,9 g de 2-acétylamino-2-(3-cyanobenzyl)malonate d'éthyle, 30 g d'iodure de lithium anhydre, 150 cm³ de diméthylformamide sec. Après 5 heures d'agitation à une température voisine de 150°C, la masse réactionnelle est refroidie à température ambiante. On ajoute 1500 cm³ d'eau et l'on extrait le mélange par 3 fois 300 cm³ d'acétate d'éthyle. Les extraits sont réunis, séchés sur sulfate de magnésium, filtrés, concentrés sous pression réduite (5 kPa) à une température voisine de 45°C. Après séchage en étuve à une température voisine de 40°C sous pression réduite (10 Pa),on obtient 11,39 g de (2RS)-2-acétylamino-3-(3-cyanophényl)propanoate d'éthyle, sous forme d'une pâte de couleur beige, (R^{f}=0,51 dans un mélange d'acétate d'éthyle-cyclohexane, 90/10 en volumes, sur plaque de silice Merck 60F_{254^{R}}).

2-Acétylamino-2-(3-cyanobenzyl)malonate d'éthyle : On procède comme dans l'exemple 5 à partir de 27,7 g d'acétamidomalonate de diéthyle, 25 g de bromure de 3-cyanobenzyle, 3,2 g de sodium, dans 400 cm³ d'éthanol absolu. Après un traitement identique, la masse réactionnelle refroidie est filtrée, le gâteau lavé par 2 fois 50 cm³ d'éthanol puis par 2 fois 50 cm³ d'eau. Après séchage en étuve sous pression réduite (10 Pa) à une température voisine de 40°C, on obtient 21,98 g d'une poudre blanche. Le filtrat est évaporé sous pression réduite (5 kPa) à une température voisine de 50°C; on obtient une meringue que l'on reprend par 100 cm³ d'eau et que l'on extrait par 100 cm³ d'acétate d'éthyle puis 2 fois 50 cm³ du même solvant. Les extraits sont réunis, lavés par 2 fois 50 cm³ d'eau, séchés sur sulfate de magnésium, filtrés, évaporés sous pression réduite (5 kPa) à une température voisine de 45°C. Le résidu obtenu est repris par 20 cm³ d'éthanol. Les cristaux qui en résultent sont essorés, lavés à l'éthanol, séchés à l'étuve sous pression réduite (10 Pa) à une température voisine de 40°C. On réunit les deux jets et l'on obtient 31,94 g de 2-acétylamino-2-(3-cyanobenzyl)malonate d'éthyle, sous forme d'une poudre blanche fondant à 138°C.

### Exemple 9

### Dichlorhydrate de (+)-(4R)-4-(4-aminobutyl)-4,5-dihydro-1,3-thiazol-2-ylamine

On procède comme dans l'exemple 1 à partir de 5,9 g de (5R)-5-{[(*tert*-butylamino)carbothioyl]amino}-6-hydroxyhexylcarbamate de benzyle dans 40 cm³ d'acide chlorhydrique aqueux 6N à une température voisine de 100°C pendant 3 heures. Après concentration du milieu réactionnel sous pression réduite (5 kPa) à une température voisine de 50°C, le résidu obtenu est repris par de l'éthanol, puis concentré à nouveau dans les conditions de ci-dessus. La pâte obtenue cristallise par trituration dans un mélange d'éthanol-méthanol (8/2 en volumes). Les cristaux sont filtrés, lavés par l'acétonitrile, séchés à l'air. On obtient 1,3 g de dichlorhydrate de (+)-(4R)-4-(4-aminobutyl)-4,5-dihydro-1,3-thiazol-2-ylamine, sous forme d'un solide blanc fondant à 188°C, (α_{D}²⁰ = +16,2 +/- 0,7 dans le méthanol à 0,5%).

(5R)-5- {[(*tert*-Butylamino)carbothioyl]amino}-6-hydroxyhexylcarbamate de benzyle : A une solution agitée de 12,7 g de (2R)-6-{[(benzyloxy)carbonyl]amino}-2-{[(*tert*-butylamino)carbothioyl]amino}hexanoate d'éthyle dans 130 cm³ d'éthanol et 65 cm³ de tétrahydrofuranne, sous atmosphère inerte et refroidie entre 0 et 5°C, on ajoute 1,78 g de chlorure de lithium puis 1,6 g de borohydrure de sodium. Après 16 heures d'agitation à une température voisine de 20°C, on rajoute 0,4 g de borohydrure, et l'on porte le mélange à environ 80°C pendant 4 heures. Le milieu réactionnel est filtré, le gâteau est lavé par de l'éthanol. Le filtrat est concentré sous pression réduite (5 kPa) à une température voisine de 40°C, et le résidu obtenu est repris dans un mélange de dichlorométhane-acétate d'éthyle (85/15 en volumes), abandonné au froid à environ 5°C. Le produit cristallisé est essoré, purifié par chromatographie à pression atmosphérique sur une colonne de gel de silice (granulométrie 40-63µ; masse de silice 600 g), en éluant par un mélange de dichlorométhane-acétate d'éthyle (85/15 en volumes). On recueille les fractions contenant le produit attendu. Celles-ci sont réunies puis concentrées sous pression réduite (5 kPa) à une température voisine de 40°C. On obtient 6,9 g de (5R)-5-{[(*tert* butylamino)carbothioyl]amino}-6-hydroxyhexylcarbamate de benzyle, sous forme d'une huile incolore, (α_{D}²⁰ = +24,0 +/- 0,8 dans le méthanol à 0,5%).

(2R)-6- {[(Benzyloxy)carbonyl]amino}-2-{[(*tert*-butylamino)carbothioyl]amino} hexanoate d'éthyle : On procède comme dans l'exemple 2 à partir de 7,4 g de(2R)-2-amino-6-{[(benzyloxy)carbonyl]amino}hexanoate d'éthyle avec 5 cm³ d'isothiocyanate de *tert*-butyle dans 200 cm³ d'éthanol anhydre à une température voisine de 20°C pendant 20 heures. On complète la réaction par addition supplémentaire de 1 cm3 d'isothiocyanate et chauffage pendant 2 heures à une température voisine de 80°C. Le milieu réactionnel est concentré sous pression réduite (5 kPa) à une température voisine de 40°C. Le résidu obtenu est repris dans l'éther, concentré de nouveau comme dans les conditions précédentes. On obtient 13 g de (2R)-6-{[(benzyloxy)carbonyl]amino}-2-{[(*tert*-butylamino)carbothioyl] amino}hexanoate d'éthyle, sous forme d'une huile incolore, (α_{D}²⁰ = -12,5 +/- 0,5 dans le méthanol à 0,5%).

(2R)-2-Amino-6-{[(benzyloxy)carbonyl]amino}hexanoate d'éthyle : A une suspension de 7,9g d'acide (2R)-2-amino-6-{[(benzyloxy)carbonyl] amino} hexanoïque (Nε-CBZ-D-Lysine) dans 120 cm³ d'éthanol anhydre refroidie à une température voisine de -25°C, on ajoute sous agitation 3,9 cm³ de chlorure de thionyle. Le mélange est agité pendant 3 heures à cette température, puis on laisse revenir celle-ci au voisinage de 20°C. Après abandon pendant 3 jours, la masse réactionnelle est concentrée sous pression réduite (5 kPa) à une température voisine de 40°C. Le résidu obtenu est repris dans 200 cm³ d'acétate d'éthyle et la solution résultante est lavée par une solution aqueuse de carbonate de sodium, puis une solution aqueuse de chlorure de sodium. La solution organique est séchée sur sulfate de magnésium, filtrée, concentrée sous pression réduite (5 kPa) à une température voisine de 40°C. On obtient 7,4 g de (2R)-2-amino-6-{[(benzyloxy)carbonyl]amino}hexanoate d'éthyle, sous forme d'une huile de couleur jaune-pâle, (α_{D}²⁰ = -11,7 +/- 0,6 dans le méthanol à 0,5%).

### Exemple 10

### Chlorhydrate de (-)-(4S)-4-(3-nitrobenzyl)-4,5-dihydro-1,3-thiazol-2-ylamine

On procède comme dans l'exemple 2 à partir de 6,4 g de N-(*tert*-butyl)-N'-[(1S)-2-hydroxy-1-(3-nitrobenzyl)éthyl]thiourée dans 80 cm³ d'acide chlorhydrique aqueux 6N. Le temps de chauffage est de 7 heures. Le produit est isolé d'une façon identique, puis purifié par chromatographie sous une pression d'argon de 80 kPa, sur une colonne de gel de silice (granulométrie 40-63µ; diamètre 3,5 cm; hauteur 30 cm) en éluant par un mélange de dichlorométhane-méthanol (95/5 en volumes). On recueille les fractions correspondant au produit attendu. Celles-ci sont réunies puis concentrées sous pression réduite (5 kPa) à une température voisine de 40°C. On obtient 3,90 g de chlorhydrate de (-)-(4S)-4-(3-nitrobenzyl)-4,5-dihydro-1,3-thiazol-2-ylamine, sous forme d'un solide blanc fondant à 220°C, (α_{D}²⁰ = -18,4 +/- 0,5 dans le méthanol à 0,5%).

N-(*tert*-Butyl)-N'-[(1S)-2-hydroxy-1-(3-nitrobenzyl)éthyl]thiourée : On procède comme dans l'exemple 2 à partir de 5,65 g de chlorhydrate de (2S)-2-amino-3-(3-nitrophényl)-1-propanol, 3,7 cm³ d'isothiocyanate de *tert*-butyle, 3,41 cm³ de triéthylamine dans 60 cm³ d'éthanol. Après 3 jours d'agitation à température voisine de 20°C puis chauffage 30 minutes à une température voisine de 60°C, on ajoute au milieu réactionnel 3,7 cm³ d'isothiocyanate de *tert*-butyle, puis on poursuit le chauffage encore 3 heures à la même température. Après un traitement identique, on obtient 6,45 g de N-(*tert*-butyl)-N'-[(1S)-2-hydroxy-1-(3-nitrobenzyl)éthyl]thiourée, sous forme d'une meringue de couleur jaune, (R^{f}=0,61 dans un mélange de dichlorométhane-méthanol-ammoniaque 40/5/0,5 en volumes, sur plaque de silice Merck 60F_{254^{R}}).

Chlorhydrate de (2S)-2-amino-3-(3-nitrophényl)-1-propanol : On procède comme dans l'exemple 2 à partir de 5,8 g de N-[(1S)-2-hydroxy-1-(3-nitrobenzyl)éthyl]acétamide dans 60 cm³ d'acide chlorhydrique aqueux 6N pendant 10 heures à une température voisine de 100°C. Après refroidissement du milieu, le précipité est filtré puis lavé par 30 cm³ d'acétone, 3 fois 50 cm³ d'éther, séché à l'étuve sous pression réduite (10 Pa) à une température voisine de 60°C. Le filtrat est concentré sous pression réduite (5 kPa) à environ 50°C. Le résidu obtenu est repris par 30 cm³ d'éther diéthylique, les cristaux filtrés, lavés par 2 fois 30 cm³ d'éther, séchés à l'étuve dans les mêmes conditions que ci-dessus. Les 2 jets cristallisés sont réunis. On obtient 5,65 g de chlorhydrate de (2S)-2-amino-3-(3-nitrophényl)-1-propanol, sous forme d'un solide beige fondant à 188°C.

N-[(1S)-2-Hydroxy-1-(3-nitrobenzyl)éthyl]acétamide : On procède comme dans l'exemple 2 à partir de 7,15 g de (2S)-2-(acétylamino)-3-(3-nitrophényl)propanoate d'éthyle, 1,46 g de borohydrure de sodium dans 70 cm³ d'éthanol. Par un traitement identique on obtient 5,8 g de N-[(1S)-2-hydroxy-1-(3-nitrobenzyl)éthyl]acétamide, sous forme d'un solide blanc fondant à 131°C.

Le (2S)-2-(acétylamino)-3-(3-nitrophényl)propanoate d'éthyle peut être préparé selon RIVIER et col. J. Med. Chem. (**1995**), 2658.

### Exemple 11

### (-)-(4S,5S)-4-Benzyl-5-méthyl-4,5-dihydro-1,3-thiazol-2-ylamine

On procède comme dans l'exemple 3 à partir de 3,9 g du diastéréoisomère A de la N-(*tert*-butyl)-N'-((1S)-1-benzyl-2-hydroxypropyl)thiourée dans 46 cm³ d'acide chlorhydrique aqueux 6N. Après chauffage 5 heures à une température voisine de 100°C, la masse réactionnelle est concentrée sous pression réduite (5 kPa) à une température voisine de 50°C. L'huile obtenue est dissoute dans 50 cm³ d'eau; la solution est lavée par 2 fois 50 cm³ de dichlorométhane. La phase aqueuse est rendue alcaline par addition de 3 cm³ de lessive de soude à 30%, puis extraite par 3 fois 50 cm³ de dichlorométhane. Les extraits organiques sont réunis et séchés sur sulfate de sodium. Après filtration et concentration sous pression réduite (5 kPa) à une température voisine de 40°C, on obtient 2,5 g de (-)-(4S,5S)-4-benzyl-5-méthyl-4,5-dihydro-1,3-thiazol-2-ylamine, sous forme d'un solide blanc fondant à 89°C, (isomère trans), (α_{D}²⁰ = -87,3 +/- 1,3 dans le méthanol à 0,5%).

N-(*tert*-Butyl)-N'-((1S)-1-benzyl-2-hydroxypropyl)thiourée : On procède comme dans l'exemple 3 à partir de 9,5 g de (3S)-3-amino-4-phényl-2-butanol dans 80 cm³ d'éthanol et en présence de 7,86 cm³ d'isothiocyanate de *tert*-butyle. Après 3 jours d'agitation du mélange à une température voisine de 20°C, et traitement identique, on obtient 15,9 g d'une huile que l'on purifie par chromatographie sous pression d'argon (50 kPa), sur une colonne de gel de silice (granulométrie 40-63µ; diamètre 5 cm; hauteur silice 38 cm), en éluant par un mélange de dichlorométhane-acétate d'éthyle (95/5 en volumes). Les fractions qui contiennent le diastéréoisomère A sont réunies et évaporées sous pression réduite (5 kPa) à une température voisine de 40°C. On obtient 3,98 g du diastéréoisomère A de la N-(*tert*-butyl)-N'-((1S)-1-benzyl-2-hydroxypropyl)thiourée, sous forme d'un solide blanc fondant à 156°C. En concentrant dans les mêmes conditions les fractions correspondant au diastéréoisomère B, on obtient 0,72 g du diastéréoisomère B de la N-(*tert*-butyl)-N'-((1S)-1-benzyl-2-hydroxypropyl)thiourée, sous forme d'un solide jaune fondant à 116°C.

(3S)-3-Amino-4-phényl-2-butanol : On procède comme dans l'exemple 3 à partir de 17,6 g de (1S)-1-benzyl-2-hydroxypropylcarbamate de *tert*-butyle dans 145 cm³ de dioxanne et 61 cm³ de dioxanne chlorhydrique 6,5N, en agitant pendant 16 heures à une température voisine de 20°C. Par un traitement identique on obtient 9,56 g de (3S)-3-amino-4-phényl-2-butanol, sous forme d'une huile de couleur orange qui est un mélange des deux diastéréoisomères (80/20),. (R^{f}=0,25 dans un mélange de dichlorométhane-méthanol-ammoniaque 40/5/0,5 en volumes, sur plaque de silice Merck 60F_{254^{R}}).

(1S)-1-Benzyl-2-hydroxypropylcarbamate de *tert*-butyle : On procède comme dans l'exemple 3 à partir de 2,66 g de(1S)-1-benzyl-2-oxopropylcarbamate de *tert*-butyle dans 25 cm³ d'éthanol, avec 0,58 g de borohydrure de sodium. Après 4h30 à une température voisine de 20°C, le milieu réactionnel est traité de manière identique. On obtient 2,7 g de (1S)-1-benzyl-2-hydroxypropylcarbamate de *tert*-butyle, sous forme d'un solide blanc fondant à 125°C, mélange des deux diastéréoisomères (70/30).

(1S)-1-Benzyl-2-oxopropylcarbamate de *tert*-butyle : On procède comme dans l'exemple 3 à partir de 5 g de (1S)-1-benzyl-2-[méthoxy(méthyl)amino]-2-oxoéthylcarbamate de *tert*-butyle, 16 cm³ d'une solution éthérée 1M de bromure de méthylmagnésium, et 100 cm³ de tétrahydrofuranne anhydre. La réaction terminée, le milieu réactionnel est agité pendant 18 heures à une température voisine de 20°C. Après un traitement identique, on obtient 3,91 g d'une huile orange que l'on purifie par chromatographie à pression atmosphérique sur une colonne de gel de silice (granulométrie 63-200µ; diamètre 3,5 cm; hauteur 20 cm) en éluant par du dichlorométhane. On recueille les fractions correspondant au produit attendu. Celles-ci sont réunies puis concentrées sous pression réduite (5 kPa) à une température voisine de 40°C. On obtient 2,82 g de (1S)-1-benzyl-2-oxopropylcarbamate de *tert*-butyle, sous forme d'un solide blanc fondant à 62°C.

(1S)-1-Benzyl-2-[méthoxy(méthyl)amino]-2-oxoéthylcarbamate de *tert*-butyle : On procède comme dans l'exemple 3 à partir de 26,5 g de L-N-Boc-phénylalanine, 22 cm³ de N-méthylmorpholine, 13 cm³ de chloroformiate d'isobutyle, 10,14 g de chlorhydrate de N,O-diméthylhydroxylamine dans 300 cm³ de dichlorométhane. Le produit est traité d'une façon identique, puis purifié par chromatographie à pression atmosphérique sur une colonne de gel de silice (ganulométrie 63-200µ; diamètre 6 cm; hauteur 25 cm) en éluant par un mélange de dichlorométhane-méthanol (97/3 en volumes). On recueille les fractions contenant le produit attendu. Celles-ci sont réunies puis concentrées sous pression réduite (5 kPa) à une température voisine de 40°C. On obtient 17,1 g de (1S)-1-benzyl-2-[méthoxy(méthyl)amino]-2-oxoéthylcarbamate de *tert*-butyle, sous forme d'une huile incolore, (R^{f}=0,65 dans un mélange de dichlorométhane-méthanol 97/3 en volumes, sur plaque de silice Merck 60F_{254^{R}}).

### Exemple 12

### Dichlorhydrate de (-)-(4S)-4-(4-aminobutyl)-4,5-dihydro-1,3-thiazol-2-ylamine

On procède comme dans l'exemple 9 à partir de 0,59 g de (2S)-6-{[(benzyloxy)carbonyl]amino}-2-{[(*tert*-butylamino)carbothioyl]amino}hexanoate d'éthyle dans 6 cm³ d'acide chlorhydrique 6N. Après concentration du milieu réactionnel dans les mêmes conditions, la meringue obtenue est reprise 3 fois par de l'éther diéthylique que l'on décante à chaque fois puis l'insoluble restant par 10 cm³ d'acétonitrile. Le produit cristallisé résultant est essoré, lavé par de l'acétonitrile chaud, puis séché sous pression réduite (5 kPa) à une température voisine de 20°C. On obtient 0,34 g de dichlorhydrate de (-)-(4S)-4-(4-aminobutyl)-4,5-dihydro-1,3-thiazol-2-ylamine, sous forme de cristaux de couleur crème fondant à 170°C, (α_{D}²⁰ = -15,5 +/- 0,6 dans le méthanol à 0,5%).

(2S)-6-{[(Benzyloxy)carbonyl]amino}-2-{[(*tert*-butylamino)carbothioyl]amino} exanoate d'éthyle : On procède comme dans l'exemple 9 à partir de 0,42 g de (5S)-5-amino-6-hydroxyhexylcarbamate de benzyle et 0,24 cm³ d'isothiocyanate de *tert*-butyle dans 30 cm³ d'éthanol à une température voisine de 20°C pendant 3 jours. On termine la réaction par addition supplémentaire de 0,48 cm³ d'isothiocyanate suivie d'un chauffage pendant 1 heure à une température voisine de 80°C. Après un traitement identique, on obtient 0,6 g de (2S)-6-{[(benzyloxy)carbonyl]amino}-2-{[(*tert*-butylamino)carbothioyl]amino}hexanoate d'éthyle, sous forme d'une huile de couleur beige ayant une tendance à la cristallisation,. (R^{f}=0,62 dans un mélange de dichlorométhane-méthanol 90/10 en volumes, sur plaque de silice Merck 60F_{254^{R}}).

(5S)-5-Amino-6-hydroxyhexylcarbamate de benzyle : Un mélange de 4 g de (5S)-5-{[(*tert*-butyloxy)carbonyl]amino}-6-hydroxyhexylcarbamate de benzyle, 30 cm³ d'acide trifluoroacétique et 20 cm³ d'éthanol est agité à une température voisine de 20°C pendant 2 heures. Après concentration du milieu réactionnel sous pression réduite (5 kPa) à une température voisine de 40°C, le résidu obtenu est repris par 50 cm³ d'eau, lavé par 2 fois 100 cm³ d'éther diéthylique. La phase aqueuse est décantée, alcalinisée par du carbonate de sodium jusqu'à pH 9-10, puis extraite par 3 fois 100 cm³ de dichlorométhane. Les extraits réunis sont lavés par une solution aqueuse de chlorure de sodium, puis séchés sur sulfate de magnésium, filtrés, concentrés sous pression réduite (5 kPa) à une température voisine de 40°C. On obtient 2,0 g de (5S)-5-amino-6-hydroxyhexylcarbamate de benzyle, sous forme d'un solide blanc fondant à 82°C.

(5S)-5-{[(*tert*-Butyloxy)carbonyl]amino}-6-hydroxyhexylcarbamate de benzyle : On procède comme dans l'exemple 9 à partir de 7 g de (2S)-6-{[(benzyloxy)carbonyl]amino}-2-{[(*tert*-butyloxy)carbonyl]amino}hexanoate de méthyle dans 70 cm³ d'éthanol, 35 cm³ de tétrahydrofuranne, 1 g de chlorure de lithium et 0,81 g de borohydrure de sodium. Après 16 heures à une température voisine de 20°C, la masse réactionnelle est filtrée, le gâteau lavé par de l'éthanol. Le filtrat résultant est concentré sous pression réduite (5 kPa) à une température voisine de 40°C. Après dissolution du résidu obtenu dans 150 cm³ de dichlorométhane, lavage par 2 fois 100 cm³ d'une solution aqueuse de chlorure de sodium et 2 fois 100 cm³ d'eau, séchage sur sulfate de magnésium, et enfin concentration dans les mêmes conditions que ci-dessus, on obtient 5,2 g de (5S)-5-{[(*tert*-butyloxy)carbonyl]amino}-6-hydroxyhexylcarbamate de benzyle, sous forme d'un solide blanc fondant à 67°C.

(2S)-6-{[(Benzyloxy)carbonyl]amino}-2-{[(*tert*-butyloxy)carbonyl]amino}hexanoate de méthyle : A une solution de 6,6 g de chlorhydrate de l'ester méthylique de la Nε-CBZ-L-Lysine dans 66 cm³ de méthanol et 66 cm³ de tétrahydrofuranne, refroidie à une température voisine de 0°C, on ajoute, sous atmosphère inerte et sous agitation 4,8 g de di-*tert*-butyldicarbonate, puis 5,7 cm³ de triéthylamine et 10 cm³ de méthanol. Après agitation du mélange à une température voisine de 5°C pendant 2 heures puis 2 heures à environ 20°C; le milieu réactionnel est concentré sous pression réduite (5 kPa) à une température voisine de 40°C. Le résidu obtenu est repris dans le dichlorométhane, la solution lavée par 100 cm³ d'eau, séchée sur sulfate de magnésium, filtrée, concentrée dans les mêmes conditions que ci-dessus. On obtient 7 g de (2S)-6-{[(benzyloxy)carbonyl]amino}-2-{[(*tert*-butyloxy)carbonyl]amino} hexanoate de méthyle, sous forme d'une huile de couleur crème,. (R^{f}=0,90 dans un mélange de dichlorométhane-méthanol 90/10 en volumes, sur plaque de silice Merck 60F_{254^{R}}).

### Exemple 13

### (4S,5R)-4-Benzyl-5-méthyl-4,5-dihydro-1,3-thiazol-2-ylamine

On procède comme dans l'exemple 3, en partant de 0,7 g du diastéréoisomère B de la N-(*tert*-butyl)-N'-((1S)-1-benzyl-2-hydroxypropyl)thiourée que l'on chauffe à une température voisine de 100°C pendant 5 heures dans 8,3 cm³ d'acide chlorhydrique aqueux 6N. Après concentration du milieu réactionnel sous pression réduite (5 kPa) à une température voisine de 50°C, on ajoute au résidu obtenu 50 cm³ d'eau, puis on extrait la solution par 3 fois 25 cm³ de dichlorométhane. La phase aqueuse est rendue alcaline par addition de 0,5 cm³ de lessive de soude à 30%, extraite par 3 fois 50 cm³ de dichlorométhane. Les extraits sont réunis, séchés sur sulfate de sodium, filtrés, puis concentrés sous pression réduite (5 kPa) à une température voisine de 50°C . On obtient une huile que l'on purifie par chromatographie sous une pression d'argon de 50 kPa, sur une colonne de gel de silice (granulométrie 40-63µ; diamètre 2 cm; hauteur 28 cm) en éluant par un mélange de dichlorométhane-méthanol (95/5 en volumes). On recueille les fractions contenant le produit attendu. Celles-ci sont réunies puis concentrées sous pression réduite (5 kPa) à une température voisine de 40°C. On obtient 0,10 g de (4S)-4-benzyl-5-méthyl-4,5-dihydro-1,3-thiazol-2-ylamine, sous forme d'un solide de couleur rose pâle fondant à 90°C en devenant pâteux (mélange de diastéréoisomères : 82% de cis [(4S,5R)-4-Benzyl-5-méthyl-4,5-dihydro-1,3-thiazol-2-ylamine]-18% de trans [(4S,5S)-4-Benzyl-5-méthyl-4,5-dihydro-1,3-thiazol-2-ylamine]), (R^{f}=0,15 dans un mélange de dichlorométhane-méthanol 95/5 en volumes, sur plaque de silice Merck 60F_{254^{R}}).

### Exemple 14

### Oxalate de (-)-(4R)-4-butyl-4,5-dihydro-1,3-thiazol-2-ylamine

On procède comme dans l'exemple 1 à partir de 2,3 g de N-(*tert*-butyl)-N'-[(1R)-1-butyl-2-hydroxyéthyl]thiourée, dans 32 cm³ d'acide chlorhydrique aqueux 6N. Après concentration sous pression réduite (5 kPa) à une température voisine de 50°C, le résidu obtenu est repris par 10 cm³ d'eau puis rendu alcalin par addition de 2 cm³ de lessive de soude à 30%. Après extraction par 3 fois 50 cm³ de dichlorométhane, les extraits organiques sont réunis puis séchés sur sulfate de sodium, concentrés sous pression réduite (5 kPa) à une température voisine de 40°C. On obtient 1,37 g d'une huile jaune dont on fait l'oxalate de la façon suivante : à partir de 0,3 g d'huile de ci-dessus dans 1 cm³ d'acétone, on ajoute 0,24 g d'acide oxalique dissout dans 1 cm³ d'acétone. Après précipitation du sel, le milieu est dilué par 5 cm³ d'acétone, filtré. Les cristaux sont lavés par 2 fois 4 cm³ d'acétone, puis séchés à l'étuve sous pression réduite (10 Pa) à une température voisine de 40°C. On obtient 0,26 g d'oxalate de (-)-(4R)-4-butyl-4,5-dihydro-1,3-thiazol-2-ylamine, sous forme d'un solide blanc fondant à 91°C ; (α_{D}²⁰ = -7 sous lampe Na 589 nm et α_{D}²⁰ =-70,1 +/- 1,2 sous lampe Hg 365 nm, dans le méthanol à 0,5%).

N-(*tert*-Butyl)-N'-[(1R)-1-butyl-2-hydroxyéthyl]thiourée : On procède comme dans l'exemple 2 à partir de 1,14 g de (2R)-2-amino-2-butyl-1-éthanol, 1,36 cm³ d'isothiocyanate de *tert*-butyle dans 15 cm³ d'éthanol, en agitant 18 heures à une température voisine de 20°C, puis 3 heures au voisinage de 60°C. Après concentration du milieu réactionnel sous pression réduite (5 kPa) à une température voisine de 40°C. On obtient 2,38 g de N-(*tert*-butyl)-N'-[(1R)-1-butyl-2-hydroxyéthyl]thiourée, sous forme d'une huile jaune [Spectre infra rouge (CH₂Cl₂) :3620; 4430; 4410; 2960; 1560; 1500; 1395; 1375 et 1205 cm⁻¹].

(2R)-2-Amino-hexanol : 3,7 g de chlorhydrate de l'ester méthylique de la L-Norleucine sont solubilisés dans 20 cm³ d'eau. A cette solution, on ajoute sous agitation à une température voisine de 20°C la quantité nécessaire d'une solution aqueuse de carbonate de sodium pour obtenir un pH de 10. Le milieu est extrait par 3 fois 50 cm³ d'acétate d'éthyle. Les extraits sont réunis, séchés sur sulfate de sodium, filtrés, concentrés sous pression réduite (5 kPa) à une température voisine de 50°C. On obtient 2,95 g de (2R)-2-amino-2-butyl-acétate de méthyle, sous forme d'une huile jaune. Puis on procède comme dans l'exemple 2, à partir de 2,9 g de (2R)-2-amino-2-butyl-acétate de méthyle, 1,14 g de borohydrure de sodium dans 50 cm³ d'éthanol. La réaction est effectuée à une température voisine de -15°C, puis le milieu réactionnel est agité pendant 18 heures à une température voisine de 20°C, et enfin 1h30 à une température voisine de 80°C. Après concentration de la masse réactionnelle sous pression réduite (5 kPa) à une température voisine de 50°C, le résidu obtenu est purifié par chromatographie sous pression d'argon (50 kPa) sur une colonne de gel de silice (granulométrie 40-63µ; diamètre 2 cm; hauteur 20 cm), en éluant par un mélange d'acétate d'éthyle-méthanol (80/20 en volumes). Les fractions contenant le produit attendu sont réunies puis concentrées sous pression réduite (5 kPa) à une température voisine de 40°C. On obtient 1,2 g de (2R)-2-amino-hexanol, sous forme d'une huile ayant tendance à cristalliser ;. (α_{D}²⁰ = +2,6 +/- 0,4 dans le méthanol à 0,5%).

### Exemple 15

### Chlorhydrate de (+)-(5S)-5-méthyl-4,5-dihydro-1,3-thiazol-2-ylamine

On procède comme dans l'exemple 1 à partir de 11,86 g de N-[(2S)-2-hydroxypropyl]-N'-*tert*-butylthiourée dans 168 cm³ d'acide chlorhydrique aqueux 6N. Après 3 heures de chauffage à une température voisine de 100°C et un traitement identique, on obtient 2,30 g de chlorhydrate de (+)-(5S)-5-méthyl-4,5-dihydro-1,3-thiazol-2-ylamine, sous forme d'un solide blanc fondant à 173°C ;. (α_{D}²⁰ = +73,8 +/-1,4 dans le méthanol à 0,5%).

N-[(2S)-2-Hydroxypropyl]-N'-*tert*-butylthiourée : On procède comme dans l'exemple 1 à partir de 5 g de (+)-(S)-1-amino-2-propanol, 8,43 g d'isothiocyanate de *tert*-butyle dans 60 cm³ d'éthanol. Après agitation du milieu réactionnel pendant 5 heures à une température voisine de 20°C, puis concentration sous pression réduite (5 kPa) à une température voisine de 50°C, on reprend le solide blanc obtenu par de l'éther diéthylique. La suspension est à nouveau concentrée dans les mêmes conditions que précédemment. On obtient 11,86 g de N-[(2S)-2-hydroxypropyl]-N'-*tert*-butylthiourée, sous forme d'un solide blanc fondant à 106°C.

### Exemple 16

### (-)-(4S)-4-Cyclohexylméthyl-4,5-dihydro-1,3-thiazol-2-ylamine

On procède comme dans l'exemple 1 à partir de 11,1 g de N-(*tert*-butyl)-N'-[(1S)-1-cyclohexylméthyl-2-hydroxyéthyl]thiourée dans 110 cm³ d'acide chlorhydrique aqueux 6N portés à une température voisine de 100°C pendant 2 heures. Par un traitement identique, on obtient 3,6 g de (-)-(4S)-4-cyclohexylméthyl-4,5-dihydro-1,3-thiazol-2-ylamine, sous forme d'un solide blanc fondant à 169°C ; (α_{D}²⁰ = -33,9 +/-0,8 dans le méthanol à 0,5%).

N-(*tert*-Butyl)-N'-[(1S)-1-cyclohexylméthyl-2-hydroxyéthyl]thiourée : On procède comme dans l'exemple 3 à partir de 8,1 g de (+)-(2S)-2-amino-3-cyclohexyl-1-propanol et de 9,8 cm³ d'isothiocyanate de *tert*-butyle dans 79 cm³ d'éthanol. Le milieu réactionnel est agité pendant 72 heures à une température voisine de 20°C . Après concentration sous pression réduite (5 kPa) à une température voisine de 50°C de la masse réactionnelle, l'huile obtenue est reprise par 110 cm³ d'éther de pétrole froid. Les cristaux sont filtrés, séchés sous pression réduite (10 Pa) à une température voisine de 60°C. On obtient 11,1 g de N-(*tert*-butyl)-N'-[(1S)-1-cyclohexylméthyl-2-hydroxyéthyl]thiourée, sous forme d'un solide blanc fondant à 97°C.

### Exemple 17

### Chlorhydrate de (+)-(4R)-4-cyclohexylméthyl-4,5-dihydro-1,3-thiazol-2-ylamine

On procède comme dans l'exemple 1 à partir de 2,9 g N-(*tert*-butyl)-N'-[(1R)-1-cyclohexylméthyl-2-hydroxyéthyl]thiourée dans 28 cm³ d'acide chlorhydrique aqueux 6N par chauffage à une température voisine de 100°C pendant 2h15. Par traitement identique, on obtient un solide que l'on purifie par dissolution dans 15 cm³ d'eau et que l'on extrait par 10 cm³ de dichlorométhane. La phase aqueuse est rendue alcaline par addition de 10 cm³ de soude 1N, extraite par 2 fois 15 cm³ d'acétate d'éthyle. Les extraits organiques réunis sont concentrés sous pression réduite (5 kPa) à une température voisine de 40°C. La pâte résultante est reprise par 2 fois 20 cm³ d'éther diéthylique. Le solide est filtré, séché à l'étuve sous pression réduite (10 Pa) à une température voisine de 60°C. On obtient 0,4 g de chlorhydrate de (+)-(4R)-4-cyclohexylméthyl-4,5-dihydro-1,3-thiazol-2-ylamine, sous forme d'un solide blanc fondant à 169°C ; (α_{D}²⁰ = +31,8 +/- 0,9 dans le méthanol à 0,5%).

N-(*tert*-Butyl)-N'-[(1R)-1-cyclohexylméthyl-2-hydroxyéthyl]thiourée : On procède comme dans l'exemple 3 à partir de 4,89 g de (2R)-2-amino-3-cyclohexyl-1-propanol avec 5,9 cm³ d'isothiocyanate de *tert*-butyle dans 48 cm³ d'éthanol absolu. Après 5 jours d'agitation à une température voisine de 20°C, le milieu réactionnel est concentré sous pression réduite (5 kPa) à une température voisine de 40°C. Le solide obtenu est repris par 200 cm³ d'éther de pétrole, puis l'insoluble est filtré, séché à l'air. On obtient 2,96 g de N-(*tert*-butyl)-N'-[(1R)-1-cyclohexylméthyl-2-hydroxyéthyl]thiourée, sous forme d'un solide de couleur blanche (R^{f}=0,29 dans le mélange acétate d'éthyle-cyclohexane, sur plaque de silice Merck 60F_{254^{R}}).

(2R)-2-Amino-3-cyclohexyl-1-propanol : On procède comme dans l'exemple 1 à partir de 6,2 g de (2R)-2-amino-3-cyclohexylpropionate d'éthyle et 1,93 g de borohydrure de sodium dans 120 cm³ d'éthanol absolu à environ 5°C pendant 10 minutes. Le milieu est ramené à température ambiante, agité encore pendant 3h30minutes. Après un nouveau refroidissement à environ 5°C et une nouvelle addition de 40,94 g de borohydrure, puis agitation à une température voisine de 20°C pendant 65 heures, le milieu réactionnel est concentré sous pression réduite (5 kpa) à une température voisine de 40°C. On obtient un solide que l'on purifie par chromatographie sous pression d'argon (100 kPa), sur une colonne de gel de silice (granulométrie 40-63µ; 320 g), en éluant par du méthanol pur. On recueille les fractions contenant le produit attendu. Celles-ci sont réunies puis concentrées sous pression réduite (5 kPa) à une température voisine de 40°C. On obtient 5,16 g de (2R)-2-amino-3-cyclohexyl-1-propanol, sous forme d'un solide blanc collant (R^{f}=0,25 dans le méthanol sur plaque de silice Merck 60F_{254^{R}}).

(2R)-2-Amino-3-cyclohexylpropionate d'éthyle : On procède comme dans l'exemple 2 à partir de 10 g de chlorhydrate de β-cyclohexyl-(D)-alanine dans 96 cm³ d'éthanol absolu en faisant passer un courant d'acide chlorhydrique sec à une température voisine de 0°C pendant 20 minutes. Après chauffage à environ 80°C pendant 4 jours, puis refroidissement à 0°C, on refait passer un courant d'acide chlorhydrique pendant 20 minutes suivi d'un chauffage à une température voisine de 80°C pendant 20 heures. Le milieu réactionnel est concentré sous pression réduite (5 kPa) à une température voisine de 40°C, tandis que le résidu obtenu est solubilisé dans 200 cm³ d'eau, rendu alcalin par addition de 19 g de carbonate de potassium solide. Après évaporation, le solide résultant est repris par 200 cm³ d'éthanol.à une température voisine de 80°C. L'insoluble est filtré, le filtrat est évaporé sous pression réduite (5 kPa) à une température voisine de 40°C. Le résidu obtenu est repris dans 130 cm³ d'éthanol à température ambiante. L'insoluble est filtré tandis que le nouveau filtrat est évaporé dans les mêmes conditions que ci-dessus. On obtient 4,8 g de (2R)-2-amino-3-cyclohexylpropionate d'éthyle, sous forme d'une pâte blanchâtre (R^{f}=0,80 dans le mélange éthanol-ammoniaque, 90/10 en volumes, sur plaque de silice Merck 60F_{254^{R}}).

### Exemple 18

### 4-(3-Nitrophényl)-4,5-dihydro-1,3-thiazol-2-ylamine

On procède comme dans l'exemple 2 à partir de 1,49 g de N-(*tert*-butyl)-N'-[2-hydroxy-1-(3-nitrophényl)éthyl]thiourée dans 14 cm³ d'acide chlorhydrique aqueux 6N par chauffage à une température voisine de 100°C pendant 2h30. Par un traitement identique, on obtient 1,01 g de 4-(3-nitrophényl)-4,5-dihydro-1,3-thiazol-2-ylamine, sous forme d'un solide de couleur crème fondant à 232°C.

### Exemple 19

### Dichlorhydrate de (+)-(4R)-4-(4-pyridylméthyl)-4,5-dihydro-1,3-thiazol-2-ylamine

On procède dans les conditions de l'exemple 1 à partir de 0,46 g de N-(*tert*-butyl)-N'-[(1R)-2-hydroxy-1-(4-pyridylméthyl)éthyl]thiourée que l'on chauffe dans 5 cm³ d'acide chlorhydrique aqueux 5N pendant 16 heures à une température voisine de 100°C. Après concentration de la masse réactionnelle sous pression réduite (5 kPa) à une température voisine de 40°C, on obtient une huile que l'on reprend dans 3 cm³ de propanol-2. Le précipité cristallisé qui en résulte est essoré, lavé par du propanol-2, séché à l'air. On obtient 0,1 g de chlorhydrate de (+)-(4R)-4-(4-pyridylméthyl)-4,5-dihydro-1,3-thiazol-2-ylamine, sous forme d'un solide orangé fondant à 150°C (α_{D}²⁰ = +38,3 +/- 0,9 dans MeOH à 0,5%).

N-(*tert*-Butyl)-N'-[(1R)-2-hydroxy-1-(4-pyridylméthyl)éthyl]thiourée : Une solution de 0,1 g de (1R)-1-(4-pyridylméthyl)-2-hydroxyéthylcarbamate de *tert*-butyle dans 3 cm³ de dioxanne chlorhydrique 4N est agitée pendant 16 heures à une température voisine de 20°C. La masse réactionnelle est concentrée sous pression réduite (5 kPa) à une température voisine de 40°C. On obtient 0,075 g d'un produit blanc que l'on solubilise dans 4 cm³ d'éthanol. Après addition de 0,08cm³ de triéthylamine puis 0,09 cm³ d'isothiocyanate de *tert*-butyle, le mélange est agité pendant 16 heures à une température voisine de 20°C, puis 6 heures à une température voisine de 50°C. Le mélange est évaporé sous pression réduite (5 kPa) à une température voisine de 40°C, puis le résidu obtenu est trituré dans 20 cm³ d'eau. L'eau est décantée, l'huile résultante est additionnée d'éthanol, puis concentrée dans les conditions de ci-dessus. On obtient 0,10 g de N-(*tert*-butyl)-N'-[(1R)-2-hydroxy-1-(4-pyridylméthyl)éthyl]thiourée, sous forme d'une huile de couleur orangée (R^{f}=0,50 dans le mélange acétate d'éthyle-acide acétique-eau, 4/1/1 en volumes, sur plaque de silice Merck 60F_{254^{R}}).

(1R)-1-(4-Pyridylméthyl)-2-hydroxyéthylcarbamate de *tert*-butyle : A un mélange agité de 1 g de Boc-D-4-pyridylalanine dans 10 cm³ de tétrahydrofuranne, on ajoute sous atmosphère inerte 0,52 cm³ de triéthylamine, puis l'on refroidit le mélange à une température voisine de -18°C. Après addition de 0,47 cm³ de chloroformiate d'isobutyle, l'agitation est poursuivie pendant 30 minutes à une température comprise entre -18 et -10°C. Le mélange est rapidement filtré puis on ajoute au filtrat 0,28 g de borohydrure de sodium préalablement solubilisé dans 2 cm³ d'eau. Le mélange est agité pendant 1 heure à une température voisine de 0°C, puis pendant 16 heures à une température voisine de 20°C. Le mélange est rendu alcalin par addition d'une solution aqueuse de carbonate de potassium, puis agité en présence d'acétate d'éthyle. La phase organique est décantée puis lavée à l'eau, séchée sur sulfate de magnésium, filtrée, concentrée sous pression réduite (5 kPa) à une température voisine de 40°C. Le résidu obtenu est purifié par chromatographie sous une pression d'argon de 50 kPa sur une colonne de gel de silice (granulométrie 40-63µ; diamètre 2,2 cm; hauteur silice 30 cm) en éluant d'abord par de l'acétate d'éthyle seul, et en recueillant des fractions de 30 cm³. Les fractions 1 à 18 sont séparées, puis on élue par un mélange d'acétate d'éthyle-méthanol (90/10 en volumes). Les fractions 23 à 26 sont recueillies puis réunies. Après concentration sous pression réduite (5 kPa) à une température voisine de 40°C, on obtient 0,10 g de (1R)-1-(4-pyridylméthyl)-2-hydroxyéthylcarbamate de *tert*-butyle, sous forme d'une huile [Spectre infra rouge (CH₂Cl₂) : 3618; 3434; 2981; 1708; 1501; 1367; 1168 et 1057 cm⁻¹].

### Exemple 20

### Dichlorhydrate de (+)-(4R,5R)-5-méthyl-(4R,5R)-4-(pyridin-4-yl-méthyl)-4,5-dihydro-thiazol-2 ylamine

Une suspension de 1,96 g de *N*-*tert*-butyl-*N'*-[(1R,2RS)-1-(pyridin-4-yl-méthyl)-2-hydroxy-propyl]-thiourée dans 25 cm³ d'acide chlorhydrique 5N est chauffée à une température voisine de 100°C pendant 18 heures. Le milieu réactionnel est concentré sous pression réduite (2 kPa) à une température voisine de 40°C. Le résidu obtenu est purifié par chromatographie sur une colonne CHIRALCEL OD 20µ dans un mélange heptane-isopropanol-triéthylamine (90/10/0,1 en volumes). Les fractions contenant le produit attendu sont concentrées sous pression réduite (1 kPa), à une température voisine de 40°C. Le résidu obtenu est purifié par chromatographie sous pression atmosphérique sur une colonne de gel de silice (granulométrie 40-60 µm ; diamètre 1,2 cm ; hauteur 30 cm), en éluant par un mélange d'acétate d'éthyle-acide acétique-eau (2/1/1 en volumes). Les fractions contenant le produit attendu sont réunies et concentrées sous pression réduite (2 kPa) à une température voisine de 40°C. Le résidu obtenu est repris par 5 cm³ d'acide chlorhydrique 5 N et de l'éthanol puis concentré sous pression réduite (2 kPa) à une température voisine de 40°C. Après séchage au dessiccateur sous pression réduite (0,1 kPa) à une température voisine de 40°C on obtient 0,4 g de dichlorhydrate de (+)(4R,5R)-4-(pyridin-4-yl-méthyl)-5-méthyl-4,5-dihydro-thiazol-2-ylamine sous forme d'une meringue très hygroscopique [Spectre de R.M.N. ¹H (300 MHz, (CD₃)₂SO d6, δ en ppm) : 1,44 (d, J = 7 Hz : 3H) ; 3,22 (dd, J =13,5 et 7,5 Hz : 1H) ; 3,32 (dd, J = 13,5 et 5 Hz : 1H) ; 3,90 (mt : 1H) ; 4,37 (mt : 1H) ; 8,01 (d large, J = 6 Hz : 2H) ; 8,88 (d large, J = 6 Hz : 2H) ; 9,34 (s large: 1H) ; 9,85 (s large: 1H) ; 10,31 (mf: 1H); (a_{D}²⁰= +75,3 +/- 1,3 dans le méthanol à 0,5%).

*N*'-*tert*-Butyl-*N*'-[(1R,2RS)-1-(pyridin-4-yl-méthyl)-2-hydroxy-propyl]-thiourée : Une solution de 1,41 g de (3R,2RS)-3-amino-4-(pyridin-4-yl)-butan-2-ol dans 30 cm³ d'éthanol, additionnée de 1,08 cm³ de *tert*-butylisothiocyanate puis de 2,16 cm³ de triéthylamine est chauffée 16 heures sous agitation à une température voisine de 50°C. Le milieu réactionnel est concentré sous pression réduite (2 kPa) à une température voisine de 40°C, repris par 10 cm³ d'eau et lavé avec 2 fois 30 cm³ de dichlorométhane, séché sur sulfate de magnésium, filtré, évaporé dans les conditions de ci-dessus puis séché au dessiccateur sous pression réduite (0,1 kPa) à une température voisine de 40°C. On obtient 2 g de *N-tert*-butyl-*N'*-[(1R,2S)-1-(pyridin-4-yl-méthyl)-2-hydroxy-propyl]-thiourée de sous forme d'une huile orangée [Spectre de R.M.N. ¹H (300 MHz, (CD₃)₂SO d6, δ en ppm). Nous observons le mélange de deux diastéréoisomères A et B dans les proportions respectives 70/30, δ = 0,99 (d, J = 6,5 Hz : 0,9H) ; 1,09 (d, J = 6,5 Hz : 2,1H) ; 1,37 (s : 6,3H) ; 1,41 (s : 2,7H) ; 2,74 (dd, J = 14,5 et 9 Hz : 0,7H) ; de 2,75 à 2,90 (mt : 0,6H) ; 2,93 (dd, J = 14,5 et 5 Hz : 0,7H) ; de 3,55 à 3,80 (mt : 1H) ; de 4,35 à 4,60 (mf: 1H) ; 4,65 (mf : 0,3H) ; 4,88 (mf: 0,7H) ; de 7,10 à 7,25 (mt : 1H) ; 7,17 (s large : 0,7H) ; 7,26 (d large, J = 6 Hz : 1,4H) ; 7,31 (d large, J = 6 Hz : 0,6H) ; 7,44 (s large : 0,3H) ; de 8,35 à 8,50 (mt : 2H).

Dichlorhydrate de (3R,2RS)-3-amino-4-(pyridin-4-yl)-butan-2-ol : A une solution de 1,85 g de N-[(1R,2RS)-1-(pyridin-4-yl-méthyl)-2-hydroxy-propyl]-carbamate de *tert*-butyle dans 20 cm³ de dioxanne on ajoute sous agitation, à une température voisine de 20°C, une solution de 20 cm³ d'acide chlorhydrique 4N dans le dioxanne. Le mélange est agité à une température voisine de 20°C pendant 5 heures, puis il est concentré sous pression réduite (2 kPa) à une température voisine de 40°C. On obtient 1,8 g de dichlorhydrate de (3R,2RS)-3-amino-4-(pyridin-4-yl)-butan-2-ol sous forme d'un solide jaune [Spectre de R.M.N. ¹H (300 MHz, (CD₃)₂SO d6 avec ajout de quelques gouttes de CD₃COOD d4, δ en ppm). Nous observons le mélange de deux diastéréoisomères A et B dans les proportions respectives 70/30 ; δ = 1,18 et 1,20 (2 d, J = 6,5 Hz : 3H en totalité) ; 3,13 (dd, J = 10,5 et 6 Hz : 0,7H) ; de 3,20 à 3,35 (mt : 1,3H) ; de 3,45 à 3,65 (mt : 0,6 H) ; 3,72 (mt : 0,7H) ; 3,96 (mt : 0,7H) ; de 8,05 à 8,15 (mt : 2H) ; 8,88 (d large, J = 6 Hz : 2H]).

N-[(1R,2RS)-1-(Pyridin-4-yl-méthyl)-2-hydroxy-propyl]-carbamate de *tert*-butyle : A une solution de 2 g de N-[(1R)-1-(pyridin-4-yl-méthyl)-2-oxo-propyl]-carbamate de *tert* butyle dans 20 cm³ d'éthanol, sous agitation et à une température voisine de 10°C on ajoute 0,43 g de borohydrure de sodium puis, on agite à une température voisine de 20°C pendant 18 heures. Le milieu réactionnel est concentré sous pression réduite (1 kPa), à une température voisine de 40°C, repris par 50 cm³ d'eau, extrait par 100 cm³ d'acétate d'éthyle, séché sur sulfate de magnésium, filtré et concentré sous pression réduite (1 kPa), à une température voisine de 40°C. On obtient 1,83 g de N-[(1R,2RS)-1-(pyridin-4-yl-méthyl)-2-hydroxy-propyl]-carbamate de *tert*-butyle sous forme d'un solide jaune [Spectre de R.M.N. ¹H (300 MHz, (CD₃)₂SO d6, δ en ppm). Nous observons le mélange de deux diastéréoisomères A et B dans les proportions respectives 70/30 ; δ = 1,03 (d, J = 6,5 Hz : 0,9H) ; 1,09 (d, J = 6,5 Hz : 2,1H) ; 1,28 (s : 2,7H) ; 1,30 (s : 6,3H) ; de 2,45 à 2,55 (mt : 0,7H) ; 2,61 (dd, J = 13,5 et 10,5 Hz : 0,3H) ; 2,81 (dd, J = 13,5 et 4 Hz : 0,3H) ; 3,01 (dd, J = 13,5 et 2 Hz : 0,7H) ; de 3,40 à 3,70 (mt : 2H) ; 4,70 (d, J = 5,5 Hz : 0,3H) ; 4,78 (d, J = 5,5 Hz : 0,7H) ; 6,53 (d, J = 9 Hz : 0,3H) ; 6,67 (d, J = 9 Hz : 0,7H) ; 7,20 (d large, J = 6 Hz : 1,4H) ; 7,25 (d large, J = 6 Hz : 0,6H) ; de 8,40 à 8,50 (mt : 2H)].

N-[(1R)-1-(Pyridin-4-yl-méthyl)-2-oxo-propyl]-carbamate de *tert*-butyle : Un mélange, sous atmosphère inerte, de 5,3 g de N-{2-[N-méthoxy-N-(méthyl)amino]-2-oxo-(1R)-1-(pyridin-4-yl-méthyl)-éthyl}-carbamate de *tert*-butyle dans 120 cm³ de tétrahydrofuranne est refroidi à une température voisine de 0°C. On ajoute alors, en 1 heure, 17 cm³ d'une solution de bromure de méthylmagnésium à 3 M dans l'éther diéthylique, puis on laisse agiter 1 heure à 0°C et 18 heures à une température voisine de 20°C. On refroidit à nouveau le milieu réactionnel à une température voisine de 0°C, puis on ajoute goutte à goutte 30 cm³ d'acide chlorhydrique 1N et 100 cm³ d'eau, on extrait avec de l'acétate d'éthyle et on lave avec 2 fois 100 cm³ d'eau. Les phases organiques réunies sont séchées sur sulfate de sodium, filtrées puis concentrées sous pression réduite (2 kPa) à une température voisine de 40°C. Le résidu obtenu est purifié par chromatographie à pression atmosphérique sur une colonne de gel de silice (granulométrie 60-200 µm ; diamètre 4 cm ; hauteur 35 cm), en éluant par de l'acétate d'éthyle. Les fractions contenant le produit attendu sont réunies et concentrées sous pression réduite (2 kPa) à une température voisine de 40°C. On obtient 0,2 g de N-[(1R,2RS)-1-(pyridin-4-yl-méthyl)-2-oxo-propyl]-carbamate de *tert*-butyle [Spectre de R.M.N. ¹H (300 MHz, (CD₃)₂SO d6, δ en ppm) : 1,34 (s : 9H) ; 2,16 (s : 3H) ; 2,71 (dd, J = 14 et 10,5 Hz : 1H) ; 3,08 (dd, J = 14 et 4,5 Hz : 1H) ; 4,21 (mt : 1H) ; 7,27 (d large, J = 5,5 Hz : 2H) ; 7,34 (d, J = 8 Hz : 1H) ; 8,47 (d large, J = 5,5 Hz : 2H)].

N-{2-[N-Méthoxy-N-(méthyl)amino]-2-oxo-(1R)-1-(pyridin-4-yl-méthyl)-éthyl}-carbamate de *tert*-butyle : Une solution, sous atmosphère inerte, de 20 g de D-N-Boc-pyridylalanine et de 16,5cm³ de N-méthylmorpholine dans 500 cm³ de dichlorométhane est refroidie à une température voisine de -15°C. On coule alors 9,75 cm³ de chloroformiate d'isobutyle et on laisse agiter 15 minutes à cette température. Après addition de 7,61 g de chlorhydrate de N,O-diméthylhydroxyl amine, l'agitation est poursuivie pendant 1 heure à une température voisine de -15°C puis 18 heures à température ambiante. Le milieu réactionnel est additionné de 250 cm³ d'eau puis extrait par 100 cm³ de dichlorométhane. La phase organique est séchée sur sulfate de sodium, filtrée et concentrée sous pression réduite (2kPa) à une température voisine de 50°C. Le résidu est purifié sur une colonne de gel de silice (granulométrie 60-200 µm ; hauteur 35 cm), en éluant avec de l'acétate d'éthyle. Les fractions contenant le produit attendu sont réunies et concentrées sous pression réduite (2 kPa) à une température voisine de 40°C. On obtient 6,5 g de N-{2-[N-méthoxy-N-(méthyl)amino]-2-oxo-(1R)-1-(pyridin-4-yl-méthyl)-éthyl}-carbamate de *tert*-butyle sous forme d'une huile jaune épaisse [Spectre de R.M.N. ¹H (300 MHz, (CD₃)₂SO d6, δ en ppm) : 1,33 (s : 9H) ; 2,78 (dd, J = 13,5 et 10 Hz : 1H) ; 2,89 (dd, J = 13,5 et 4,5 Hz : 1H) ; 3,14 (s large : 3H) ; 3,76 (s large : 3H) ; 4,63 (mt : 1H) ; 7,24 (mt : 1H) ; 7,27 (d large, J = 5,5 Hz : 2H) ; 8,48 (d large, J = 5,5 Hz : 2H)].

### Exemples 21 et 22

### Dichlorhydrate de (+)-4-(thiazol-5-yl-méthyl)-4,5-dihydro-thiazol-2-ylamine et dichlorhydrate de (-)-4-(thiazol-5-yl-méthyl)-4,5-dihydro-thiazol-2-ylamine

Un mélange racémique de 0,38 g de dichlorhydrate de (4RS)-4-(thiazol-5-yl-méthyl)-4,5-dihydro-thiazol-2-ylamine est séparé sur une colonne CHIRALCEL OD10µ dans un mélange heptane-isopropanol-2-triéthylamine (80/20/0,1 en volumes). Les fractions contenant le produit attendu sont concentrées sous pression réduite (1 kPa), à une température voisine de 40°C pour obtenir 0,053 g de dichlorhydrate de (+)-4-(thiazol-5-ylméthyl)-4,5-dihydro-thiazol-2-ylamine et 0,057 g de dichlorhydrate de (-)-4-(thiazol-5-yl-méthyl)-4,5-dihydro-thiazol-2-ylamine.

Dichlorhydrate de (+)-4-(thiazol-5-yl-méthyl)-4,5-dihydro-thiazol-2-ylamine : Spectre de R.M.N. ¹H (300 MHz, (CD₃)₂SO d6, δ en ppm) : de 3,20 à 3,40 (mt : 3H) ; 3,70 (dd, J = 11,5 et 8 Hz : 1H) ; 4,58 (mt : 1H) ; 7,85 (s : 1H) ; 9,10 (s : 1H) ; 9,26 (mf: 1H) ; 9,67 (mf: 1H) ; 10,13 (s large : 1H).

Dichlorhydrate de (-)-4-(thiazol-5-yl-méthyl)-4,5-dihydro-thiazol-2-ylamine : Spectre de R.M.N. ¹H (300 MHz, (CD₃)₂SO d6, δ en ppm) : de 3,20 à 3,40 (mt : 3H) ; 3,70 (dd, J = 12 et 8 Hz : 1H) ; 4,58 (mt : 1H) ; 7,85 (s : 1H) ; 9,10 (s : 1H) ; 9,26 (mf: 1H) ; 9,67 (mf: 1H) ; 10,14 (s large : 1H) ; (α_{D}²⁰= -4,2 +/- 0,6 dans le méthanol à 0,5%)

Dichlorhydrate de (4RS)-4-(thiazol-5-yl-méthyl)-4,5-dihydro-thiazol-2-ylamine : Une solution de 0,56 g de N-(*tert*-butyl)-N'-[(1RS)-1-hydroxyméthyl-2-(thiazol-5-yl)-éthyl]-thiourée dans 5,5 cm³ d'acide chlorhydrique 6N est chauffée à une température voisine de 110°C pendant 3 heures. Le milieu réactionnel est concentré sous pression réduite (1 kPa), à une température voisine de 60°C, repris par 4 cm³ d'éthanol et 6 cm³ d'éther diéthylique et évaporé dans les mêmes conditions que ci-dessus. Le résidu est repris par 6 cm³ d'éthanol, filtré et le solide est séché au dessiccateur sous pression réduite (0,1 kPa) à une température voisine de 40°C puis dissout dans une solution de 5,5 cm³ d'acide chlorhydrique 6N et porté à reflux à une température voisine de 110°C pendant 9 heures. Le milieu réactionnel est concentré sous pression réduite (1 kPa), à une température voisine de 40°C et séché au dessiccateur sous pression réduite (0,1 kPa) à une température voisine de 40°C pour obtenir 0,54 g de dichlorhydrate de (4RS)-4-(thiazol-5-yl-méthyl)-4,5-dihydro-thiazol-2-ylamine sous forme d'un solide pâteux [Spectre de R.M.N. ¹H (300 MHz, (CD₃)₂SO d6, δ en ppm) : de 3,20 à 3,40 (mt : 3H) ; de 3,60 à 3,80 (mt : 1H) ; 4,57 (mt : 1H) ; 7,85 (s : 1H) ; 9,10 (s : 1H) ; 9,26 (mf : 1H) ; 9,66 (mf : 1H) ; 10,13 (s large : 1H].

N-(*tert*-Butyl)-N'-[(1RS)-1-hydroxyméthyl-2-(thiazol-5-yl)-éthyl]-thiourée : A une solution de 0,56 g de chlorhydrate de (2RS)-2-amino-3-(thiazol-5-yl)-propan-1-ol dans 8 cm³ d'éthanol on ajoute 0,52 cm³ de triéthylamine puis 0,55 cm³ de *tert*-butylisothiocyanate. Le mélange est agité à une température voisine de 20°C pendant 18 heures puis chauffé à une température voisine de 60°C pendant 1 heure 30. Après refroidissement le milieu réactionnel est concentré sous pression réduite (1 kPa), à une température voisine de 40°C, repris par 6 cm³ d'eau et lavé par 3 fois 20 cm³ de dichlorométhane, séché sur sulfate de magnésium, filtré, évaporé dans les conditions de ci-dessus puis séché au dessiccateur sous pression réduite (0,1 kPa) à une température voisine de 40°C . On obtient 0,56 g de N'-(*tert*-butyl)-N-[(1RS)-1-hydroxyméthyl-2-(thiazol-5-yl)-éthyl]-thiourée sous forme d'une huile visqueuse jaune [Spectre de R.M.N. ¹H (300 MHz, (CD₃)₂SO d6, δ en ppm) : 1,43 (s : 9H) ; 3,03 (dd, J = 15 et 7 Hz : 1H) ; 3,19 (dd, J = 15 et 7 Hz : 1H) ; de 3,30 à 3,50 (mt : 2H) ; 4,41 (mt : 1H) ; 4,97 (t, J = 5 Hz : 1H) ; 7,23 (d, J = 8 Hz : 1H) ; 7,30 (s large :1H) ; 7,67 (s : 1H) ; 8,94 (s : 1H)].

Chlorhydrate de (2RS)-2-amino-3-(thiazol-5-yl)-propan-1-ol : Une solution de 0,63 g de N-[(1RS)-1-hydroxyméthyl-2-(thiazol-5-yl)-éthyl]-acétamide dans 7,86 cm³ d'acide chlorhydrique 6N est chauffée à une température voisine de 110°C pendant 3 heures. Le mélange réactionnel est filtré, concentré sous pression réduite (1 kPa) à une température voisine de 40°C, repris dans l'éther isopropylique, concentré dans les conditions ci-dessus, repris par 10 cm³ d'éther isopropylique, filtré et séché au dessiccateur sous pression réduite (0,1 kPa) à une température voisine de 40°C. On obtient 0,56 g de chlorhydrate de (2RS)-2-amino-3-(thiazol-5-yl)-propan-1-ol sous forme d'un solide beige fondant à 192°C [Spectre de R.M.N. ¹H (300 MHz, (CD₃)₂SO d6, δ en ppm) : 3,21 (d, J = 7 Hz : 2H) ; 3,35 (mt : 1H) ; 3,48 (dd, J = 11,5 et 5,5 Hz : 1H) ; 3,61 (dd, J = 11,5 et 4 Hz : 1H) ; 7,83 (s large : 1H) ; 8,19 (mf : 3H) ; 9,10 (s large : 1H].

N-[(1RS)-1-Hydroxyméthyl-2-(thiazol-5-yl)-éthyl]-acétamide : A une solution de 1,2 g de (2RS)-2-acétylamino-3-(thiazol-5-yl)-propanoate d'éthyle dans 20 cm³ d'éthanol on ajoute 0,4 g de borohydrure de sodium puis, sous atmosphère inerte, on agite à une température voisine de 20°C. Au bout de 18 heures, on ajoute à nouveau 0,1 g de borohydrure de sodium puis on agite 24 heures à une température voisine de 20°C. Le milieu réactionnel est concentré sous pression réduite (1 kPa), à une température voisine de 40°C. Le résidu est repris par 10 cm³ d'eau et extrait par 3 fois 30 cm³ d'acétate d'éthyle. La phase organique est séchée sur sulfate de magnésium, filtrée et concentrée sous pression réduite (1 kPa), à une température voisine de 40°C. Le résidu est chromatographié sous une pression d'argon de 60 kPa, sur une colonne de gel de silice (granulométrie 40-63µ ; diamètre 3,5 cm ; hauteur 31 cm), en éluant par 100 cm³ de dichlorométhane, 600 cm³ d'un mélange de dichlorométhane-méthanol (98/2 en volumes), 500 cm³ d'un mélange de dichlorométhane-méthanol (96/4 en volumes) et 1 dm³ d'un mélange de dichlorométhane-méthanol (90/10 en volumes). Les fractions contenant le produit attendu sont réunies puis concentrées dans les conditions ci-dessus. On obtient 0,63 g de N-[(1RS)-1-hydroxyméthyl-2-(thiazol-5-yl)-éthyl]-acétamide sous forme d'une huile jaune [Spectre de R.M.N. ¹H (300 MHz, (CD₃)₂SO d6, δ en ppm) : 1,80 (s : 3H) ; 2,85 (dd, J = 15 et 9 Hz : 1H) ; 3,16 (dd, J = 15 et 5 Hz : 1H) ; de 3,25 à 3,45 (mt : 2H) ; 3,87 (mt : 1H) ; 4,85 (mt : 1H) ; 7,65 (s : 1H) ; 7,79 (d, J = 8,5 Hz : 1H) ; 8,91 (s : 1H)].

(2RS)-2-Acétylamino-3-(thiazol-5-yl)-propanoate d'éthyle : A une solution de 3,3 g de 2-acétylamino-2-(thiazol-5-yl-méthyl)-malonate de diéthyle dans 70 cm³ d'éthanol sont additionnés goutte à goutte 2,7 cm³ de soude 6N. Le mélange est agité 5 heures puis on coule goutte à goutte 1,5 cm³ d'acide chlorhydrique 12N ; un précipité se forme. On concentre sous pression réduite (1 kPa) à une température voisine de 40°C pour obtenir un solide brun que l'on sèche 12 heures au dessiccateur sous pression réduite (0,1 kPa) à une température voisine de 40°C. Ce résidu est repris avec 50 cm³ de dioxanne puis chauffé à une température voisine de 100°C pendant 3 heures 30. Le milieu réactionnel est évaporé sous pression réduite (1 kPa), à une température voisine de 40°C. Le résidu est chromatographié sous une pression d'argon de 60 kPa, sur une colonne de gel de silice (granulométrie 40-63µ ; diamètre 3,5 cm ; hauteur 31 cm), en éluant par 675 cm³ de dichlorométhane, 500 cm³ d'un mélange de dichlorométhane-méthanol (99/1 en volumes), 500 cm³ d'un mélange de dichlorométhane-méthanol (98/02 en volumes), 500 cm³ d'un mélange de dichlorométhane-méthanol (97/3 en volume), 500 cm³ d'un mélange de dichlorométhane-méthanol (95/5 en volumes), 500 cm³ d'un mélange de dichlorométhane-méthanol-ammoniaque (12/1,5/0,5 en volumes) et 500 cm³ d'un mélange de dichlorométhane-méthanol-ammoniaque (12/3/0,5 en volumes). Les fractions contenant le produit attendu sont réunies puis concentrées dans les conditions ci-dessus. On obtient 0,8 g de (2RS)-2-acétylamino-3-(thiazol-5-yl)-propanoate d'éthyle sous forme d'huile marron [Spectre de masse : DCI m/z=243 MH⁺]

2-Acétylamino-2-(thiazol-5-yl-méthyl)-malonate de diéthyle : Après dissolution de 1,24 g de sodium dans 20 cm³ d'éthanol on ajoute 10,86 cm³ d'acétamido-malonate de diéthyle puis on chauffe à une température voisine de 75°C. Au bout de 15 minutes, on ajoute une solution de 5-chlorométhyl-thiazole dans 20 cm³ d'éthanol, puis on chauffe pendant 2 heures à une température voisine de 75°C. Le milieu réactionnel est évaporé sous pression réduite (1 kPa), à une température voisine de 40°C. Le résidu est chromatographié sous une pression d'argon de 60 kPa, sur une colonne de gel de silice (granulométrie 60-200µ ; diamètre 6,5 cm ; hauteur 40 cm), en éluant par un mélange de dichlorométhane-méthanol (99/1 en volumes), un mélange de dichlorométhane-méthanol (98/02 en volumes) puis un mélange de dichlorométhane-méthanol (95/5 en volumes). Les fractions contenant le produit attendu sont réunies puis concentrées dans les conditions ci-dessus. On obtient 3,3 g de 2-acétylamino-2-(thiazol-5-yl-méthyl)-malonate de diéthyle sous forme d'un solide orange [Spectre de R.M.N. ¹H (300 MHz, (CD₃)₂SO d6, δ en ppm) : 1,18 (t, J = 7,5 Hz : 6H) ; 2,00 (s : 3H) ; 3,75 (s : 2H) ; 4,17 (q, J = 7,5 Hz : 4H) ; 7,61 (s : 1H) ; 8,37 (s large : 1H) ; 9,00 (s : 1H)].

5-Chlorométhyl-thiazole : A une solution de 8,2 g de 5-méthyl-thiazole dans 250 cm³ de tétrachlorure de carbone on ajoute 11 g de N-chloro-succinimide puis 0,1 g de peroxyde de benzoyle. Le mélange est chauffé pendant 20 heures à une température voisine de 80°C puis exposé aux U.V. pendant 5 heures. Le mélange réactionnel est refroidi, filtré, concentré sous pression réduite (1 kPa), à une température voisine de 20°C. On obtient 6,4 g de 5-Chlorométhyl-thiazole [Spectre de masse: DCI m/z=134 MH⁺]

### Exemple 23

### Dichlorhydrate de (+)-(4R)-4-(pyrazin-2-yl-méthyl)-4,5-dihydro-thiazol-2-ylamine

Un mélange racémique de 2.9 g de dichlorhydrate de (4RS)-4-(pyrazin-2-yl-méthyl)-4,5-dihydro-thiazol-2-ylamine est séparé sur une colonne CHIRALCEL OD10µ dans un mélange heptane-éthanol-triéthylamine (80/20/0,1 en volumes). Les fractions contenant le produit attendu sont concentrées sous pression réduite (1 kPa), à une température voisine de 40°C. Le résidu obtenu est dissout dans 5 cm³ d'éthanol puis on ajoute 6 cm³ d'acide chlorhydrique en solution dans l'éther diéthylique et 15 cm³ d'éther diéthylique. Après filtration, lavage par 15 cm³ d'éther diéthylique et séchage on obtient 0,371 g de dichlorhydrate de (+)-(4R)-4-(pyrazin-2-yl-méthyl)-4,5-dihydro-thiazol-2-ylamine sous forme d'un solide beige fondant à 154°C [Spectre de R.M.N. ¹H (300 MHz, (CD₃)₂SO d6, δ en ppm) : 3,22 (d, J = 6,5 Hz : 2H) ; 3,45 (dd, J = 11,5 et 5,5 Hz : 1H) ; 3,75 (dd, J = 11,5 et 8 Hz : 1H) ; 4,73 (mt : 1H) ; 8,60 (d large, J = 2,5 Hz : 1H) ; 8,63 (dd, J = 2,5 et 1,.5 Hz : 1H) ; 8,66 (s large : 1H) ; 9,09 (mf : 1H) ; 9,58 (mf : 1H) ; 9,95 (s large : 1H) ; (a_{D}²⁰= +46,3 +/-1,1 dans le méthanol à 0,5%)].

Dichlorhydrate de (4RS)-4-(pyrazin-2-yl-méthyl)-4,5-dihydro-thiazol-2-ylamine : Une solution de 7,2 g de N-(*tert*-Butyl)-N'-[(1RS)-1-hydroxyméthyl-2-(pyrazin-2-yl)-éthyl]-thiourée dans 20 cm³ d'acide chlorhydrique 5N est chauffée à une température voisine de 110°C pendant 20 heures. Le milieu réactionnel est concentré sous pression réduite (1 kPa), à une température voisine de 55°C puis repris par de l'éthanol en concentrant dans les mêmes conditions que ci-dessus. Le résidu est chromatographié sous pression atmosphérique, sur une colonne de gel de silice (granulométrie 40-60µ ; diamètre 4 cm ; hauteur 30 cm), en éluant par un mélange d'acide acétique-eau-acétate d'éthyle (1/1/4 en volumes). Les fractions contenant le produit attendu sont réunies puis concentrées dans les conditions ci-dessus. L'huile obtenue est reprise dans l'éthanol et le précipité obtenu est filtré puis séché au dessiccateur sous pression réduite (0,1 kPa) à une température voisine de 40°C pour obtenir 2,9 g de dichlorhydrate de (4R,S)-4-(pyrazin-2-yl-méthyl)-4,5-dihydro-1,3-thiazol-2-ylamine sous forme d'un solide brun fondant à 190°C [Spectre de R.M.N. ¹H (300 MHz, (CD₃)₂SO d6, δ en ppm) : 3,23 (d, J = 6 Hz : 2H) ; 3,43 (dd, J = 11,5 et 5,5 Hz : 1H) ; 3,72 (dd, J = 11,5 et 7,5 Hz : 1H) ; 4,74 (mt : 1H) ; 8,59 (s large : 1H) ; 8,64 (s large : 1H) ; 8,67 (s large : 1H) ; 9,29 (mf: 1H) ; 9,77 (mf : 1H) ; 10,16 (s large : 1H)].

N-(*tert*-Butyl)-N'-[(1RS)-1-hydroxyméthyl-2-(pyrazin-2-yl)-éthyl]-thiourée : A une solution de 7,91 g de dichlorhydrate de (2RS)-2-amino-3-(pyrazin-2-yl)-propan-1-ol dans 80 cm³ d'éthanol on ajoute sous agitation 11,2 cm³ de triéthylamine puis 7,2 cm³ de *tert*-butyl-isothiocyanate. Le mélange est chauffé à une température voisine de 50°C pendant 18 heures. Après refroidissement le milieu réactionnel est concentré sous pression réduite (1 kPa), à une température voisine de 40°C, repris par 100 cm³ d'eau et extrait par du dichlorométhane. Les phases organiques sont réunies, lavées avec 50 cm³ d'eau, séchées sur sulfate de magnésium, filtrées puis concentrées sous pression réduite (1 kPa), à une température voisine de 40°C. On obtient 7,2 g de N-(*tert*-butyl)-N'-[(1RS)-1-hydroxyméthyl-2-(pyrazin-2-yl)-éthyl]-thiourée sous forme d'une pâte incolore [Spectre de masse : DCI m/z=269 MH⁺]

Dichlorhydrate de (2RS)-2-amino-3-(pyrazin-2-yl)-propan-1-ol : Une solution de 11g de N-[(1RS)-1-hydroxy-méthyl-2-(pyrazin-2-yl)-éthyl]-acétamide dans 30 cm³ d'acide chlorhydrique 5N est chauffée à une température voisine de 115°C pendant 18 heures. Le mélange réactionnel est concentré sous pression réduite (1 kPa), à une température voisine de 40°C. On obtient 7,9 g de dichlorhydrate de (2RS)-2-amino-3-(pyrazin-2-yl)-propan-1-ol sous forme d'une pâte noire [Spectre de masse : DCI m/z=154 MH⁺]

N-[(1RS)-1-Hydroxyméthyl-2-(pyrazin-2-yl)-éthyl]-acétamide : A une solution de 8,5 g (2RS)-2-acétylamino-3-(pyrazin-2-yl)-propanoate d'éthyle dans 100 cm³ d'éthanol on ajoute 2,8 g de borohydrure de sodium puis on agite à une température voisine de 20°C. Au bout de 18 heures le milieu réactionnel est repris par 50 cm³ d'eau et 100 cm³ de dichlorométhane et la phase aqueuse est concentrée sous pression réduite (1 kPa), à une température voisine de 40°C. On obtient 11 g de N-[(1RS)-1-hydroxyméthyl-2-(pyrazin-2-yl)-éthyl]-acétamide sous forme d'une pâte brune [Spectre de R.M.N. ¹H (300 MHz, (CD₃)₂SO d6 avec ajout de quelques gouttes de CD₃COOD d4, δ en ppm) : 1,71 (s : 3H) ; 2,81 (dd, J = 14,5 et 9 Hz : 1H) ; 3,03 (dd, J = 14,5 et 5 Hz : 1H) ; 3,35 (dd, J = 11 et 6 Hz : 1H) ; 3,42 (dd, J = 11 et 5 Hz : 1H) ; 4,11 (mt : 1H) ; 7,75 (d résiduel, J = 8,5 Hz : 0,5H) ; 8,45 (d, J = 2,5 Hz : 1H) ; 8,50 (s large : 1H) ; 8,53 (mt : 1H]).

(2RS)-2-Acétylamino-3-(pyrazin-2-yl)-propanoate d'éthyle : A une solution de 14 g de 2-acétylamino-2-(pyrazin-2-yl-méthyl)-malonate de diéthyle dans 240 cm³ d'éthanol on ajoute goutte à goutte 12 cm³ de soude 6N puis on agite à une température voisine de 20°C. Au bout de 1 heure le milieu réactionnel est neutralisé par 6 cm³ d'acide chlorhydrique 12N. Après 2 heures d'agitation à une température voisine de 20°C le milieu réactionnel est filtré puis les filtrats sont concentrés sous pression réduite (1 kPa), à une température voisine de 40°C. Le résidu est repris par 200 cm³ de dioxanne, porté au reflux pendant 2 heures et concentré comme ci dessus. On obtient 8,5 g de (2RS)-2-acétylamino-3-(pyrazin-2-yl)-propanoate d'éthyle sous forme d'un solide crème [Spectre de R.M.N. ¹H (300 MHz, (CD₃)₂SO d6, δ en ppm) : 1,13 (t, J = 7 Hz : 3H) ; 1,80 (s : 3H) ; 3,12 (dd, J = 14 et 8,5 Hz : 1H) ; 3,22 (dd, J = 14 et 6 Hz : 1H) ; 4,07 (q, J = 7 Hz : 2H) ; 4,71 (mt : 1H) ; 8,36 (d, J = 8 Hz : 1H) ; 8,52 (d, J = 2,5 Hz : 1H) ; de 8,55 à 8,65 (mt : 2H].

Le 2-acétylamino-2-(pyrazin-2-yl-méthyl)-malonate de diéthyle peut être préparé selon C.Petermann et J.L. Fauchere ; Helv.Chim.Acta (1983), 66(5), 1513-1518.

### Exemples 24 et 25

### 4-(Imidazol-1-yl-méthyl)-4,5-dihydro-thiazol-2-ylamine, énantiomère A et 4-(imidazol-1-yl-méthyl)-4,5-dihydro-thiazol-2-ylamine, énantiomère B

Un mélange racémique de 0,75 g de (4RS)-4-(imidazol-1-yl-méthyl)-4,5-dihydro-thiazol-2-ylamine est séparé sur une colonne CHIRALCEL OD10µ dans un mélange heptane-isopropanol-2-triéthylamine (80/20/0,1 en volumes). Les fractions contenant le produit attendu sont réunies puis concentrées sous pression réduite (1 kPa), à une température voisine de 40°C pour obtenir 0,215 g de 4-(imidazol-1-yl-méthyl)-4,5-dihydro-thiazol-2-ylamine, énantiomère A et 0,21 g de 4-(imidazol-1-yl-méthyl)-4,5-dihydro-thiazol-2-ylamine, énantiomère B.

4-(Imidazol-1-ylméthyl)-4,5-dihydro-thiazol-2-ylamine, énantiomère A : Spectre de R.M.N. ¹H (300 MHz, (CD₃)₂SO d6, δ en ppm) : 2,93 (dd, J = 11 et 7 Hz : 1H) ; 3,25 (dd, J = 11 et 7 Hz : 1H) ; 4,03 (d, J = 6 Hz : 2H) ; 4,38 (mt : 1H) ; 6,49 (mf : 2H) ; 6,88 (s large : 1H) ; 7,20 (s large : 1H) ; 7,64 (s large : 1H).

4-(Imidazol-1-ylméthyl)-4,5-dihydro-thiazol-2-ylamine, énantiomère B : Spectre de R.M.N. ¹H (300 MHz, (CD₃)₂SO d6, δ en ppm) : 2,96 (dd, J = 11 et 7,5 Hz : 1H) ; de 3,20 à 3,40 (mt : 1H) ; 4,05 (d, J = 6 Hz : 2H) ; 4,41 (mt : 1H) ; 6,88 (s large : 1H) ; 7,20 (s large : 1H) ; 7,64 (s large : 1H).

(4RS)-4-(Imidazol-1-ylméthyl)-4,5-dihydro-thiazol-2-ylamine : A une solution de 1,39 g de N-[(4RS)-4-(imidazol-1-yl-méthyl)-4,5-dihidro-thiazol-2-yl]-carbamate de *tert*-butyle dans 5 cm³ de dioxanne on ajoute 5 cm³ d'acide chlorhydrique 4N dans le dioxanne puis on ajoute du méthanol pour dissoudre le milieu réactionnel. Après une agitation à une température voisine de 20°C pendant 48 heures, le milieu réactionnel est concentré sous pression réduite (1 kPa), à une température voisine de 40°C, lavé par de l'oxyde d'isopropyle, par de l'acétate d'éthyle puis du méthanol. La suspension obtenue est filtrée et le filtrat est évaporé comme ci-dessus puis chromatographié sur une colonne de gel de silice en éluant par un mélange de dichlorométhane-méthanol-ammoniaque 28% (50-5-1 en volumes). Les fractions contenant le produit attendu sont réunies puis concentrées dans les conditions ci-dessus pour obtenir 0,33 g de (4RS)-4-(imidazol-1-ylméthyl)-4,5-dihydro-thiazol-2-ylamine sous forme d'une huile visqueuse jaune [Spectre de R.M.N. ¹H (300 MHz, (CD₃)₂SO d6, δ en ppm) : 2,93 (mt : 1H) ; 3,26 (mt : 1H) ; 4,03 (d, J = 6 Hz : 2H) ; 4,39 (mt : 1H) ; 6,49 (mf : 2H) ; 6,87 (mt : 1H) ; 7,20 (mt : 1H) ; 7,63 (s large : 1H)].

N-[(4RS)-4-(Imidazol-1-ylméthyl)-4,5-dihidro-thiazol-2-yl]-carbamate de *tert-* butyle : A une solution de 0,085 g d'hydrure de sodium dans 20 cm³ de diméthylformamide on ajoute 0.21 g d'imidazole préalablement dissout dans 10 cm³ de diméthylformamide. Après disparition de l'hydrure de sodium on ajoute une solution de 1 g de N-[(4RS)-4-(p-toluènesulfonyloxy-méthyl)-4,5-dihydro-thiazol-2-yl]-carbamate de *tert*-butyle dans 10 cm³ de diméthylformamide puis on agite à une température voisine de 70°C pendant 3 heures. Le milieu réactionnel est dilué par de l'acétate d'éthyle, lavé à l'eau, séché au sulfate de magnésium puis concentré sous pression réduite (1 kPa), à une température voisine de 40°C. Le résidu obtenu est chromatographié sur colonne d'alumine en éluant par de l'acétate d'éthyle et un mélange d'acétate d'éthyle-méthanol (80/20 en volumes). Les fractions contenant le produit attendu sont réunies puis concentrées dans les conditions ci-dessus pour obtenir 0,25 g de N-[(4RS)-4-(imidazol-1-ylméthyl)-4,5-dihydro-thiazol-2-yl]-carbamate de *tert*-butyle sous forme d'une masse collante jaune [Spectre de masse : DCI m/z=283 MH⁺ m/z=183 (M-C₅H₇O₂)⁺].

N-[(4RS)-4-(p-Toluènesulfonyloxy-méthyl)-4,5-dihydro-thiazol-2-yl]-carbamate de *tert*-butyle : Une solution de 0,8 g de N-[(4RS)-4-hydroxyméthyl-4,5-dihydro-thiazol-2-yl]-carbamate de *tert*-butyle, de 0,76 g de chlorure de p-toluène-sulfonyle et de 0,56 cm³ de triéthylamine dans 25 cm³ de dichlorométhane est agitée pendant 16 heures à une température voisine de 20°C. La solution obtenue est concentrée sous pression réduite (1 kPa) à une température voisine de 40°C. Le résidu d'évaporation obtenu est purifié par chromatographie sous pression atmosphérique sur une colonne de gel de silice (granulométrie 60-200µ ; diamètre 2 cm ; hauteur 25 cm), en éluant par un mélange de cyclohexane-acétate d'éthyle (70/30 en volumes) et en recueillant des fractions de 30 cm³. Les fractions contenant le produit attendu sont réunies puis concentrées dans les conditions de ci-dessus. On obtient 0,8 g de N-[(4RS)-4-(p-toluènesulfonyloxy-méthyl)-4,5-dihydro-thiazol-2-yl]-carbamate de *tert*-butyle sous forme d'un solide blanc [Spectre de R.M.N. ¹H (300 MHz, CDCl₃, δ en ppm) : 1,48 (s : 9H) ; 2,46 (s : 3H) ; 3,10 (dd, J = 11,5 et 5,5 Hz : 1H) ; 3,33 (dd, J = 11,5 et 8,5 Hz : 1H) ; 3,97 (dd, J = 9,5 et 8 Hz : 1H) ; 4,06 (dd, J = 9,5 et 4,5 Hz : 1H) ; 4,43 (mt : 1H) ; 7,36 (d, J = 8 Hz : 2H) ; 7,80 (d, J = 8 Hz : 2H) ; de 8,50 à 9,40 (mf très étalé : 1H)].

N-[(4RS)-4-Hydroxyméthyl-4,5-dihydro-thiazol-2-yl]-carbamate de *tert*-butyle : A une solution de 2g de 2-[(*tert*-butoxycarbonyl)imino]-(4RS)-4-[(*tert*-butoxycarbonyl)oxy]-méthyl-1,3-thiazolidine-3-carboxylate de *tert*-butyle dans 20 cm³ de méthanol on ajoute 10 cm³ de soude aqueuse N puis on agite à une température voisine de 20°C pendant 4 heures. Le mélange réactionnel est concentré sous pression réduite (1 kPa) à une température voisine de 50°C. Le résidu obtenu est repris par 30 cm³ eau, filtré, lavé par de l'acétate d'éthyle puis de l'eau. On obtient 0,37 g de N-[(4RS)-4-hydroxyméthyl-4,5-dihydro-thiazol-2-yl]-carbamate de *tert*-butyle sous forme de solide blanc [Spectre de masse : DCI m/z=233 MH⁺ m/z=177 (M-C₄H₇)⁺].

2-[(*tert*-Butoxycarbonyl)imino]-(4RS)-4-[(*tert*-butoxycarbonyl)oxy]-méthyl-1,3-thiazolidine-3-carboxylate de *tert*-butyle : A une solution de 1,98 g de (4RS)-4-hydroxyméthyl-4,5-dihydro-thiazol-2-ylamine dans 20 cm³ de dichlorométhane on ajoute 10,91 g de di-*tert*-butyldicarbonate et 2,81 cm³ de triéthylamine puis on agite à une température voisine de 20°C. Au bout de 4 heures, on ajoute à nouveau 3 cm³ de triéthylamine, puis on agite pendant 16 heures à une température voisine de 20°C. On ajoute 50 cm³ d'eau au mélange réactionnel et on décante. La phase organique est sèchée sur sulfate de magnésium, filtrée et concentrée sous pression réduite (1 kPa), à une température voisine de 40°C. On obtient 7 g de 2-[(*tert*-butoxycarbonyl)imino]-(4RS)-4-[(*tert*-butoxycarbonyl)oxy]-méthyl-1,3-thiazolidine-3-carboxylate de *tert*-butyle sous forme d'un solide blanc [Spectre de masse : DCI m/z=433 MH⁺ m/z=333 (M-C₅H₇O₂)⁺].

(4RS)-4-Hydroxyméthyl-4,5-dihydro-thiazol-2-ylamine : Une solution de 90 g de 1-*tert*-butyl-3-(2-hydroxy-1-hydroxyméthyl-éthyl)-thiourée dans 500 cm³ d'acide chlorhydrique 6N est agitée à une température voisine de 100°C. Après 3 heures, le mélange réactionnel est refroidi à une température voisine de 20°C puis est concentré sous pression réduite (1 kPa) à une température voisine de 50°C. Le résidu obtenu est repris par 100 cm³ d'eau, alcalinisé par 100 cm³ de soude 5N puis concentré comme précédemment. L'huile obtenue est agitée pendant 20 heures à une température voisine de 20°C dans 300 cm³ d'éthanol, filtrée, lavée par 5 fois 50 cm³ d'éthanol et 3 fois 100 cm³ de méthanol. Les différents filtrats sont réunis, évaporés sous pression réduite (1 kPa), à une température voisine de 40°C puis cristallisés dans 400 cm³ d'éthanol pour obtenir 31 g de (4RS)-4-hydroxyméthyl-4,5-dihydro-thiazol-2-ylamine sous forme d'un solide blanc fondant à 122°C [Spectre infra-rouge (KBr): 3311; 3164; 1648; 1601; 1349; 1051 et 982 cm⁻¹].

1-*tert*-Butyl-3-(2-hydroxy-1-hydroxyméthyl-éthyl)-thiourée : A une solution de 14,6 g de 2 amino-1,3-propane-diol dans 245 cm³ d'éthanol on ajoute 30,4 cm³ d'isothiocyanate de *tert*-butyle puis on agite à une température voisine de 20°C pendant 94 heures. Le milieu réactionnel est concentré sous pression réduite (5 kPa) à une température voisine de 40°C et le résidu est trituré dans un mélange de 160 cm³ d'éther de pétrole et de 26 cm³ d'éthanol, filtré puis séché au dessiccateur sous pression réduite (0,1 kPa) à une température voisine de 60°C. On obtient 30 g de 1-*tert*-butyl-3-(2-hydroxy-1-hydroxyméthyl-éthyl)-thiourée sous forme d'un solide blanc [Spectre de R.M.N. ¹H (250 MHz, (CD₃)₂SO d6, δ en ppm) : 1,42 (s : 9H) ; 3,38 (mt : 2H) ; 3,54 (mt : 2H) ; 4,17 (mf: 1H) ; 4,70 (t, J = 5 Hz : 2H) ; 7,08 (d, J = 8 Hz : 1H) ; 7,38 (s : 1H]).

### Exemple 26

### Dichlorhydrate de (+)-(4R)-4-(thiazol-4-yl-méthyl)-4,5-dihydro-thiazol-2-ylamine

Une solution de 1,75 g de N-(*tert*-butyl)-N'-[(1R)-1-hydroxyméthyl-2-(thiazol-4-yl)-éthyl]-thiourée dans 20 cm³ d'acide chlorhydrique 6N est chauffée à une température voisine de 110°C pendant 20 heures. Le milieu réactionnel est concentré sous pression réduite (1 kPa), à une température voisine de 60°C, repris par 5 cm³ d'éthanol puis concentré dans les mêmes conditions que ci-dessus. Le résidu est repris par 5 cm³ d'éthanol, puis filtré et séché au dessiccateur sous pression réduite (0,1 kPa) à une température voisine de 40°C. On obtient 0,54 g de dichlorhydrate de (+)-(4R)-4-(thiazol-4-yl-méthyl)-4,5-dihydro-thiazol-2-ylamine sous forme d'un solide crème fondant à 195°C [Spectre de R.M.N. ¹H (300 MHz, (CD₃)₂SO d6, δ en ppm) : 3,67 (mt : 2H) ; 3,41 (dd, J = 11,5 et 5,5 Hz : 1H) ; 3,68 (dd, J = 11,5 et 8 Hz : 1H) ; 4,63 (mt : 1H) ; 7,59 (d, J = 2 Hz : 1H) ; 9,14 (d, J = 2 Hz : 1H) ; 9,18 (mf : 1H) ; 9,66 (mf : 1H) ; 10,04 (s large : 1H) ; (a_{D}²⁰= +24,2 +/- 0,8 dans le méthanol à 0,5%)].

N-(*tert*-Butyl)-N'-[(1R)-1-hydroxyméthyl-2-(thiazol-4-yl)-éthyl]-thiourée : Une solution de 2,5 g de N-[(1R)-1-hydroxyméthyl-2-(thiazol-4-yl)-éthyl]-carbamate de *tert*-butyle dans 20 cm³ d'acide chlorhydrique 6N et 20 cm³ de dioxanne est agitée à une température voisine de 20°C pendant 18 heures. Le mélange réactionnel est concentré sous pression réduite (1 kPa) à une température voisine de 40°C. On obtient 1,9 g de solide blanc. On reprend le solide obtenu dans 6 cm³ d'éthanol absolu auquel on ajoute 2 cm³ de triéthylamine puis 2,1 cm³ de *tert*-butylisothiocyanate. Le mélange est agité à une température voisine de 50°C pendant 20 heures. Le milieu réactionnel est concentré sous pression réduite (1 kPa) à une température voisine de 40°C. Le résidu est repris par de l'eau et la phase aqueuse est extraite avec du dichlorométhane. La phase organique est séchée sur sulfate de magnésium, filtrée et concentrée dans les conditions ci-dessus. Le résidu est séché au dessiccateur sous pression réduite (0,1 kPa) à une température voisine de 40°C. On obtient 1,75 g de N-(*tert*-butyl)-N'-[(1R)-1-hydroxyméthyl-2-(thiazol-4-yl)-éthyl]-thiourée sous forme d'un solide jaune [Spectre de R.M.N. ¹H (300 MHz, (CD₃)₂SO d6, δ en ppm) : 1,41 (s : 9H) ; 2,96 (dd, J = 15 et 7 Hz : 1H) ; 3,05 (dd, J = 15 et 7 Hz : 1H) ; 3,41 (mt : 2H) ; 4,62 (mf : 1H) ; 4,85 (t, J = 5 Hz : 1H) ; 7,21 (d, J = 8,5 Hz : 1H) ; 7,23 (s large : 1H) ; 7,37 (d, J = 2 Hz : 1H) ; 9,04 (d, J = 2 Hz : 1H)].

N-[(1R)-1-Hydroxyméthyl-2-(thiazol-4-yl)-éthyl]-carbamate de *tert*-butyle : A une solution de 3 g de BOC-D-(4-thiazolyl)alanine dans 50 cm³ de tétrahydrofuranne on ajoute sous agitation 1,59 cm³ de triéthylamine. Le milieu réactionnel est refroidi à une température voisine de -18°C puis on ajoute sous agitation et sous atmosphère inerte 1,16 cm³ de chloroformiate d'isobutyle. Après 40 minutes d'agitation à une température voisine de -15°C le milieu réactionnel est filtré rapidement à froid puis est ajouté aux filtrats, à une température voisine de -15°C et sous agitation une solution de 0,85 g de borohydrure de sodium préalablement dissout dans 6 cm³ d'eau. Après 18 heures d'agitation à une température voisine de 20°C le milieu réactionnel est dilué avec 100 cm³ d'eau et extrait par de l'acétate d'éthyle. La phase organique est lavée avec de l'eau, séchée sur sulfate de magnésium, filtrée et concentrée sous pression réduite (1 kPa) à une température voisine de 50°C. On obtient 2,5 g de N-[(1R)-1-hydroxyméthyl-2-(thiazol-4-yl)-éthyl]-carbamate de *tert*-butyle sous forme d'une huile incolore [Spectre infra-rouge (CCl₄) : 3442, 3368, 2980, 2932, 2874, 1710, 1501, 1392, 1367, 1243, 1171, 1048 et 875 cm⁻¹].

### Exemple 27

### Dichlorhydrate de (+)-(4R)-4-(3-aminopropyl)-4,5-dihydro-thiazol-2ylamine

Une solution de 9,6 g de N-[(4R)-4-(3-*tert*-butyl-thiouréido)-5-hydroxy-pentyl]-carbamate de benzyle dans 50 cm³ d'acide chlorhydrique 6N est chauffé à une température voisine de 110°C pendant 18 heures. Le milieu réactionnel est concentré sous pression réduite (1 kPa), à une température voisine de 40°C, repris successivement par 30 cm³ d'éthanol et 20 cm³ d'isopropanol en concentrant dans les même conditions que ci-dessus entre chaque lavage. Le solide obtenu est repris de l'éthanol et de l'éther diéthylique, filtré et séché au dessiccateur sous pression réduite (0,1 kPa) à une température voisine de 40°C pour obtenir 2,8 g de dichlorhydrate de (+)-(4R)-4-(3-aminopropyl)-4,5-dihydro-thiazol-2-ylamine sous forme d'un solide blanc fondant à 166°C [Spectre de R.M.N. ¹H (300 MHz, (CD₃)₂SO d6, δ en ppm) : de 1,55 à 1,85 (mt : 4H) ; 2,81 (mt : 2H) ; 3,25 (dd, J = 11 et 6 Hz : 1H) ; 3,69 (dd, J = 11 et 8 Hz : 1H) ; 4,27 (mt : 1H) ; 8,16 (mf: 3H) ; 9,50 (mf étalé : 2H) ; de 9,50 à 10,60 (mftrès étalé : 1H) ; (a_{D}²⁰= +7,5 +/- 0,4 dans le méthanol à 0,5%].

N-[(4R)-4-(3-*tert*-Butyl-thiouréido)-5-hydroxy-pentyl]-carbamate de benzyle : A une solution de 16 g de (2R)-5-{[(benzyloxy)carbonyl]amino}-2-(3-*tert*-butyl-thiouréido) pentanoate d'éthyle dans 200 cm³ d'éthanol et 100 cm³ de tétrahydrofuranne on ajoute sous agitation 2,24 g de chlorure de lithium. Après 15 minutes d'agitation à une température voisine de 0°C on ajoute 2,24 g de borohydrure de sodium. Après 20 heures d'agitation à une température voisine de 20°C on ajoute de nouveau 0,5 g de borohydrure de sodium. Le milieu réactionnel est filtré et les filtrats sont concentrés sous pression réduite (1 kPa), à une température voisine de 40°C. Le résidu est chromatographié sous pression atmosphérique, sur une colonne de gel de silice (granulométrie 60-200µ ; diamètre 5,6 cm ; hauteur 40 cm), en éluant par un mélange de dichlorométhane-acétate d'éthyle (8/2 en volumes) et un mélange de dichlorométhane-acétate d'éthyle (6/4 en volumes). Les fractions contenant le produit attendu sont réunies puis concentrées dans les conditions ci-dessus. On obtient 9,6 g de N-[(4R)-4-(3-*tert*-butyl-thiouréido)-5-hydroxy-pentyl]-carbamate de benzyle sous forme d'une huile jaune [Spectre de R.M.N. ¹H (300 MHz, (CD₃)₂SO d6, δ en ppm) : de1,30 à 1,60 (mt : 4H) ; 1,42 (s : 9H) ; 3,00 (mt : 2H) ; de 3,25 à 3,40 (mt : 1H) ; 3,45 (mt : 1H) ; 4,21 (mf : 1H) ; 4,76 (mf : 1H) ; 5,02 (s : 2H) ; 7,09 (d, J = 8,5 Hz : 1H) ; 7,17 (s large : 1H) ; 7,27 (t, J = 5,5 Hz : 1H) ; de 7,30 à 7,45 (mt : 5H)].

(2R)-5-{[(Benzyloxy)carbonyl]amino}-2-(3-*tert*-butyl-thiouréido)-pentanoate d'éthyle : A une solution de 13 g de chlorhydrate de (2R)-2-amino-5-[(benzyloxy)carbonyl]amino-pentanoate d'éthyle dans 200 cm³ d'éthanol on ajoute 7,5 cm³ d'isothiocyanate de *tert*-butyle puis on agite à une température voisine de 20°C. Au bout de 48 heures, le milieu réactionnel est concentré sous pression réduite (1 kPa), à une température voisine de 40°C pour obtenir 16 g de (2R)-5-{[(benzyloxy)carbonyl]amino}-2-(3-*tert*-butyl-thiouréido)-pentanoate d'éthyle sous forme d'une huile incolore [Spectre de masse : EI m/z=409 M⁺ m/z=232 (C₁₃H₁₄NO₃)⁺ m/z=142 (232-PhCH)⁺ m/z=91 (C₇H₇)⁺].

Chlorhydrate de (2R)-2-amino-5-[(benzyloxy)carbonyl]amino-pentanoate d'éthyle : Une solution de 12,5 g de chlorhydrate de l'acide (2R)-2-amino-5-[(benzyloxy)carbonyl]amino-pentanoïque dans 200 cm³ d'éthanol est refroidie à une température voisine de -20°C. On coule goutte à goutte 6,5 cm³ de chlorure de thionyle puis on agite à une température voisine de 20°C pendant 18 heures. Le milieu réactionnel est concentré sous pression réduite (1 kPa), à une température voisine de 40°C pour obtenir 13 g de chlorhydrate de (2R)-2-amino-5-[(benzyloxy)carbonyl]amino-pentanoate d'éthyle [Spectre de masse : DCI m/z=295 MH⁺].

Chlorhydrate de l'acide (2R)-2-amino-5-[(benzyloxy)carbonyl]amino-pentanoïque : A une solution de 15,5 g de chlorhydrate de D-ornithine dans 1 dm³ d'eau on ajoute 30 g de carbonate basique de cuivre II. Le mélange est chauffé à une température voisine de 100°C pendant 2 heures, filtré à chaud et lavé à l'eau. Les filtrats sont refroidis à une température voisine de 20°C puis, sous agitation, on ajoute 14,5 g d'oxyde de magnésium. Le milieu réactionnel est refroidi à une température voisine de 0°C puis on coule en 4 fois 15,5 cm³ de chloroformiate de benzyle. Après 18 heures à une température voisine de 20°C, le mélange est filtré. Le solide est lavé successivement avec 3 fois 30 cm³ d'eau et 3 fois 30 cm³ d'éther diéthylique puis mis en suspension dans 500 cm³ d'acide chlorhydrique N avec un léger courant de sulfure d'hydrogène pendant 2 heures. La suspension est filtrée et le solide lavé avec de l'acide chlorhydrique 0,5N. Les filtrats sont amenés à pH 4-5 avec de l'ammoniaque diluée ; formation d'un précipité blanc. Après filtration et séchage au dessiccateur on obtient 12,5 g de chlorhydrate de l'acide (2R)-2-amino-5-[(benzyloxy)carbonyl]amino-pentanoïque sous forme d'un solide blanc [Spectre de R.M.N. ¹H (300 MHz, (CD₃)₂SO d6, δ en ppm) : de 1,40 à 1,80 (mt : 4H) ; 2,99 (mt : 2H) ; 3,11 (mt : 1H) ; 5,02 (s large : 2H) ; de 7,25 à 7,45 (mt : 6H].

### Exemple 28

### Chlorhydrate de (+)-(4R)-4-(4-hydroxy-benzyl)-4,5-dihydro-thiazol-2-ylamine

Une solution de 0,2 g de N-(*tert*-butyl)-N'-[(1R)-1-hydroxyméthyl-2-(4-hydroxy-phényl)-éthyl]-thiourée dans 2,6 cm³ d'acide chlorhydrique 6N est chauffée à une température voisine de 110°C pendant 18 heures. Le milieu réactionnel est concentré sous pression réduite (1 kPa) à une température voisine de 55°C puis repris successivement par 10 cm³ d'éther isopropylique, 10 cm³ d'éther diéthylique, 10 cm³ de pentane, 10 cm³ d'éther de pétrole, 10cm³ d'éther isopropylique en concentrant dans les même conditions que ci-dessus entre chaque lavage. Le solide obtenu est séché au dessiccateur sous pression réduite (0,1 kPa) à une température voisine de 40°C pour obtenir 0,098 g de chlorhydrate de (+)-(4R)-4-(4-hydroxy-benzyl)-4,5-dihydro-thiazol-2-ylamine sous forme d'un solide orangé [Spectre de R.M.N. ¹H (300 MHz, (CD₃)₂SO d6, δ en ppm) : 2,78 (dd, J = 13,5 et 7,5 Hz : 1H) ; 2,88 (dd, J = 13,5 et 5,5 Hz : 1H) ; 3,26 (dd, J = 11,5 et 6 Hz : 1H) ; 3,55 (dd, J = 11,5 et 7,5 Hz : 1H) ; 4,45 (mt : 1H) ; 6,74 (d, J = 8,5 Hz : 2H) ; 7,07 (d, J = 8,5 Hz : 2H) ; 9,09 (mf : 1H) ; 9,41 (s: 1H) ; 9,56 (mf: 1H) ; 9,97 (s large: 1H), (a_{D}²⁰= +9,5 +/- 0,5 dans le méthanol à 0,5%)].

N-(*tert*-Butyl)-N'-[(1R)-1-hydroxyméthyl-2-(4-hydroxy-phényl)-éthyl]-thiourée : A une solution de 0,2 g de 4-[(2R)-2-amino-3-hydroxy-propyl]-phénol dans 10 cm³ d'éthanol absolu on ajoute 0,15 cm³ de triéthylamine puis 0,18 cm³ de *tert*-butylisothiocyanate. Le mélange est agité à une température voisine de 20°C pendant 18 heures puis chauffé à une température voisine de 60°C pendant 4 heures. Après refroidissement le milieu réactionnel est évaporé sous pression réduite (1 kPa) à une température voisine de 40°C puis chromatographié sous une pression d'argon de 60 kPa, sur une colonne de gel de silice (granulométrie 40-63µ ; diamètre 2,5 cm ; hauteur 29 cm), en éluant par un mélange de dichlorométhane-méthanol (98/2 en volumes). Les fractions contenant le produit attendu sont réunies puis concentrées dans les conditions de ci-dessus. On obtient 0,2 g de N-(*tert*-butyl)-N'-[(1R)-1-hydroxyméthyl-2-(4-hydroxy-phényl)-éthyl]-thiourée, sous forme d'une huile visqueuse incolore [Spectre de R.M.N. ¹H (300 MHz, (CD₃)₂SO d6, δ en ppm) : 1,42 (s : 9H) ; 2,63 (dd, J = 13,5 et 8 Hz : 1H) ; 2,72 (dd, J = 13,5 et 5,5 Hz : 1H) ; de 3,25 à 3,40 (mt : 2H) ; 4,29 (mf : 1H) ; 4,83 (t, J = 4,5 Hz : 1H) ; 6,67 (d, J = 8 Hz : 2H) ; 7,05 (d, J = 8 Hz : 2H) ; 7,15 (d, J = 8 Hz : 1H) ; 7,26 (s large : 1H) ; 9,16 (s : 1H].

4-[(2R)-2-Amino-3-hydroxy-propyl)]-phénol : A une solution de 20,5 cm³ d'acide chlorhydrique 4N dans le dioxanne on ajoute sous agitation et sous atmosphère inerte, à une température voisine de 20°C, une solution de 2,2 g de N-[(1R)-1-hydroxyméthyl-2-(4-hydroxy-phényl)-éthyl]-carbamate de *tert*-butyle dans 24 cm³ de dichlorométhane et 4 cm³ de dioxanne. Le mélange est chauffé à une température voisine de 60°C pendant 18 heures, puis il est refroidi et concentré sous pression réduite (1 kPa) à une température voisine de 60°C. Le résidu est repris dans 50 cm³ de dichlorométhane, filtré, trituré dans 20 cm³ d'éther diéthylique, refiltré, lavé avec 2 fois 5 cm³ d'éther diéthylique puis séché au dessiccateur sous pression réduite (0,1 kPa) à une température voisine de 40°C. On obtient 1,4 g de 4-[(2R)-2-amino-3-hydroxy-propyl]-phénol sous forme d'un solide de couleur beige fondant à 156°C [Spectre de R.M.N. ¹H (300 MHz, (CD₃)₂SO d6, δ en ppm) : 2,69 (dd, J = 13 et 9 Hz : 1H) ; 2,79 (dd, J = 13 et 5,5 Hz : 1H) ; 3,23 (mt : 1H) ; de 3,30 à 3,45 (mt : 1H) ; 3,51 (ddd, J = 11,5 - 5 et 4,5 Hz : 1H) ; 5,32 (t, J = 5 Hz : 1H) ; 6,73 (d, J = 8,5 Hz : 2H) ; 7,06 (d, J = 8,5 Hz : 2H) ; 7,95 (mf : 3H) ; 9,37 (s : 1H]).

N-[(1R)-1-Hydroxyméthyl-2-(4-hydroxy-phényl)-éthyl]-carbamate de *tert*-butyle : A une solution de 4,38 g de BOC-D-tyrosynate de méthyle dans 25 cm³ de tétrahydrofuranne on ajoute sous agitation et sous atmosphère inerte, à une température voisine de 20°C, une solution de 29,6 cm³ d'hydrure d'aluminium lithium à 1M dans le tétrahydrofuranne. Après un ajout de 60 cm³ de tétrahydrofuranne le mélange réactionnel est chauffé à une température voisine de 70°C pendant 3 heures. Le mélange réactionnel est refroidi puis concentré sous pression réduite (1 kPa) à une température voisine de 60°C. Le résidu d'évaporation est séché au dessiccateur sous pression réduite (0,1 kPa) à une température voisine de 40°C puis chromatographié sous une pression d'argon de 60 kPa, sur une colonne de gel de silice (granulométrie 40-63µ ; diamètre 7 cm ; hauteur 24 cm), en éluant par 10 dm³ d'un mélange de dichlorométhane-méthanol (98/2 en volumes), 2 dm³ d'un mélange de dichlorométhane-méthanol (95/5 en volumes) et 2 dm³ d'un mélange de dichlorométhane-méthanol (90/10 en volumes). Les fractions contenant le produit attendu sont réunies puis concentrées dans les conditions ci-dessus. On obtient 2,2 g de N-[(1R)-1-hydroxyméthyl-2-(4-hydroxy-phényl)-éthyl]-carbamate de *tert*-butyle sous forme d'une meringue blanche [Spectre de R.M.N. ¹H (400 MHz, (CD₃)₂SO d6, δ en ppm) : 1,35 (s : 9H) ; 2,46 (dd, J = 14 et 8 Hz : 1H) ; 2,68 (dd, J = 14 et 6 Hz : 1H) ; de 3,15 à 3,40 (mt : 2H) ; 3,50 (mt : 1H) ; 4,61 (mt : 1H) ; 6,48 (d, J = 8,5 Hz : 1H) ; 6,65 (d, J = 8 Hz : 2H) ; 6,97 (d, J = 8 Hz : 2H) ; 9,11 (s large : 1H].

### Exemple 29

### (+)-4-(Pyridin-4-ylsulfanylméthyl)-4,5-dihydro-thiazol-2-ylamine

Un mélange racémique de 1,75 g de dichlorhydrate de (4RS)-4-(pyridin-4-ylsulfanylméthyl)-4,5-dihydro-thiazol-2-ylamine est séparé sur une colonne CHIRALCEL OD 10µ dans un mélange heptane-isopropanol-2-triéthylamine (80/20/0,1 en volumes) pour obtenir 0,43 g de (+)-4-(pyridin-4-ylsulfanylméthyl)-4,5-dihydro-thiazol-2-ylamine [Spectre de R.M.N. ¹H (300 MHz, (CD₃)₂SO d6, δ en ppm) : 3,12 (dd, J = 11 et 6,5 Hz : 1H) ; 3,20 (d, J = 6,5 Hz : 2H) ; 3,44 (dd, J = 11 et 7,5 Hz : 1H) ; 4,32 (mt : 1H) ; 6,49 (mf: 2H) ; 7,30 (dd, J = 5 et 1,5 Hz : 2H) ; 8,37 (dd, J = 5 et 1,5 Hz : 2H), (a_{D}²⁰= +13,3 +/- 0,6 dans le méthanol à 0,5%)].

Dichlorhydrate de (4RS)-4-(pyridin-4-ylsulfanylméthyl)-4,5-dihydro-thiazol-2-ylamine : A une solution de 0,356 g de N-[(4RS)-4-(pyridin-4-ylsulfanylméthyl)-4,5-dihydro-thiazol-2-yl]-carbamate de *tert*-butyle dans 5 cm³ de méthanol et 20 cm³ de dioxanne on ajoute 5 cm³ d'acide chlorhydrique 4N dans le dioxanne puis on agite à une température voisine de 20°C pendant 18 heures. Le milieu réactionnel est concentré sous pression réduite (5 kPa), à une température voisine de 40°C, repris dans de l'éther diisopropylique puis filtré pour obtenir 0,287 g de dichlorhydrate de (4RS)-4-(pyridin-4-ylsulfanylméthyl)-4,5-dihydro-thiazol-2-ylamine sous forme d'un solide crème [Spectre de R.M.N. ¹H (300 MHz, (CD₃)₂SO d6, δ en ppm) : 3,45 (dd, J = 11 et 4,5 Hz : 1H) ; 3,61 (dd, J = 14 et 7 Hz : 1H) ; 3,69 (dd, J = 14 et 5,5 Hz : 1H) ; 3,80 (dd, J = 12 et 8 Hz : 1H) ; 4,59 (mt : 1H) ; 8,00 (d large, J = 6,5 Hz : 2H) ; 8,66 (d large, J = 6,5 Hz : 2H) ; 9,60 (mf: 1H) ; 9,81 (mf: 1H) ; de 10,00 à 10,50 (mf étalé : 1H).

N-[(4RS)-4-(Pyridin-4-ylsulfanylméthyl)-4,5-dihydro-thiazol-2-yl]-carbamate de *tert*-butyle : On chauffe une suspension de 1,5g de N-[(4RS)-4-(p-toluènesulfonyloxyméthyl)-4,5-dihydro-thiazol-2-yl]-carbamate de *tert*-butyle dans 50 cm³ d'acétonitrile jusqu'à dissolution puis on ajoute sous agitation, 0,604 g de 4-mercapto-pyridine. Après refroidissement à une température voisine de 20°C on ajoute 1,07 g de carbonate de potassium. Après 48 heures d'agitation à une température voisine de 20°C le mélange réactionnel est filtré. Le filtrat est concentré sous pression réduite (1 kPa) à une température voisine de 50°C. Le résidu obtenu est repris dans de l'acétate d'éthyle et de l'eau et décanté. La phase organique est lavée par une solution de chlorure de sodium, séchée sur sulfate de magnésium et chromatographié sur colonne d'alumine en éluant par des mélanges successifs d'acétate d'éthyle-cyclohexane (50/50 en volumes). Les fractions contenant le produit attendu sont purifiées par chromatographie sur une colonne de gel de silice en éluant par de l'acétate d'éthyle. Les fractions contenant le produit attendu sont réunies puis concentrées dans les conditions de ci-dessus. On obtient 0,356 g de N-[(4RS)-4-(pyridin-4-ylsulfanylméthyl)-4,5-dihydro-thiazol-2-yl]-carbamate de *tert*-butyle sous forme d'une huile jaune [Spectre de R.M.N. ¹H (300 MHz, (CD₃)₂SO d6, δ en ppm) : 1,42 (s : 9 H) ; 3,09 (dd, J = 11,5 et 6 Hz : 1H) ; 3,29 (mt : 2H) ; 3,40 (dd, J = 11,5 et 8 Hz : 1H) ; 4,25 (mt : 1H) ; 7,35 (dd, J = 5 et 1,5 Hz : 2H) ; 8,40 (dd, J = 5 et 1,5 Hz : 2H) ; de 9,70 à 10,00 (mf étalé : 1H].

### Exemple 30

### Chlorhydrate de (4R)-4-(1-oxy-piridin-4-ylméthyl)-4,5-dihydro-thiazol-2-ylamine

Une solution de 0,6 g de N-(*tert*-butyl)-N'-[(1R)-1-hydroxyméthyl-2-(1-oxy-pyridin-4-yl)-éthyl]-thiourée dans 6 cm³ d'acide chlorhydrique 6N est chauffée à une température voisine de 110°C pendant 18 heures. Le milieu réactionnel est concentré sous pression réduite (1 kPa), à une température voisine de 55°C, repris par 2 fois 20 cm³ d'éthanol, concentré dans les mêmes conditions que ci-dessus, repris par 10 cm³ d'éthanol, filtré et séché au dessiccateur sous pression réduite (0,1 kPa) à une température voisine de 40°C pour obtenir 0,296 g de chlorhydrate de (4R)-4-(1-oxy piridin-4-ylméthyl)-4,5-dihydro-thiazol-2-ylamine sous forme de cristaux crèmes [Spectre de R.M.N. ¹H (300 MHz, (CD₃)₂SO d6, δ en ppm) : 3,04 (AB limite : 2H) ; 3,34 (dd, J = 12 et 5,5 Hz : 1H) ; 3,68 (dd, J = 12 et 8 Hz : 1H) ; 4,63 (mt : 1H) ; 7,75 (d, J = 7 Hz : 2H) ; 8,65 (d, J = 7 Hz : 2H) ; 9,30 (mf : 1H) ; 9,79 (mf : 1H) ; 10,30 (s large : 1H].

N-(*tert*-Butyl)-N'-[(1R)-1-hydroxyméthyl-2-(1-oxy-pyridin-4-yl)-éthyl]-thiourée: A une solution de 1,5 g de chlorhydrate de (2R)-2-amino-3-(1-oxy-pyridin-4-yl)-propan-1-ol dans 30 cm³ d'éthanol absolu on ajoute 2,6 cm³ de triéthylamine puis 1,53 cm³ de *tert*-butylisothiocyanate. Après avoir rajouté 20 cm³ de méthanol le mélange est agité à une température voisine de 20°C pendant 18 heures puis est concentré sous pression réduite (1 kPa), à une température voisine de 40°C. Au résidu d'évaporation repris dans 10 cm³ de méthanol on ajoute 1 cm³ de triéthylamine puis 1,5 cm³ de *tert*-butylisothiocyanate. Le mélange est agité à une température voisine de 20°C pendant 48 heures. Le milieu réactionnel est concentré sous pression réduite (5 kPa), à une température voisine de 40°C, repris par 25 cm³ d'acétate d'éthyle, filtré, lavé par 10 cm³ d'acétate d'éthyle et 20 cm³ d'oxyde d'isopropyle. Le chlorhydrate de triéthylamine est éliminé par un passage sur colonne d'alumine. On obtient 0,6 g de N-(*tert*-Butyl)-N'-[(1R)-1-hydroxyméthyl-2-(1-oxy-pyridin-4-yl)-éthyl]-thiourée sous forme de cristaux blancs [R^{f}= 0,36 dans un mélange dichlorométhane- méthanol (90/10 en volume) sur plaque d'alumine Merck 60 F₂₅₄ (type E].

Chlorhydrate de (2R)-2-amino-3-(1-oxy-pyridin-4-yl)-propan-1-ol : A une solution de 2 g de N-[(1R)-1-hydroxyméthyl-2-(1-oxy-pyridin-4-yl)-éthyl]-carbamate de *tert*-butyle dans 20 cm³ de dioxanne on ajoute sous agitation, à une température voisine de 20°C, une solution de 20 cm³ d'acide chlorhydrique 4N dans le dioxanne. Le mélange est agité à une température voisine de 20°C pendant 18 heures, puis il est repris dans 40 cm³ de dioxanne, agité 30 minutes et filtré. Le précipité est lavé par 10 cm³ de dioxanne puis par 25 cm³ d'éther isopropylique et séché au dessiccateur sous pression réduite (0,1 kPa) à une température voisine de 40°C. On obtient 1,54 g de chlorhydrate de (2R)-2-amino-3-(1-oxy-pyridin-4-yl)-propan-1-ol sous forme d'un solide jaune pâle [Spectre de R.M.N. ¹H (300 MHz, (CD₃)₂SO d6, δ en ppm) : 3,08 (d, J = 6 Hz : 2H) ; de 3,40 à 3,55 (mt : 2H) ; de 3,55 à 3,70 (mt : 1H) ; 7,78 (d, J = 6,5 Hz : 2H) ; 8,26 (s large : 3H) ; 8,71 (d, J = 6,5 Hz : 2H)].

N-[(1R)-1-Hydroxyméthyl-2-(1-oxy-pyridin-4-yl)-éthyl]-carbamate de *tert*-butyle : A une solution de 1,01 g d'acide m-chloro-per-benzoïque à 77% dans 10 cm³ de dichlorométhane on ajoute 1 g de (1R)-1-(pyridin-4-yl-méthyl)-2-hydroxyéthylcarbamate de *tert*-butyle dans 5 cm³ de dichlorométhane puis on chauffe à une température voisine de 60°C. Au bout de 45 minutes, on ajoute à nouveau 0,65 g d'acide m-chloro-per-benzoïque puis on chauffe 1 heure à une température voisine de 60°C, puis on renouvelle l'opération ci-dessus. Après 2 heures de chauffage à une température voisine de 60°C, on refroidit puis on reprend le milieu réactionnel par 50 cm³ de dichlorométhane et 50 cm³ de soude N. La phase aqueuse est concentrée sous pression réduite (1 kPa) à une température voisine de 40°C, reprise par 50 cm³ de dichlorométhane, séchée sur sulfate de magnésium, filtrée et concentrée sous pression réduite (1 kPa) à une température voisine de 40°C. On obtient 0,15 g de N-[(1R)-1-hydroxyméthyl-2-(1-oxy-pyridin-4-yl-)-éthyl]-carbamate de *tert*-butyle [Spectre de R.M.N. ¹H (300 MHz, (CD₃)₂SO d6, δ en ppm) : 1,32 (s : 9H) ; de 2,45 à 2,60 (mt : 1H) ; 2,86 (d très large, J = 13,5 Hz : 1H) ; de 3,20 à 3,45 (mt : 2H) ; 3,63 (mt : 1H) ; 4,80 (mt : 1H) ; 6,67 (d large, J = 9 Hz : 1H) ; 7,23 (d large, J = 5,5Hz : 2H) ; 8,14 (d large, J = 5,5 Hz : 2H)].

### Exemple 31

### Dichlorhydrate de (+)-4-(1,2,4-triazol-1-yl-méthyl)-4,5-dihydro-thiazol-2-ylamine

Un mélange racémique de 0,35 g de dichlorhydrate de (4RS)-4-(1,2,4)-triazol-1-ylméthyl-4,5-dihydro-thiazol-2-ylamine est séparé sur une colonne CHIRALPAK AS 20µ dans un mélange heptane-éthanol-triéthylamine (80/20/0,1 en volumes). Les fractions contenant le produit attendu sont réunies puis concentrées sous pression réduite (1 kPa), à une température voisine de 40°C pour obtenir 0,08 g de (+)-4-(1,2,4-triazol-1-yl-méthyl)-4,5-dihydro-thiazol-2-ylamine sous forme d'un solide jaune [Spectre de R.M.N. ¹H (300 MHz, (CD₃)₂SO d6, d en ppm) : 3,46 (dd, J = 11,5 et 4,5 Hz : 1H) ; 3,76 (dd, J = 11,5 et 8,5 Hz : 1H) ; 4,51 (d, J = 5 Hz : 2H) ; 4,69 (mt : 1H) ; 8,13 (s large : 1H) ; 8,68 (s large : 1H) ; 9,30 (mf: 1H) ; 9,73 (mf: 1H) ; 10,10 (s large : 1H), (a_{D}²⁰= +13,2 +/- 0,7 dans le méthanol à 0,5%)].

Dichlorhydrate de (4RS)-4-(1,2,4-triazol-1-yl-méthyl)-4,5-dihydro-thiazol-2-ylamine : Une solution de 0,3 g de N-[(4RS)-4-(1,2,4-triazol-1-yl-méthyl)-4,5-dihydro-thiazol-2-yl]-carbamate de *tert*-butyle dans 10 cm³ d'acide chlorhydrique 4N en solution dans le dioxanne est agité à une température voisine de 20°C pendant 34 heures. Le milieu réactionnel est filtré puis lavé avec 3 fois 5 cm³ d'éther diéthylique et séché au dessiccateur. On obtient 0,14 g de dichlorhydrate de (4RS)-4-(1,2,4-triazol-1-yl-méthyl)-4,5-dihydro-thiazol-2-ylamine sous forme d'une meringue blanche [Spectre de masse : EI m/z=184 MH⁺ m/z=183 M^{+.} m/z=114 C₄H₆N₂^{+.} m/z=101 C₃H₅N₂S⁺ pic de base m/z=36 HCl DCI m/z=184 MH⁺].

N-[(4RS)-4-(1,2,4-Triazol-1-yl-méthyl)-4,5-dihydro-thiazol-2-yl]-carbamate de *tert*-butyle : A une solution de 0,35 g d'hydrure de sodium dans 7 cm³ de diméthylsulfoxyde on ajoute à une température voisine de 20°C et sous atmosphère inerte 0,6 g de 1,2,4 triazole. Le mélange est agité à une température voisine de 20°C pendant 45 minutes puis on ajoute 1 g de N-[(4RS)-4-(iodométhyl)-4,5-dihydro-thiazol-2-yl]-carbamate de *tert*-butyle. Après 3 heures d'agitation à une température voisine de 60°C on ajoute au milieu réactionnel 30 cm³ d'une solution saturée de chlorure d'ammonium et on extrait avec 4 fois 10 cm³ d'acétate d'éthyle. Les phases organiques sont lavées avec 10 cm³ d'une solution saturée de chlorure de sodium, séchées sur sulfate de magnésium, filtrées puis sont concentrées sous pression réduite (1 kPa), à une température voisine de 40°C. Le résidu est chromatographié sous une pression d'argon de 70 kPa, sur une colonne de gel de silice (granulométrie 40-63µ ; diamètre 4 cm ; hauteur 18 cm), en éluant par un mélange de dichlorométhane-méthanol (5/1 en volumes). Les fractions contenant le produit attendu sont réunies puis concentrées dans les conditions de ci-dessus. On obtient 0,34 g de N-[(4RS)-4-(1,2,4-triazol-1-yl-méthyl)-4,5-dihydro-thiazol-2-yl]-carbamate de *tert*-butyle sous forme d'une mousse blanche [Spectre de masse : EI m/z=283 M^{+.} m/z=201 C₈H₁₃N₂O₂S⁺ m/z=145 C₄H₅N₂O₂S⁺ m/z=101 C₃H₅N₂S⁺ m/z=82 C₃H₄N₃⁺ m/z=57 C₄H₉⁺].

N-[(4RS)-4-(Iodométhyl)-4,5-dihydro-thiazol-2-yl]-carbamate de *tert*-butyle : A une suspension de 25,4 g d'allylsulfanyl-[(*tert*-butoxycarbonyl)amino]-méthylidène carbamate de *tert*-butyle dans 650 cm³ de dichlorométhane on ajoute à une température voisine de 20°C, 17 g de bicarbonate de sodium puis une solution de 24,4 g d'iode préalablement dissout dans 850 cm³ de dichlorométhane. Après 72 heures à une température voisine de 20°C on ajoute au mélange réactionnel 500 cm³ d'eau et 500 cm³ d'une solution saturée de bicarbonate de sodium puis on extrait avec 2 fois 1 dm³ d'acétate d'éthyle. Les phases organiques sont lavées avec une solution saturée de sulfite de sodium puis une solution saturée de chlorure de sodium, séchées sur sulfate de magnésium, filtrées puis sont concentrées sous pression réduite (1 kPa) à une température voisine de 50°C. Le résidu obtenu est cristallisé dans de l'acétate d'éthyle. On obtient 20,5 g de N-[(4RS)-4-(iodométhyl)-4,5-dihydro-thiazol-2-yl]-carbamate de *tert*-butyle sous forme d'un solide jaune [Spectre de R.M.N. ¹H (300 MHz, (CD₃)₂SO d6, δ en ppm) : 1,41 (s : 9H) ; 2,97 (dd, J = 11,5 et 7 Hz : 1H) ; de 3,30 à 3,50 (mt : 3H) ; 4,12 (mt : 1H) ; 9,89 (mf : 1H].

Allylsulfanyl-[(*tert*-butoxycarbonyl)amino]-méthylidène carbamate de *tert*-butyle : A une solution de 5 g de chlorhydrate de 2-allyl-isothiourée dans 50 cm³ de dichlorométhane on ajoute une quantité catalytique de 4-(diméthyl-amino)-pyridine et 4,7 cm³ de triéthylamine puis on coule goutte à goutte une solution de 7,1 g de di-*tert*-butyldicarbonate préalablement dissous dans 30 cm³ de dichlorométhane. Au bout de 48 heures le milieu réactionnel est concentré sous pression réduite (1 kPa), à une température voisine de 40°C. Le résidu est chromatographié sous une pression d'argon de 70 kPa, sur une colonne de gel de silice (granulométrie 40-63µ ; diamètre 4 cm ; hauteur environ 30 cm), en éluant par un mélange d'acétate d'éthyle-cyclohexane (90/10 en volumes). Les fractions contenant le produit attendu sont réunies puis concentrées dans les conditions de ci-dessus. On obtient 0,2 g d'allylsulfanyl-[(*tert*-butoxycarbonyl)amino]-méthylidène carbamate de *tert*-butyle [Spectre de masse: DCI m/z=317 MH⁺ m/z=261 C₁₀H₁₇O₄N₂S⁺ m/z=217 C₉H₁₇O₂N₂S⁺ m/z=161 C₅H₉O₂N₂S⁺].

Chlorhydrate de 2-allyl-isothiourée : A une suspension de 15 g de thiourée dans 120 cm³ d'éthanol on ajoute à une température voisine de 20°C, 16 cm³ de chlorure d'allyle. Après 15 heures à une température voisine de 80°C le milieu réactionnel est concentré sous pression réduite (1 kPa) à une température voisine de 50°C. Le solide obtenu est repris par 3 fois 100 cm³ d'éther diéthylique puis filtré. On obtient 29 g de chlorhydrate de 2-allyl-isothiourée sous forme d'un solide blanc [Spectre de masse : DCI m/z=117 MH⁺].

### Exemple 32

### (+)-(4R,5R)-5-Ethyl-4-(pyridin-4-yl-méthyl)-4,5-dihydro-thiazol-2-ylamine

Une suspension de 4,2 g de N-*tert*-butyl-N'-[(1R,2S)-1-(pyridin-4-yl-méthyl)-2-hydroxy-butyl]-thiourée dans 40 cm³ d'acide chlorhydrique 6N est chauffée à une température voisine de 100°C pendant 20 heures. Après refroidissement à température ambiante le mélange réactionnel est concentré sous pression réduite (1 kPa) à une température voisine de 50°C. Le résidu est chromatographié sous pression atmosphérique, sur une colonne de gel de silice (granulométrie 40-63µ ; diamètre 3,6 cm ; hauteur 24 cm), en éluant avec un mélange de dichlorométhane-méthanol (98/2 en volumes) puis un mélange de dichlorométhane-méthanol (97/3 en volumes). Les fractions contenant le produit attendu sont réunies puis concentrées dans les conditions ci-dessus, reprises par 30 cm³ de dichlorométhane, alcalinisées par de la soude N. La phase organique est séchée sur sulfate de magnésium, filtrée et séchée au dessiccateur sous pression réduite (0,1 kPa) à une température voisine de 40°C. On obtient 1,4 g de (+)-(4R,5R)-5-éthyl-4-(pyridin-4-yl-méthyl)-4,5-dihydro-thiazol-2-ylamine sous forme d'un solide blanc fondant à 124°C [Spectre de R.M.N. ¹H (300 MHz, (CD₃)₂SO d6, δ en ppm) : 0,83 (t, J = 7,5 Hz : 3H) ; 1,43 (mt : 1H) ; 1,61 (mt : 1H) ; 2,70 (AB limite : 2H) ; 3,40 (mt : 1H) ; 4,08 (mt : 1H) ; 6,28 (mf : 2H) ; 7,29 (d large, J = 5,5 Hz : 2H) ; 8,45 (d large, J = 5,5 Hz : 2H), (a_{D}²⁰= +166,9 +/- 2,4 dans le méthanol à 0,5%].

N-*tert*-Butyl-N'-[(1R,2S)-1-(pyridin-4-yl-méthyl)-2-hydroxy-butyl]-thiourée : A une solution de 3,6 g de dichlorhydrate de (2R,3S)-2-amino-1-(pyridin-4-yl)-pentan-3-ol dans 60 cm³ d'éthanol on ajoute 4,3 cm³ de triéthylamine puis 2,6 cm³ de *tert*-butylisothiocyanate. Le mélange est chauffé à une température voisine de 50°C pendant 18 heures. Après refroidissement le milieu réactionnel est concentré sous pression réduite (1 kPa) à une température voisine de 40°C, repris par 50 cm³ d'eau et 100 cm³ de dichlorométhane. La phase organique est lavée par 30 cm³ d'eau, séchée sur sulfate de sodium, filtrée et concentrée dans les conditions de ci-dessus. On obtient 4,3 g de N-*tert*-butyl-N'-[(1R,2S)-1-(pyridin-4-yl-méthyl)-2-hydroxy-butyl]-thiourée sous forme d'une huile épaisse incolore [Spectre de R.M.N. ¹H (300 MHz, (CD₃)₂SO d6, δ en ppm) : 0,80 (t, J = 7,5 Hz : 3H) ; de 1,25 à 1,55 (mt : 2H) ; 1,37 (s : 9H) ; 2,72 (dd, J = 14 et 9 Hz : 1H) ; 2,93 (dd, J = 14 et 4,5 Hz : 1H) ; 3,37 (mt : 1H) ; 4,60 (mt : 1H) ; 4,88 (mf : 1H) ; 7,11 (s : 1H) ; 7,16 (d, J = 8,5 Hz : 1H) ; 7,26 (d large, J = 5,5 Hz : 2H) ; 8,43 (d large, J = 5,5 Hz : 2H)].

Dichlorhydrate de (2R,3S)-2-amino-1-(pyridin-4-yl)-pentan-3-ol : A une solution de 4 g de N-[(1R,2S)-2-hydroxy-1-(pyridin-4-yl-méthyl)-butyl]-carbamate de *tert*-butyle 50 cm³ de dioxanne on ajoute sous agitation, à une température voisine de 20°C, une solution de 50 cm³ d'acide chlorhydrique 4N dans le dioxanne. Le mélange est agité à une température voisine de 20°C pendant 18 heures puis concentré sous pression réduite (1 kPa) à une température voisine de 60°C. On obtient 3,6 g de dichlorhydrate de (2R,3S)-2-amino-1-(pyridin-4-yl)-pentan-3-ol sous forme d'un solide blanc [Spectre de R.M.N. ¹H (300 MHz, (CD₃)₂SO d6, δ en ppm) : 0,92 (t, J = 7,5 Hz : 3H) ; de 1,30 à 1,65 (mt : 2H) ; 3,13 (dd, J = 14 et 9 Hz : 1H) ; 3,28 (dd, J = 14 et 5 Hz : 1H) ; 3,52 (mt : 1H) ; 3,68 (mt : 1H) ; 8,11 (d large, J = 6,5 Hz : 2H) ; 8,25 (mf : 3H) ; 8,90 (d large, J = 6,5 Hz : 2H].

N-[(1R,2S)-2-Hydroxy-1-(pyridin-4-yl-méthyl)-butyl]-carbamate de *tert*-butyle : 9,5 g d'un mélange de 85%15% des deux diastéréoisomères du N-[(2RS)-2-hydroxy-(1R)-1-(pyridin-4-yl-méthyl)-butyl]-carbamate de *tert*-butyle est séparé sur une colonne KROMASIL®10µ C8 dans un mélange acétonitrile-méthanol-tétrahydrofuranne-eau (15/15/5/65 en volumes). Les fractions contenant le produit attendu sont concentrées sous pression réduite (1 kPa), à une température voisine de 40°C pour obtenir 4 g de dichlorhydrate de N-[(1R,2S)-2-hydroxy-1-(pyridin-4-yl-méthyl)-butyl]-carbamate de *tert*-butyle sous forme d'un solide blanc [Spectre de R.M.N. ¹H (300 MHz, (CD₃)₂SO d6, δ en ppm) : 0,91 (t, J = 7,5 Hz : 3H) ; 1,26 (s : 9H) ; 1,30 (mt : 1H) ; 1,52 (mt : 1H) ; de 2,45 à 2,60 (mt : 1H) ; 3,02 (dd, J = 14 et 3,5 Hz : 1H) ; 3,28 (mt : 1H) ; 3,53 (mt : 1 H) ; 4,71 (d, J = 6 Hz : 1H) ; 6,62 (d, J = 9,5 Hz : 1H) ; 7,20 (d large, J = 5,5 Hz : 2H) ; 8,42 (d large, J = 5,5 Hz : 2H].

N-[(2RS)-2-Hydroxy-(1R)-1-(pyridin-4-yl-méthyl)-butyl]-carbamate de *tert*-butyle : A une solution de 10,5 g de N-[(1R)-2-oxobutyl -1-(pyridin-4-yl-méthyl)]-carbamate de *tert*-butyle dans 150 cm³ d'éthanol, sous agitation et à une température voisine de 10°C on ajoute 2,1 g de borohydrure de sodium puis, on agite à une température voisine de 20°C pendant 18 heures. Le milieu réactionnel est concentré sous pression réduite (1 kPa), à une température voisine de 40°C, repris par 100 cm³ d'eau, extrait par 200 cm³ d'acétate d'éthyle. La phase organique est séchée sur sulfate de magnésium, filtrée et concentrée sous pression réduite (1 kPa), à une température voisine de 40°C. On obtient 9,5 g d'un mélange de 85%15% des deux diastéréoisomères du N-[(2RS)-2-hydroxy-(1R)-1-(pyridin-4-yl-méthyl)-butyl]-carbamate de *tert*-butyle sous forme d'une gomme jaune [Spectre de R.M.N. ¹H (300 MHz, (CD₃)₂SO d6, δ en ppm). Nous observons un mélange de deux diastéréoisomères dans les proportions 85-15 ; δ =de 0,85 à 0,95 (mt : 3H) ; de 1,25 à 1,60 (mt : 2H) ; 1,26 et 1,29 (2 s : 9H en totalité) ; de 2,45 à 2,60 (mt : 0,85H) ; 2,67 (dd, J = 14 et 10 Hz : 0,15H) ; 2,80 (dd, J = 14 et 4,5 Hz : 0,15H) ; 3,02 (dd, J = 14 et 4,5 Hz : 0,85H) ; de 3,20 à 3,35 (mt : 1H) ; 3,52 (mt : 0,85H) ; 3,69 (mt : 0,15H) ; 4,61 (d, J = 6 Hz : 0,15H) ; 4,71 (d, J = 6 Hz : 0,85H) ; 6,41 (d, J = 9,5 Hz : 0,15H) ; 6,62 (d, J = 9,5 Hz : 0,85H) ; 7,20 (d, J = 6 Hz : 1,7H) ; 7,23 (d, J = 6 Hz : 0,3H) ; de 8,35 à 8,50 (mt : 2H en totalité)].

N-[(1R)-2-Oxo-1-(pyridin-4-yl-méthyl)-butyl]-carbamate de *tert*-butyle : A un mélange, sous atmosphère inerte, de 14 g de N-{2-[N-méthoxy-N-(méthyl)amino]-2-oxo-(1R)-1-(pyridin-4-yl-méthyl)-éthyl}-carbamate de *tert*-butyle dans 350 cm³ de tétrahydrofuranne, refroidi à une température voisine de 0°C, on ajoute en 1 heure, 50 cm³ d'une solution de bromure d'éthylmagnésium 3N en solution dans l'éther diéthylique. Après une agitation de 48 heures à 0°C on ajoute de l'eau au milieu réactionnel, on extrait avec du dichlorométhane et on lave avec de l'eau. La phase organique est séchée sur sulfate de sodium, filtrée puis concentrée sous pression réduite (2 kPa) à une température voisine de 40°C. On obtient 10,5 g de N-[(1R)-2-oxo-1-(pyridin-4-yl-méthyl)-butyl]-carbamate de *tert*-butyle sous forme d'huile de couleur jaune [Spectre de R.M.N. ¹H (300 MHz, (CD₃)₂SO d6, δ en ppm) : 0,94 (t, J = 7 Hz : 3H) ; 1,32 (s : 9H) ; de 2,40 à 2,65 (mt : 2H) ; 2,70 (dd, J = 14 et 10,5 Hz : 1H) ; 3,06 (dd, J = 14 et 4,5 Hz : 1H) ; 4,22 (mt : 1H) ; 7,26 (d large, J = 5,5 Hz : 2H) ; 7,44 (d large, J = 8 Hz : 1H) ; 8,45 (d large, J = 5,5 Hz : 2H].

### Exemple 33

### Chlorhydrate de (4R)-4-(thién-2-yl-méthyl)-4,5-dihydro-thiazol-2-ylamine

A une solution de 2,5 g (9,23 mmol) de Boc-D-2-thiénylalanine et de 1,3 cm³ (9,4 mmol) de triéthylamine dans 100 cm³ de tétrahydrofuranne anhydre à -20°C est ajouté au goutte à goutte 0,9 cm³ (9,4 mmol) de chloroformiate d'éthyle. La suspension résultante est agitée à -20°C pendant 1 heure puis filtrée. Le filtrat est refroidi à -20°C, puis une solution de 0,71 g (20,9 mmol) de borohydrure de sodium dans 5 cm³ d'eau est ajoutée. Le mélange réactionnel est agité à une température voisine de 20°C pendant 64 heures, puis il est concentré sous vide pour donner un résidu qui est utilisé directement sans purification. Une suspension du produit obtenu dans 20 cm³ de dioxanne est traitée avec 6 cm³ d'une solution 4M d'acide chlorhydrique dans le dioxanne, puis agitée à une température voisine de 20°C pendant 16 heures. Après concentration du milieu réactionnel sous vide, on obtient 7,5 g d'un solide blanc qui est utilisé directement sans purification.

Une suspension du produit ci-dessus (1,0 g), de triéthylamine (4 cm³, 28,7 mmol) et de tert-butyl-isothiocyanate (0,50 g, 4,3 mmol) dans 10 cm³ d'éthanol est chauffée sous agitation à une température voisine de 50°C pendant 4 heures. Après concentration sous vide, le résidu est repris 2 fois dans 50 cm³ d'acétate d'éthyle, puis purifié par chromatographie sur une colonne de gel de silice (acétate d'éthyle/cyclohexane 1 :1 en volumes). Les fractions contenant le produit attendu sont concentrées sous pression réduite pour donner 255 mg d'un solide blanc qui est utilisé directement.

Une suspension du produit obtenu précédemment (240 mg, 0,88 mmol) est chauffée au reflux dans 6 cm³ d'une solution d'acide chlorhydrique 6N pendant 8 heures. Le mélange réactionnel est concentré à sec sous vide et le résidu brun est trituré dans un mélange d'éthanol (5 cm³) et d'acétate d'éthyle (3 cm³), puis filtré. Cette opération de trituration est répétée 3 fois, jusqu'à ce qu'un solution jaune clair soit obtenue. Après concentration partielle, le produit cristallise et est filtré puis séché sous vide pour donner 64 mg de chlorhydrate de (4R)-4-(thién-2-yl-méthyl)-4,5-dihydro-thiazol-2-ylamine sous forme de cristaux jaune clair fondant à 125-127°C [Spectre de RMN ¹H (300 MHz, (CD₃)₂SO d6, δ en ppm): 10.1 (bs,1H), 9.7 (bs, 1H), 9.3 (bs, 1H), 7.4 (m, 1H), 7.0 (m, 2H), 4.5 (m, 1H), 3.6 (m,1H), 3.3 (m, 1H), 3.2 (m, 2H].

Les compositions pharmaceutiques selon l'invention sont constitués par un composé de formule (I) ou un isomère ou un tautomère ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops ets élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles pour le traitement et/ou la prévention de la sclérose en plaques, l'ischémie cérébrale focale ou globale, les traumatismes cérébraux ou spinaux, la maladie de Parkinson, la maladie de Huntington, la maladie d'Alzheimer, la sclérose latérale amyotrophique, la migraine, la dépression, la schizophrénie, l'anxiété, l'épilepsie, le diabète, l'athérosclérose, la myocardite, l'arthrite, l'arthrose, l'asthme, le syndrome du colon irritable, la maladie de Crohn, la péritonite, le reflux gastro-oesophagien, l'uvéite, le syndrome de Guillain-Barré, la glomérulo-néphrite, le lupus érythémateux, le psoriasis, la croissance de certaines formes de tumeurs comme par exemple les épithéliomes, les adénocarcinomes ou les sarcomes, et les infections par des bactéries intracellulaires ou extracellulaires, Gram-plus ou Gram-moins.

Les doses dépendent de l'effet recherché, de la durée du traitement et de la voie d'administration utilisée; elles sont généralement comprises entre 1 mg et 100 mg par jour par voie orale pour un adulte avec des doses unitaires allant de 0,5 mg à 50 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

La présente invention concerne également la méthode de prévention et de traitement des maladies dans lesquelles une production anormale de monoxyde d'azote (NO) par induction de NO-synthase inductible (NOS-2 ou iNOS) est impliquée par administration d'un composé de formule (I) ses racémiques, énantiomères, diastéréoisomères et leurs mélanges, son tautomère et ses sels pharmaceutiquement acceptables.

## Revendications

1. Utilisation des dérivés de 2-aminothiazoline de formule : 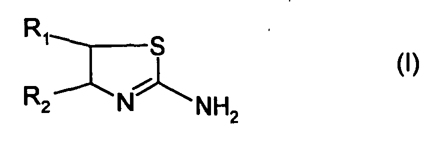 dans laquelle
soit R₁ est un atome d'hydrogène ou un radical alkyle et R₂ est un radical alkyle, -alk-NH₂, -CH₂-R₃, -CH₂-S-R₄ ou phényle substitué par un radical nitro ou -NH-C(=NH)CH₃,
soit R₁ est un radical alkyle et R₂ est un atome d'hydrogène,
R₃ est un radical cycloalkyle (3-6C), pyridyle, N-oxyde de pyridyle, thiényle, thiazolyle, imidazolyle, pyrazinyle, triazolyle, phényle ou phényle substitué par un radical nitro, hydroxy ou carboxy,
R₄ représente un radical pyridyle ou N-oxyde de pyridyle
alk représente un radical alkylène
étant entendu que les radicaux alkyle et alkylène contiennent 1 à 6 atomes de carbone en chaîne droite ou ramifiée,
leurs racémiques, énantiomères, diastéréoisomères et leurs mélanges, leurs tautomères et leurs sels pharmaceutiquement acceptables pour la préparation de compositions pharmaceutiques destinées à prévenir et traiter les maladies dans lesquelles une production anormale de monoxyde d'azote (NO) par induction de NO-synthase inductible (NOS-2) est impliquée.

2. Utilisation selon la revendication 1 **caractérisée en ce que** dans la formule (I) R₁ est un atome d'hydrogène ou un radical alkyle et R₂ est un radical -alk-NH₂, phényle substitué par un radical -NH-C(=NH)CH₃, -CH₂-R₃ et R₃ est un radical pyridyle, thiényle, thiazolyle, imidazolyle, pyrazinyle, triazolyle, phényle ou phényle substitué par un radical nitro ou carboxy ou -CH₂-S-R₄ et R₄ est un radical pyridyle.

3. Utilisation selon l'une des revendications 1 ou 2 **caractérisée en ce que** R₂ est une chaîne -CH₂-R₃ ou -CH₂-S-R₄, R₃ est un radical 3- ou 4-pyridyle, 2- ou 3-thiényle, 4-ou 5-thiazolyle, 1-imidazolyle, 1-triazolyle, 2-pyrazinyle, phényle ou phényle substitué en position -3 par un radical nitro ou carboxy et R₄ est un radical 4-pyridyle.

4. Utilisation selon la revendication 1 **caractérisée en ce que** le composé de formule (I) est choisi parmi les composés suivants :
4-(3-pyridylméthyl)-4,5-dihydro-1,3-thiazol-2-ylamine
4-(3-nitrobenzyl)-4,5-dihydro-1,3-thiazol-2-ylamine
4-benzyl-5-méthyl-4,5-dihydro-1,3-thiazol-2-ylamine
4-(3-thiénylméthyl)-4,5-dihydro-1,3-thiazol-2-ylamine
4-(2-thiénylméthyl)-4,5-dihydro-1,3-thiazol-2-ylamine
[3-(2-amino-4,5-dihydro-thiazol-4-yl)-phényl]-(1-imino-éthyl)-amine
4-benzyl-4,5-dihydro-1,3-thiazol-2-ylamine
4-(3-carboxybenzyl)-4,5-dihydro-1,3-thiazol-2-ylamine
4-(4-aminobutyl)-4,5-dihydro-1,3-thiazol-2-ylamine
4-butyl-4,5-dihydro-1,3-thiazol-2-ylamine
5-méthyl-4,5-dihydro-1,3-thiazol-2-ylamine
4-cyclohexylméthyl-4,5-dihydro-1,3-thiazol-2-ylamine
4-(3-nitrophényl)-4,5-dihydro-1,3-thiazol-2-ylamine
4-(4-pyridylméthyl)-4,5-dihydro-1,3-thiazol-2-ylamine
5-méthyl-4-(4-pyridylméthyl)-4,5-dihydro-1,3-thiazol-2-ylamine
5-éthyl-4-(4-pyridylméthyl)-4,5-dihydro-1,3-thiazol-2-ylamine
4-(4-thiazolylméthyl)-4,5-dihydro-1,3-thiazol-2-ylamine
4-(1-imidazolylméthyl)-4,5-dihydro-1,3-thiazol-2-ylamine
4-(2-pyrazinylméthyl)-4,5-dihydro-1,3-thiazol-2-ylamine
4-(5-thiazolylméthyl)-4,5-dihydro-1,3-thiazol-2-ylamine
4-(4-hydroxybenzyl)-4,5-dihydro-1,3-thiazol-2-ylamine
4-(4-pyridylsulfanylméthyl)-4,5-dihydro-1,3-thiazol-2-ylamine
4-(3-aminopropyl)-4,5-dihydro-1,3-thiazol-2-ylamine
4-(1-triazolylméthyl) -4,5-dihydro-1,3-thiazol-2-ylamine
N-oxyde de 4-(4-pyridylméthyl)-4,5-dihydro-1,3-thiazol-2-ylamine
leurs racémiques, énantiomères, diastéréoisomères et leurs mélanges, leurs tautomères et leurs sels pharmaceutiquement acceptables.

5. Utilisation selon la revendication 1 **caractérisée en ce que** le composé de formule (I) est choisi parmi les composés suivants :
(+)-(4R)-4-(3-pyridylméthyl)-4,5-dihydro-1,3-thiazol-2-ylamine
(+)-(4R)-4-(3-nitrobenzyl)-4,5-dihydro-1,3-thiazol-2-ylamine
(+)-(4R,5S)-4-benzyl-5-méthyl-4,5-dihydro-1,3-thiazol-2-ylamine
(+)-(4R,5R)-4-benzyl-5-méthyl-4,5-dihydro-1,3-thiazol-2-ylamine
(-)-(4R)-4-(3-thiénylméthyl)-4,5-dihydro-1,3-thiazol-2-ylamine
(4R)-4-(2-thiénylméthyl)-4,5-dihydro-1,3-thiazol-2-ylamine
[3-(2-amino-4,5-dihydro-thiazol-4-yl)-phényl]-(1-imino-éthyl)-amine
(+)-(4R)-4-benzyl-4,5-dihydro-1,3-thiazol-2-ylamine
(+)-(4R)-4-(3-carboxybenzyl)-4,5-dihydro-1,3-thiazol-2-ylamine
(+)-(4R)-4-(4-aminobutyl)-4,5-dihydro-1,3-thiazol-2-ylamine
(-)-(4S)-4-(3-nitrobenzyl)-4,5-dihydro-1,3-thiazol-2-ylamine
(-)-(4S,5S)-4-benzyl-5-méthyl-4,5-dihydro-1,3-thiazol-2-ylamine
(-)-(4S)-4-(4-aminobutyl)-4,5-dihydro-1,3-thiazol-2-ylamine
(4S,5R)-4-benzyl-5-méthyl-4,5-dihydro-1,3-thiazol-2-ylamine
(-)-(4R)-4-butyl-4,5-dihydro-1,3-thiazol-2-ylamine
(+)-(5S) -5-méthyl-4,5-dihydro-1,3-thiazol-2-ylamine
(-)-(4S)-4-cyclohexylméthyl-4,5-dihydro-1,3-thiazol-2-ylamine
(+)-(4R)-4-cyclohexylméthyl-4,5-dihydro-1,3-thiazol-2-ylamine
4-(3-nitrophényl)-4,5-dihydro-1,3-thiazol-2-ylamine
(+)-(4R)-4-(4-pyridylméthyl)-4,5-dihydro-1,3-thiazol-2-ylamine
(+)-(4R,5R)-5-méthyl-4-(4-pyridylméthyl)-4,5-dihydro-1,3-thiazol-2-ylamine
(+)-(4R,5R)-5-éthyl-4-(4-pyridylméthyl)-4,5-dihydro-1,3-thiazol-2-ylamine
(+)-4-(5-thiazolylméthyl)-4,5-dihydro-1,3-thiazol-2-ylamine
(-)-4-(5-thiazolylméthyl)-4,5-dihydro-1,3-thiazol-2-ylamine
(+)-(4R)-4-(2-pyrazinylméthyl)-4,5-dihydro-1,3-thiazol-2-ylamine
(4R)-4-(1-imidazolylméthyl)-4,5-dihydro-1,3-thiazol-2-ylamine
(4S)-4-(1-imidazolylméthyl)-4,5-dihydro-1,3-thiazol-2-ylamine
(+)-(4R)-4-(4-thiazolylméthyl)-4,5-dihydro-1,3-thiazol-2-ylamine
(+)-(4R)-4-(3-aminopropyl)-4,5-dihydro-1,3-thiazol-2-ylamine
(+)-(4R)-4-(4-hydroxybenzyl)-4,5-dihydro-1,3-thiazol-2-ylamine
(+)-4-(4-pyridylsulfanylméthyl)-4,5-dihydro-1,3-thiazol-2-ylamine
N-oxyde de (4R)-4-(4-pyridylméthyl)-4,5-dihydro-1,3-thiazol-2-ylamine
(+)-4-(1-triazolylméthyl) -4,5-dihydro-1,3-thiazol-2-ylamine
leurs tautomères ainsi que leurs sels pharmaceutiquement acceptables.

6. Utilisation selon la revendication 2 **caractérisée en ce que** le composé de formule (I) est choisi parmi les composés suivants :
4-(3-pyridylméthyl)-4,5-dihydro-1,3-thiazol-2-ylamine
4-(3-nitrobenzyl)-4,5-dihydro-1,3-thiazol-2-ylamine
4-benzyl-5-méthyl-4,5-dihydro-1,3-thiazol-2-ylamine
4-(3-thiénylméthyl)-4,5-dihydro-1,3-thiazol-2-ylamine
4-(2-thiénylméthyl)-4,5-dihydro-1,3-thiazol-2-ylamine
[3-(2-amino-4,5-dihydro-thiazol-4-yl)-phényl]-(1-imino-éthyl)-amine
4-benzyl-4,5-dihydro-1,3-thiazol-2-ylamine
4-(3-carboxybenzyl)-4,5-dihydro-1,3-thiazol-2-ylamine
4-(4-aminobutyl)-4,5-dihydro-1,3-thiazol-2-ylamine
4-(4-pyridylméthyl)-4,5-dihydro-1,3-thiazol-2-ylamine
5-méthyl-4-(4-pyridylméthyl)-4,5-dihydro-1,3-thiazol-2-ylamine
5-éthyl-4-(4-pyridylméthyl)-4,5-dihydro-1,3-thiazol-2-ylamine
4-(4-thiazolylméthyl)-4,5-dihydro-1,3-thiazol-2-ylamine
4-(1-imidazolylméthyl)-4,5-dihydro-1,3-thiazol-2-ylamine
4-(2-pyrazinylméthyl)-4,5-dihydro-1,3-thiazol-2-ylamine
4-(5-thiazolylméthyl)-4,5-dihydro-1,3-thiazol-2-ylamine
4-(1-triazolylméthyl)-4,5-dihydro-1,3-thiazol-2-ylamine
4-(4-pyridylsulfanylméthyl)-4,5-dihydro-1,3-thiazol-2-ylamine
leurs racémiques, énantiomères, diastéréoisomères et leurs mélanges, leurs tautomères et leurs sels pharmaceutiquement acceptables,

7. Utilisation selon la revendication 2 **caractérisée en ce que** le composé de formule (I) est choisi parmi les composés suivants :
(+)-(4R)-4-(3-pyridylméthyl)-4,5-dihydro-1,3-thiazol-2-ylamine
(+)-(4R)-4-(3-nitrobenzyl)-4,5-dihydro-1,3-thiazol-2-ylamine
(+)-(4R,5S)-4-benzyl-5-méthyl-4,5-dihydro-1,3-thiazol-2-ylamine
(+)-(4R,5R)-4-benzyl-5-méthyl-4,5-dihydro-1,3-thiazol-2-ylamine
(-)-(4R)-4-(3-thiénylméthyl)-4,5-dihydro-1,3-thiazol-2-ylamine
(4R)-4-(2-thiénylméthyl)-4,5-dihydro-1,3-thiazol-2-ylamine
[3-(2-amino-4,5-dihydro-thiazol-4-yl)-phényl]-(1-imino-éthyl)-amine
(+)-(4R)-4-benzyl-4,5-dihydro-1,3-thiazol-2-ylamine
(+)-(4R)-4-(3-carboxybenzyl)-4,5-dihydro-1,3-thiazol-2-ylamine
(+)-(4R)-4-(4-aminobutyl)-4,5-dihydro-1,3-thiazol-2-ylamine
(-)-(4S)-4-(3-nitrobenzyl)-4,5-dihydro-1,3-thiazol-2-ylamine
(-)-(4S,5S)-4-benzyl-5-méthyl-4,5-dihydro-1,3-thiazol-2-ylamine
(-)-(4S)-4-(4-aminobutyl)-4,5-dihydro-1,3-thiazol-2-ylamine
(4S,5R)-4-benzyl-5-méthyl-4,5-dihydro-1,3-thiazol-2-ylamine
(+)-(4R)-4-(4-pyridylméthyl)-4,5-dihydro-1,3-thiazol-2-ylamine
(+)-(4R,5R)-5-méthyl-4-(4-pyridylméthyl)-4,5-dihydro-1,3-thiazol-2-ylamine
(+)-(4R,5R)-5-éthyl-4-(4-pyridylméthyl)-4,5-dihydro-1,3-thiazol-2-ylamine
(+)-4-(5-thiazolylméthyl)-4,5-dihydro-1,3-thiazol-2-ylamine
(-)-4-(5-thiazolylméthyl)-4,5-dihydro-1,3-thiazol-2-ylamine
(+)-(4R)-4-(2-pyrazinylméthyl)-4,5-dihydro-1,3-thiazol-2-ylamine
(4R)-4-(1-imidazolylméthyl)-4,5-dihydro-1,3-thiazol-2-ylamine
(4S)-4-(1-imidazolylméthyl)-4,5-dihydro-1,3-thiazol-2-ylamine
(+)-(4R)-4-(4-thiazolylméthyl)-4,5-dihydro-1,3-thiazol-2-ylamine
(+)-4-(4-pyridylsulfanylméthyl)-4,5-dihydro-1,3-thiazol-2-ylamine
(+)-4-(1-triazolylméthyl) -4,5-dihydro-1,3-thiazol-2-ylamine
leurs tautomères ainsi que leurs sels pharmaceutiquement acceptables.

8. Composés de formule : 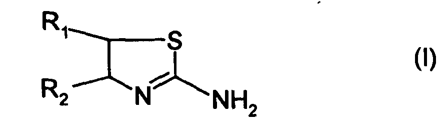 dans laquelle
soit R₁ est un atome d'hydrogène ou un radical alkyle et R₂ est un radical alkyle, -alk-NH₂, -CH₂-R₃, -CH₂-S-R₄ ou phényle substitué par un radical nitro ou -NH-C(=NH)CH₃,
soit R₁ est un radical alkyle et R₂ est un atome d'hydrogène,
R₃ est un radical cycloalkyle (3-6C), pyridyle, thiényle, thiazolyle, imidazolyle, pyrazinyle, triazolyle, phényle ou phényle substitué par un radical nitro, hydroxy ou carboxy,
R₄ représente un radical pyridyle,
alk représente un radical alkylène,
leurs racémiques, énantiomères, diastéréoisomères et leurs mélanges, leurs tautomères et leurs sels pharmaceutiquement acceptables
à l'exception des composés pour lesquels R₁ est hexyle ou méthyle et R₂ est hydrogène ou bien R₁ et R₂ sont méthyle et les racémiques des composés pour lesquels R₁ est hydrogène et R₂ est méthyle, éthyle ou n-propyle,
étant entendu que les radicaux alkyle et alkylène contiennent 1 à 6 atomes de carbone en chaîne droite ou ramifiée.

9. Composés selon la revendication 8 pour lesquels R₁ est un atome d'hydrogène ou un radical alkyle et R₂ est un radical -alk-NH₂, phényle substitué par un radical -NH-C(=NH)CH₃, -CH₂-R₃ pour lequel R₃ est un radical pyridyle, thiényle, thiazolyle, imidazolyle, triazolyle, pyrazinyle, phényle ou phényle substitué par un radical nitro ou carboxy ou -CH₂-S-R₄ pour lequel R₄ est un radical pyridyle, étant entendu que les radicaux alkyle et alkylène contiennent 1 à 6 atomes de carbone en chaîne droite ou ramifiée,
leurs racémiques, énantiomères, diastéréoisomères et leurs mélanges, leurs tautomères et leurs sels pharmaceutiquement acceptables.

10. Composés selon l'une des revendications 8 ou 9 **caractérisés en ce que** R₃ est un radical 3- ou 4-pyridyle, 2- ou 3-thiényle, 4- ou 5-thiazolyle, 1-imidazolyle, 1-triazolyle, 2-pyrazinyle, phényle ou phényle substitué en position -3 par un radical nitro ou carboxy et R₄ est un radical 4-pyridyle.

11. Composés selon la revendication 8 choisis parmi les suivants :
4-(3-pyridylméthyl)-4,5-dihydro-1,3-thiazol-2-ylamine
4-(3-nitrobenzyl)-4,5-dihydro-1,3-thiazol-2-ylamine
4-benzyl-5-méthyl-4,5-dihydro-1,3-thiazol-2-ylamine
4-(3-thiénylméthyl)-4,5-dihydro-1,3-thiazol-2-ylamine
4-(2-thiénylméthyl)-4,5-dihydro-1,3-thiazol-2-ylamine
[3-(2-amino-4,5-dihydro-thiazol-4-yl)-phényl]-(1-imino-éthyl)-amine
4-benzyl-4,5-dihydro-1,3-thiazol-2-ylamine
4-(3-carboxybenzyl)-4,5-dihydro-1,3-thiazol-2-ylamine
4-(4-aminobutyl)-4,5-dihydro-1,3-thiazol-2-ylamine
4-(4-pyridylméthyl)-4,5-dihydro-1,3-thiazol-2-ylamine
5-méthyl-4-(4-pyridylméthyl)-4,5-dihydro-1,3-thiazol-2-ylamine
5-éthyl-4-(4-pyridylméthyl)-4,5-dihydro-1,3-thiazol-2-ylamine
4-(4-thiazolylméthyl)-4,5-dihydro-1,3-thiazol-2-ylamine
4-(1-imidazolylméthyl)-4,5-dihydro-1,3-thiazol-2-ylamine
4-(2-pyrazinylméthyl)-4,5-dihydro-1,3-thiazol-2-ylamine
4-(5-thiazolylméthyl)-4,5-dihydro-1,3-thiazol-2-ylamine
4-(1-triazolylméthyl) -4,5-dihydro-1,3-thiazol-2-ylamine
4-(4-pyridylsulfanylméthyl)-4,5-dihydro-1,3-thiazol-2-ylamine
leurs racémiques, énantiomères, diastétéoisomères et elurs mélanges, leurs tautomères et leurs sels pharmaceutiquement acceptables.

12. Composés selon la revendication 8 choisis parmi les suivants :
(+)-(4R)-4-(3-pyridylméthyl)-4,5-dihydro-1,3-thiazol-2-ylamine
(+)-(4R)-4-(3-nitrobenzyl)-4,5-dihydro-1,3-thiazol-2-ylamine
(+)-(4R,5S)-4-benzyl-5-méthyl-4,5-dihydro-1,3-thiazol-2-ylamine
(+)-(4R,5R)-4-benzyl-5-méthyl-4,5-dihydro-1,3-thiazol-2-ylamine
(-)-(4R)-4-(3-thiénylméthyl)-4,5-dihydro-1,3-thiazol-2-ylamine
(4R)-4-(2-thiénylméthyl)-4,5-dihydro-1,3-thiazol-2-ylamine ??
[3-(2-amino-4,5-dihydro-thiazol-4-yl)-phényl]-(1-imino-éthyl)-amine
(+)-(4R)-4-benzyl-4,5-dihydro-1,3-thiazol-2-ylamine
(+)-(4R)-4-(3-carboxybenzyl)-4,5-dihydro-1,3-thiazol-2-ylamine
(+)-(4R)-4-(4-aminobutyl)-4,5-dihydro-1,3-thiazol-2-ylamine
(-)-(4S)-4-(3-nitrobenzyl)-4,5-dihydro-1,3-thiazol-2-ylamine
(-)-(4S,5S)-4-benzyl-5-méthyl-4,5-dihydro-1,3-thiazol-2-ylamine
(-)-(4S)-4-(4-aminobutyl)-4,5-dihydro-1,3-thiazol-2-ylamine
(4S,5R)-4-benzyl-5-méthyl-4,5-dihydro-1,3-thiazol-2-ylamine
(+)-(4R)-4-(4-pyridylméthyl)-4,5-dihydro-1,3-thiazol-2-ylamine
(+)-(4R,5R)-5-méthyl-4-(4-pyridylméthyl)-4,5-dihydro-1,3-thiazol-2-ylamine
(+)-(4R,5R)-5-éthyl-4-(4-pyridylméthyl)-4,5-dihydro-1,3-thiazol-2-ylamine
(+)-4-(5-thiazolylméthyl)-4,5-dihydro-1,3-thiazol-2-ylamine
(-)-4-(5-thiazolylméthyl)-4,5-dihydro-1,3-thiazol-2-ylamine
(+)-(4R)-4-(2-pyrazinylméthyl)-4,5-dihydro-1,3-thiazol-2-ylamine
(4R)-4-(1-imidazolylméthyl)-4,5-dihydro-1,3-thiazol-2-ylamine
(4S)-4-(1-imidazolylméthyl)-4,5-dihydro-1,3-thiazol-2-ylamine
(+)-(4R)-4-(4-thiazolylméthyl)-4,5-dihydro-1,3-thiazol-2-ylamine
(+)-4-(4-pyridylsulfanylméthyl)-4,5-dihydro-1,3-thiazol-2-ylamine
(+)-4-(1-triazolylméthyl) -4,5-dihydro-1,3-thiazol-2-ylamine
leurs tautomères ainsi que leurs sels pharmaceutiquement acceptables.

13. Compositions pharmaceutiques contenant en tant qu'ingrédient actif au moins un composé de formule 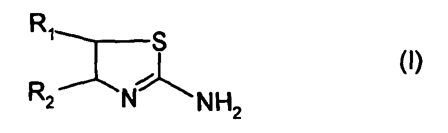 dans laquelle
soit R₁ est un atome d'hydrogène ou un radical alkyle et R₂ est un radical alkyle, -alk-NH₂, -CH₂-R₃, -CH₂-S-R₄ ou phényle substitué par un radical nitro ou -NH-C(=NH)CH₃,
soit R₁ est un radical alkyle et R₂ est un atome d'hydrogène,
R₃ est un radical cycloalkyle (3-6C), pyridyle, thiényle, thiazolyle, imidazolyle, pyrazinyle, triazolyle, phényle ou phényle substitué par un radical nitro, hydroxy ou carboxy,
R₄ représente un radical pyridyle,
alk représente un radical alkylène,
leurs racémiques, énantiomères, diastéréoisomères et leurs mélanges, leurs tautomères et leurs sels pharmaceutiquement acceptables,
à l'exception des composés racémiques pour lesquels R₁ est un radical méthyle et R₂ est un atome d'hydrogène ou bien R₁ est hydrogène et R₂ est méthyle, éthyle ou n-propyle,
étant entendu que les radicaux alkyle et alkylène contiennent 1 à 6 atomes de carbone en chaîne droite ou ramifiée

14. Procédé de préparation des composés de formule (I) selon la revendication 8 **caractérisée en ce que** l'on cyclise un dérivé de formule : 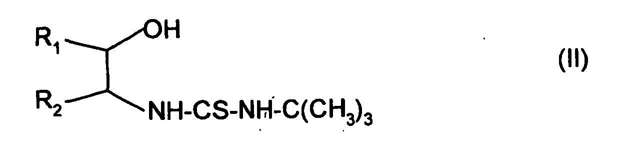 dans laquelle R₁ et R₂ ont les mêmes significations que dans la revendication 8, isole le produit et le transforme éventuellement en sel pharmaceutiquement acceptable.

15. Procédé de préparation des composés de formule (I) selon la revendication 8 pour lesquels R₂ est un radical phényle substitué par un radical -NH-C(=NH)CH₃ **caractérisé en ce que** l'on fait réagir un dérivé de formule : 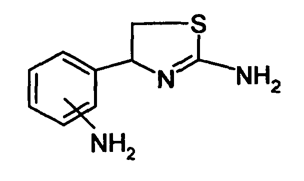 avec du chlorhydrate de benzyléthanimidothioate, isole le produit et le transforme éventuellement en sel pharmaceutiquement acceptable.

16. Procédé de préparation des composés de formule (I) selon la revendication 8 pour lesquels R₂ est un radical -CH₂-R₃ dans lequel R₃ est un radical 1-imidazolyle ou 1-(1,2,4-triazolyle) **caractérisé en ce que** l'on fait réagir l'imidazole ou le 1,2,4-triazole sur un dérivé de formule : 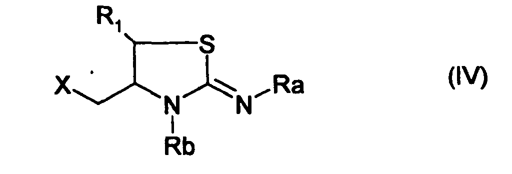 dans laquelle R₁ a les mêmes significations que dans revendication 8, X est un atome d'halogène ou un radical tosyle, Ra et Rb sont des atomes d'hydrogène ou des groupes protecteurs de la fonction amine, suivie éventuellement d'une déprotection, isole le produit et le transforme éventuellement en sel pharmaceutiquement acceptable.

17. Procédé de préparation des composés de formule (I) selon la revendication 8 pour lesquels R₂ est un radical -CH₂-S-R₄ **caractérisé en ce que** l'on fait réagir un composé de formule : 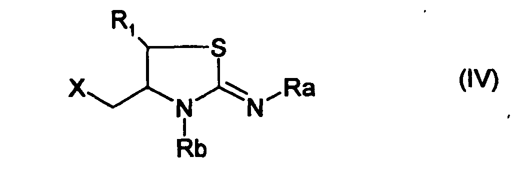 dans laquelle R₁ a les mêmes significations que dans revendication 8, X est un atome d'halogène ou un radical tosyle, Ra et Rb sont des atomes d'hydrogène ou des groupes protecteurs de la fonction amine, sur un dérivé de formule HS-R₄ dans laquelle R₄ a la même signification que dans la revendication 8, suivie éventuellement d'une déprotection de la fonction amine, isole le produit et le transforme éventuellement en sel pharmaceutiquement acceptable.

18. Composés de formule :

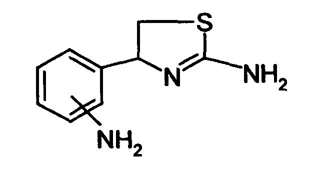

19. Procédé de préparation des composés de formule (II) telle que définie à la revendication 14 et dans laquelle R₁ est un atome d'hydrogène **caractérisé en ce qu'**on soumet un composé de formule (IIa) 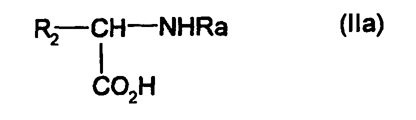 R₂ étant tel que défini à la revendication 1 et Ra étant soit un atome d'hydrogène, soit un groupe protecteur de la formation amine tel que CO₂ tbu à l'action d'un agent de réduction afin d'obtenir le composé de formule (IIb) 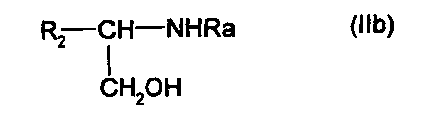 que l'on soumet à l'action d'un agent de déprotection afin d'obtenir un composé de formule (IIc) 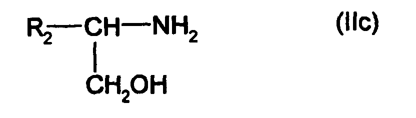 que l'on soumet à l'action du tertbutylthiocyanate afin d'obtenir un composé de formule (IId) 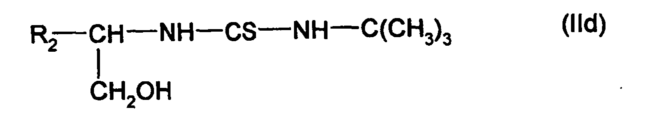 correspondant au composé de formule (II) avec R₁ représentant un atome d'hydrogène.

20. A titre de composés intermédiaires, les composés de formule (IId) tels que définis à la revendication 19.

21. A titre de composés intermédiaires, le composé de formule (IId) telle que définie à la revendication 19 et dans laquelle R₂ représente un groupement 4-pyridylméthyl.

## Patentansprüche

1. Verwendung von 2-Aminothiazolin-Derivaten der Formel (I) 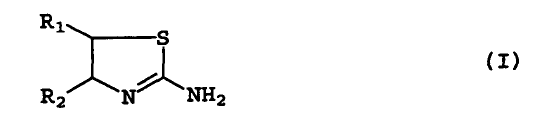 in der
entweder R₁ ein Wasserstoffatom oder ein Rest Alkyl ist und R₂ einen Rest Alkyl, -alk-NH₂, -CH₂-R₃, -CH₂-S-R₄ oder Phenyl darstellt, substituiert durch einen Rest Nitro oder -NH-C(=NH)CH₃, oder R₁ ein Rest Alkyl ist und R₂ ein Wasserstoffatom darstellt, R₃ einen Rest Cycloalkyl (3-6C), Pyridyl, Pyridyl-N-oxid, Thienyl, Thiazolyl, Imidazolyl, Pyrazinyl, Triazolyl, Phenyl oder Phenyl, substituiert durch einen Rest Nitro, Hydroxy oder Carboxy, bedeutet,
R₄ einen Rest Pyridyl oder Pyridyl-N-oxid darstellt,
alk ein Rest Alkylen ist,
mit der Maßgabe, daß die Reste Alkyl und Alkylen 1 bis 6 Kohlenstoffatome in gerader oder verzweigter Kette enthalten,
sowie von ihren Racematen, Enantiomeren, Diastereoisomeren und ihren Mischungen, ihren Tautomeren und ihren pharmazeutisch akzeptablen Salzen zur Herstellung von pharmazeutischen Zusammensetzungen, die vorgesehen sind für die Vorbeugung und Behandlung von Erkrankungen, bei denen eine anormale Produktion von Stickstoffmonoxid (NO) durch Induktion von induktibler NO-Synthase (NOS-2) einbezogen ist.

2. Verwendung nach Anspruch 1, **dadurch gekennzeichnet, daß** in der Formel (I) R₁ ein Wasserstoffatom oder ein Rest Alkyl ist und R₂ einen Rest -alk-NH₂, Phenyl, substituiert durch einen Rest -NH-C(=NH)CH₃, -CH₂-R₃ darstellt und R₃ einen Rest Pyridyl, Thienyl, Thiazolyl, Imidazolyl, Pyrazinyl, Triazolyl, Phenyl oder Phenyl, substituiert durch einen Rest Nitro oder Carboxy, oder -CH₂-S-R₄ bedeutet und R₄ ein Rest Pyridyl ist.

3. Verwendung nach einem der Ansprüche 1 oder 2, **dadurch gekennzeichnet, daß** R₂ eine Kette -CH₂-R₃ oder -CH₂-S-R₄ ist, R₃ einen Rest 3- oder 4-Pyridyl, 2- oder 3-Thienyl, 4- oder 5-Thiazolyl, 1-Imidazolyl, 1-Triazolyl, 2-Pyrazinyl, Phenyl oder Phenyl, substituiert in Position -3 durch einen Rest Nitro oder Carboxy, bedeutet und R₄ ein Rest 4-Pyridyl ist.

4. Verwendung nach Anspruch 1, **dadurch gekennzeichnet, daß** die Verbindung der Formel (I) unter den folgenden Verbindungen ausgewählt wird:
4-(3-Pyridylmethyl)-4,5-dihydro-1,3-thiazol-2-yl-amin
4-(3-Nitrobenzyl)-4,5-dihydro-1,3-thiazol-2-yl-amin
4-Benzyl-5-methyl-4,5-dihydro-1,3-thiazol-2-yl-amin
4-(3-Thienylmethyl)-4,5-dihydro-1,3-thiazol-2-yl-amin
4-(2-Thienylmethyl)-4,5-dihydro-1,3-thiazol-2-yl-amin
[3-(2-Amino-4,5-dihydro-thiazol-4-yl)-phenyl]-(1-imino-ethyl)-amin
4-Benzyl-4,5-dihydro-1,3-thiazol-2-yl-amin
4-(3-Carboxybenzyl)-4,5-dihydro-1,3-thiazol-2-yl-amin
4-(4-Aminobutyl)-4,5-dihydro-1,3-thiazol-2-yl-amin
4-Butyl-4,5-dihydro-1,3-thiazol-2-yl-amin
5-Methyl-4,5-dihydro-1,3-thiazol-2-yl-amin
4-Cyclohexylmethyl-4,5-dihydro-1,3-thiazol-2-yl-amin
4-(3-Nitrophenyl)-4,5-dihydro-1,3-thiazol-2-yl-amin
4-(4-Pyridylmethyl)-4,5-dihydro-1,3-thiazol-2-yl-amin
5-Methyl-4-(4-pyridylmethyl)-4,5-dihydro-1,3-thiazol-2-yl-amin
5-Ethyl-4-(4-pyridylmethyl)-4,5-dihydro-1,3-thiazol-2-yl-amin
4-(4-Thiazolylmethyl)-4,5-dihydro-1,3-thiazol-2-yl-amin
4-(1-Imidazolylmethyl)-4,5-dihydro-1,3-thiazol-2-yl-amin
4-(2-Pyrazinylmethyl)-4,5-dihydro-1,3-thiazol-2-yl-amin
4-(5-Thiazolylmethyl)-4,5-dihydro-1,3-thiazol-2-yl-amin
4-(4-Hydroxybenzyl)-4,5-dihydro-1,3-thiazol-2-yl-amin
4-(4-Pyridylsulfanylmethyl)-4,5-dihydro-1,3-thiazol-2-yl-amin
4-(3-Aminopropyl)-4,5-dihydro-1,3-thiazol-2-yl-amin
4-(1-Triazolylmethyl)-4,5-dihydro-1,3-thiazol-2-yl-amin
N-Oxid von 4-(4-Pyridylmethyl)-4,5-dihydro-1,3-thiazol-2-yl-amin, sowie ihren Racematen, Enantiomeren, Diastereoisomeren und ihren Mischungen, ihren Tautomeren und ihren pharmazeutisch akzeptablen Salzen.

5. Verwendung nach Anspruch 1, **dadurch gekennzeichnet, daß** die Verbindung der Formel (I) unter den folgenden Verbindungen ausgewählt wird:
(+)-(4R)-4-(3-Pyridylmethyl)-4,5-dihydro-1,3-thiazol-2-yl-amin
(+)-(4R)-4-(3-Nitrobenzyl)-4,5-dihydro-1,3-thiazol-2-yl-amin
(+)-(4R,5S)-4-Benzyl-5-methyl-4,5-dihydro-1,3-thiazol-2-yl-amin
(+)-(4R,5R)-4-Benzyl-5-methyl-4,5-dihydro-1,3-thiazol-2-yl-amin
(-)-(4R)-4-(3-Thienylmethyl)-4,5-dihydro-1,3-thiazol-2-yl-amin
(4R)-4-(2-Thienylmethyl)-4,5-dihydro-1,3-thiazol-2-yl-amin
[3-(2-Amino-4,5-dihydro-thiazol-4-yl)-phenyl]-(1-imino-ethyl)-amin
(+)-(4R)-4-Benzyl-4,5-dihydro-1,3-thiazol-2-yl-amin
(+)-(4R)-4-(3-Carboxybenzyl)-4,5-dihydro-1,3-thiazol-2-yl-amin
(+)-(4R)-4-(4-Aminobutyl)-4,5-dihydro-1,3-thiazol-2-yl-amin
(-)-(4S)-4-(3-Nitrobenzyl)-4,5-dihydro-1,3-thiazol-2-yl-amin
(-)-(4S,5S)-4-Benzyl-5-methyl-4,5-dihydro-1,3-thiazol-2-yl-amin
(-)-(4S)-4-(4-Aminobutyl)-4,5-dihydro-1,3-thiazol-2-yl-amin
(4S,5R)-4-Benzyl-5-methyl-4,5-dihydro-1,3-thiazol-2-yl-amin
(-)-(4R)-4-Butyl-4,5-dihydro-1,3-thiazol-2-yl-amin
(+)-(5S)-5-Methyl-4,5-dihydro-1,3-thiazol-2-yl-amin
(-)-(4S)-4-Cyclohexylmethyl-4,5-dihydro-1,3-thiazol-2-yl-amin
(+)-(4R)-4-Cyclohexylmethyl-4,5-dihydro-1,3-thiazol-2-yl-amin
4-(3-Nitrophenyl)-4,5-dihydro-1,3-thiazol-2-yl-amin
(+)-(4R)-4-(4-Pyridylmethyl)-4,5-dihydro-1,3-thiazol-2-yl-amin
(+)-(4R,5R)-5-Methyl-4-(4-pyridylmethyl)-4,5-dihydro-1,3-thiazol-2-yl-amin
(+)-(4R,5R)-5-Ethyl-4-(4-pyridylmethyl)-4,5-dihydro-1,3-thiazol-2-yl-amin
(+)-4-(5-Thiazolylmethyl)-4,5-dihydro-1,3-thiazol-2-yl-amin
(-)-4-(5-Thiazolylmethyl)-4,5-dihydro-1,3-thiazol-2-yl-amin
(+)-(4R)-4-(2-Pyrazinylmethyl)-4,5-dihydro-1,3-thiazol-2-yl-amin
(4R)-4-(1-Imidazolylmethyl)-4,5-dihydro-1,3-thiazol-2-yl-amin
(4S)-4-(1-Imidazolylmethyl)-4,5-dihydro-1,3-thiazol-2-yl-amin
(+)-(4R)-4-(4-Thiazolylmethyl)-4,5-dihydro-1,3-thiazol-2-yl-amin
(+)-(4R)-4-(3-Aminopropyl)-4,5-dihydro-1,3-thiazol-2-yl-amin
(+)-(4R)-4-(4-Hydroxybenzyl)-4,5-dihydro-1,3-thiazol-2-yl-amin
(+)-4-(4-Pyridylsulfanylmethyl)-4,5-dihydro-1,3-thiazol-2-yl-amin
N-Oxid von (4R)-4-(4-Pyridylmethyl)-4,5-dihydro-1,3-thiazol-2-yl-amin
(+)-4-(1-Triazolylmethyl)-4,5-dihydro-1,3-thiazol-2-yl-amin,
sowie ihren Tautomeren und ihren pharmazeutisch akzeptablen Salzen.

6. Verwendung nach Anspruch 2, **dadurch gekennzeichnet, daß** die Verbindung der Formel (I) unter den folgenden Verbindungen ausgewählt wird:
4-(3-Pyridylmethyl)-4,5-dihydro-1,3-thiazol-2-yl-amin
4-(3-Nitrobenzyl)-4,5-dihydro-1,3-thiazol-2-yl-amin
4-Benzyl-5-methyl-4,5-dihydro-1,3-thiazol-2-yl-amin
4-(3-Thienylmethyl)-4,5-dihydro-1,3-thiazol-2-yl-amin
4-(2-Thienylmethyl)-4,5-dihydro-1,3-thiazol-2-yl-amin
[3-(2-Amino-4,5-dihydro-thiazol-4-yl)-phenyl]-(1-imino-ethyl)-amin
4-Benzyl-4,5-dihydro-1,3-thiazol-2-yl-amin
4-(3-Carboxybenzyl)-4,5-dihydro-1,3-thiazol-2-yl-amin
4-(4-Aminobutyl)-4,5-dihydro-1,3-thiazol-2-yl-amin
4-(4-Pyridylmethyl)-4,5-dihydro-1,3-thiazol-2-yl-amin
5-Methyl-4-(4-pyridylmethyl)-4,5-dihydro-1,3-thiazol-2-yl-amin
5-Ethyl-4-(4-pyridylmethyl)-4,5-dihydro-1,3-thiazol-2-yl-amin
4-(4-Thiazolylmethyl)-4,5-dihydro-1,3-thiazol-2-yl-amin
4-(1-Imidazolylmethyl)-4,5-dihydro-1,3-thiazol-2-yl-amin
4-(2-Pyrazinylmethyl)-4,5-dihydro-1,3-thiazol-2-yl-amin
4-(5-Thiazolylmethyl)-4,5-dihydro-1,3-thiazol-2-yl-amin
4-(1-Triazolylmethyl)-4,5-dihydro-1,3-thiazol-2-yl-amin
4-(4-Pyridylsulfanylmethyl)-4,5-dihydro-1,3-thiazol-2-yl-amin,
sowie ihren Racematen, Enantiomeren, Diastereoisomeren und ihren Mischungen, ihren Tautomeren und ihren pharmazeutisch akzeptablen Salzen.

7. Verwendung nach Anspruch 2, **dadurch gekennzeichnet, daß** die Verbindung der Formel (I) unter den folgenden Verbindungen ausgewählt wird:
(+)-(4R)-4-(3-Pyridylmethyl)-4,5-dihydro-1,3-thiazol-2-yl-amin
(+)-(4R)-4-(3-Nitrobenzyl)-4,5-dihydro-1,3-thiazol-2-yl-amin
(+)-(4R,5S)-4-Benzyl-5-methyl-4,5-dihydro-1,3-thiazol-2-yl-amin
(+)-(4R,5R)-4-Benzyl-5-methyl-4,5-dihydro-1,3-thiazol-2-yl-amin
(-)-(4R)-4-(3-Thienylmethyl)-4,5-dihydro-1,3-thiazol-2-yl-amin
(4R)-4-(2-Thienylmethyl)-4,5-dihydro-1,3-thiazol-2-yl-amin
[3-(2-Amino-4,5-dihydro-thiazol-4-yl)-phenyl]-(1-imino-ethyl)-amin
(+)-(4R)-4-Benzyl-4,5-dihydro-1,3-thiazol-2-yl-amin
(+)-(4R)-4-(3-Carboxybenzyl)-4,5-dihydro-1,3-thiazol-2-yl-amin
(+)-(4R)-4-(4-Aminobutyl)-4,5-dihydro-1,3-thiazol-2-yl-amin
(-)-(4S)-4-(3-Nitrobenzyl)-4,5-dihydro-1,3-thiazol-2-yl-amin
(-)-(4S,5S)-4-Benzyl-5-methyl-4,5-dihydro-1,3-thiazol-2-yl-amin
(-)-(4S)-4-(4-Aminobutyl)-4,5-dihydro-1,3-thiazol-2-yl-amin
(4S,5R)-4-Benzyl-5-methyl-4,5-dihydro-1,3-thiazol-2-yl-amin
(+)-(4R)-4-(4-Pyridylmethyl)-4,5-dihydro-1,3-thiazol-2-yl-amin
(+)-(4R,5R)-5-Methyl-4-(4-pyridylmethyl)-4,5-dihydro-1,3-thiazol-2-yl-amin
(+)-(4R,5R)-5-Ethyl-4-(4-pyridylmethyl)-4,5-dihydro-1,3-thiazol-2-yl-amin
(+)-4-(5-Thiazolylmethyl)-4,5-dihydro-1,3-thiazol-2-yl-amin
(-)-4-(5-Thiazolylmethyl)-4,5-dihydro-1,3-thiazol-2-yl-amin
(+)-(4R)-4-(2-Pyrazinylmethyl)-4,5-dihydro-1,3-thiazol-2-yl-amin
(4R)-4-(1-Imidazolylmethyl)-4,5-dihydro-1,3-thiazol-2-yl-amin
(4S)-4-(1-Imidazolylmethyl)-4,5-dihydro-1,3-thiazol-2-yl-amin
(+)-(4R)-4-(4-Thiazolylmethyl)-4,5-dihydro-1,3-thiazol-2-yl-amin
(+) -4-(4-Pyridylsulfanylmethyl)-4,5-dihydro-1,3-thiazol-2-yl-amin
(+)-4-(1-Triazolylmethyl)-4,5-dihydro-1,3-thiazol-2-yl-amin,
sowie ihren Tautomeren und ihren pharmazeutisch akzeptablen Salzen.

8. Verbindungen der Formel 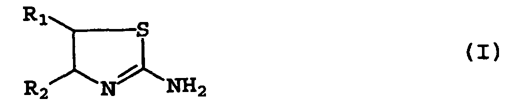 in der
entweder R₁ ein Wasserstoffatom oder ein Rest Alkyl ist und R₂ einen Rest Alkyl, -alk-NH₂, -CH₂-R₃, -CH₂-S-R₄ oder Phenyl darstellt, substituiert durch einen Rest Nitro oder -NH-C(=NH)CH₃, oder R₁ ein Rest Alkyl ist und R₂ ein Wasserstoffatom darstellt, R₃ einen Rest Cycloalkyl (3-6C), Pyridyl, Thienyl, Thiazolyl, Imidazolyl, Pyrazinyl, Triazolyl, Phenyl oder Phenyl, substituiert durch einen Rest Nitro, Hydroxy oder Carboxy, bedeutet,
R₄ einen Rest Pyridyl darstellt,
alk ein Rest Alkylen ist,
sowie ihre Racemate, Enantiomeren, Diastereoisomeren und ihre Mischungen, ihre Tautomeren und ihre pharmazeutisch akzeptablen Salze,
mit Ausnahme der Verbindungen, bei denen R₁ Hexyl oder Methyl ist und R₂ Wasserstoff darstellt, oder R₁ und R₂ Methyl sind, und die Racemate dieser Verbindungen, bei denen R₁ Wasserstoff ist und R₂ Methyl, Ethyl oder n-Propyl darstellt,
mit der Maßgabe, daß die Reste Alkyl und Alkylen 1 bis 6 Kohlenstoffatome in gerader oder verzweigter Kette enthalten.

9. Verbindungen nach Anspruch 8, in denen R₁ ein Wasserstoffatom oder ein Rest Alkyl ist und R₂ einen Rest -alk-NH₂, Phenyl, substituiert durch einen Rest -NH-C(=NH)CH₃, -CH₂-R₃ darstellt und R₃ einen Rest Pyridyl, Thienyl, Thiazolyl, Imidazolyl, Triazolyl, Pyrazinyl, Phenyl oder Phenyl, substituiert durch einen Rest Nitro oder Carboxy, oder -CH₂-S-R₄ bedeutet, worin R₄ ein Rest Pyridyl ist, mit der Maßgabe, daß die Reste Alkyl und Alkylen 1 bis 6 Kohlenstoffatome in gerader oder verzweigter Kette enthalten,
sowie ihre Racemate, Enantiomeren, Diastereoisomeren und ihre Mischungen, ihre Tautomeren und ihre pharmazeutisch akzeptablen Salze.

10. Verbindungen nach einem der Ansprüche 8 oder 9, **dadurch gekennzeichnet, daß** R₃ einen Rest 3- oder 4-Pyridyl, 2- oder 3-Thienyl, 4- oder 5-Thiazolyl, 1-Imidazolyl, 1-Triazolyl, 2-Pyrazinyl, Phenyl oder Phenyl, substituiert in Position -3 durch einen Rest Nitro oder Carboxy, bedeutet und R₄ ein Rest 4-Pyridyl ist.

11. Verbindungen nach Anspruch 8, ausgewählt unter den folgenden:
4-(3-Pyridylmethyl)-4,5-dihydro-1,3-thiazol-2-yl-amin
4-(3-Nitrobenzyl)-4,5-dihydro-1,3-thiazol-2-yl-amin
4-Benzyl-5-methyl-4,5-dihydro-1,3-thiazol-2-yl-amin
4-(3-Thienylmethyl)-4,5-dihydro-1,3-thiazol-2-yl-amin
4-(2-Thienylmethyl)-4,5-dihydro-1,3-thiazol-2-yl-amin
[3-(2-Amino-4,5-dihydro-thiazol-4-yl)-phenyl]-(1-imino-ethyl)-amin
4-Benzyl-4,5-dihydro-1,3-thiazol-2-yl-amin
4-(3-Carboxybenzyl)-4,5-dihydro-1,3-thiazol-2-yl-amin
4-(4-Aminobutyl)-4,5-dihydro-1,3-thiazol-2-yl-amin
4-(4-Pyridylmethyl)-4,5-dihydro-1,3-thiazol-2-yl-amin
5-Methyl-4-(4-pyridylmethyl)-4,5-dihydro-1,3-thiazol-2-yl-amin
5-Ethyl-4-(4-pyridylmethyl)-4,5-dihydro-1,3-thiazol-2-yl-amin
4-(4-Thiazolylmethyl)-4,5-dihydro-1,3-thiazol-2-yl-amin
4-(1-Imidazolylmethyl)-4,5-dihydro-1,3-thiazol-2-yl-amin
4-(2-Pyrazinylmethyl)-4,5-dihydro-1,3-thiazol-2-yl-amin
4-(5-Thiazolylmethyl)-4,5-dihydro-1,3-thiazol-2-yl-amin
4-(1-Triazolylmethyl)-4,5-dihydro-1,3-thiazol-2-yl-amin
4-(4-Pyridylsulfanylmethyl)-4,5-dihydro-1,3-thiazol-2-yl-amin,
sowie ihre Racemate, Enantiomeren, Diastereoisomeren und ihre Mischungen, ihre Tautomeren und ihre pharmazeutisch akzeptablen Salze.

12. Verbindungen nach Anspruch 8, ausgewählt unter den folgenden:
(+)-(4R)-4-(3-Pyridylmethyl)-4,5-dihydro-1,3-thiazol-2-yl-amin
(+)-(4R)-4-(3-Nitrobenzyl)-4,5-dihydro-1,3-thiazol-2-yl-amin
(+)-(4R,5S)-4-Benzyl-5-methyl-4,5-dihydro-1,3-thiazol-2-yl-amin
(+)-(4R,5R)-4-Benzyl-5-methyl-4,5-dihydro-1,3-thiazol-2-yl-amin
(-)-(4R)-4-(3-Thienylmethyl)-4,5-dihydro-1,3-thiazol-2-yl-amin
(4R)-4-(2-Thienylmethyl)-4,5-dihydro-1,3-thiazol-2-yl-amin
[3-(2-Amino-4,5-dihydro-thiazol-4-yl)-phenyl]-(1-imino-ethyl)-amin
(+)-(4R)-4-Benzyl-4,5-dihydro-1,3-thiazol-2-yl-amin
(+)-(4R)-4-(3-Carboxybenzyl)-4,5-dihydro-1,3-thiazol-2-yl-amin
(+)-(4R)-4-(4-Aminobutyl)-4,5-dihydro-1,3-thiazol-2-yl-amin
(-)-(4S)-4-(3-Nitrobenzyl)-4,5-dihydro-1,3-thiazol-2-yl-amin
(-)-(4S,5S)-4-Benzyl-5-methyl-4,5-dihydro-1,3-thiazol-2-yl-amin
(-)-(4S)-4-(4-Aminobutyl)-4,5-dihydro-1,3-thiazol-2-yl-amin
(4S,5R)-4-Benzyl-5-methyl-4,5-dihydro-1,3-thiazol-2-yl-amin
(+)-(4R)-4-(4-Pyridylmethyl)-4,5-dihydro-1,3-thiazol-2-yl-amin
(+)-(4R,5R)-5-Methyl-4-(4-pyridylmethyl)-4,5-dihydro-1,3-thiazol-2-yl-amin
(+)-(4R,5R)-5-Ethyl-4-(4-pyridylmethyl)-4,5-dihydro-1,3-thiazol-2-yl-amin
(+)-4-(5-Thiazolylmethyl)-4,5-dihydro-1,3-thiazol-2-yl-amin
(-)-4-(5-Thiazolylmethyl)-4,5-dihydro-1,3-thiazol-2-yl-amin
(+)-(4R)-4-(2-Pyrazinylmethyl)-4,5-dihydro-1,3-thiazol-2-yl-amin
(4R)-4-(1-Imidazolylmethyl)-4,5-dihydro-1,3-thiazol-2-yl-amin
(4S)-4-(1-Imidazolylmethyl)-4,5-dihydro-1,3-thiazol-2-yl-amin
(+)-(4R)-4-(4-Thiazolylmethyl)-4,5-dihydro-1,3-thiazol-2-yl-amin
(+)-4-(4-Pyridylsulfanylmethyl)-4,5-dihydro-1,3-thiazol-2-yl-amin
(+)-4-(1-Triazolylmethyl)-4,5-dihydro-1,3-thiazol-2-yl-amin,
sowie ihre Tautomeren und ihren pharmazeutisch akzeptablen Salze.

13. Pharmazeutische Zusammensetzungen, enthaltend als Wirkstoff mindestens eine Verbindung der Formel (I) 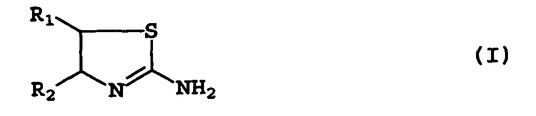 in der
entweder R₁ ein Wasserstoffatom oder ein Rest Alkyl ist und R₂ einen Rest Alkyl, -alk-NH₂, -CH₂-R₃, -CH₂-S-R₄ oder Phenyl darstellt, substituiert durch einen Rest Nitro oder -NH-C(=NH)CH₃, oder R₁ ein Rest Alkyl ist und R₂ ein Wasserstoffatom darstellt, R₃ einen Rest Cycloalkyl (3-6C), Pyridyl, Thienyl, Thiazolyl, Imidazolyl, Pyrazinyl, Triazolyl, Phenyl oder Phenyl, substituiert durch einen Rest Nitro, Hydroxy oder Carboxy, bedeutet,
R₄ einen Rest Pyridyl darstellt,
alk ein Rest Alkylen ist,
sowie ihre Racemate, Enantiomeren, Diastereoisomeren und ihre Mischungen, ihre Tautomeren und ihre pharmazeutisch akzeptablen Salze,
mit Ausnahme der racemischen Verbindungen, bei denen R₁ ein Rest Methyl ist und R₂ ein Wasserstoffatom darstellt, oder R₁ Wasserstoff ist und R₂ Methyl, Ethyl oder n-Propyl bedeutet,
mit der Maßgabe, daß die Reste Alkyl und Alkylen 1 bis 6 Kohlenstoffatome in gerader oder verzweigter Kette enthalten.

14. Verfahren zur Herstellung der Verbindungen der Formel (I) nach Anspruch 8, **dadurch gekennzeichnet, daß** man ein Derivat der Formel (II) 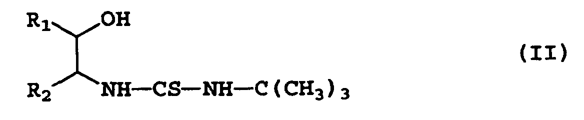 in der
R₁ und R₂ die gleichen Bedeutungen wie in Anspruch 8 besitzen, cyclisiert, das Produkt isoliert und gegebenenfalls in ein pharmazeutisch akzeptables Salz überführt.

15. Verfahren zur Herstellung der Verbindungen der Formel (I) nach Anspruch 8, worin R₂ ein Rest Phenyl ist, substituiert durch einen Rest -NH-C(=NH)CH₃, **dadurch gekennzeichnet, daß** man ein Derivat der Formel 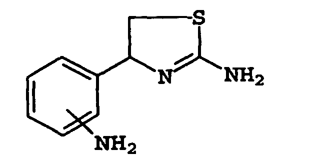 mit Benzylethanimidothioat-Hydrochlorid zur Reaktion bringt, das Produkt isoliert und gegebenenfalls in ein pharmazeutisch akzeptables Salz überführt.

16. Verfahren zur Herstellung der Verbindungen der Formel (I) nach Anspruch 8, worin R₂ ein Rest -CH₂-R₃ ist und R₃ einen Rest 1-Imidazolyl oder 1-(1,2,4-Triazolyl) darstellt, **dadurch gekennzeichnet, daß** man Imidazol oder 1,2,4-Triazol mit einem Derivat der Formel (IV) 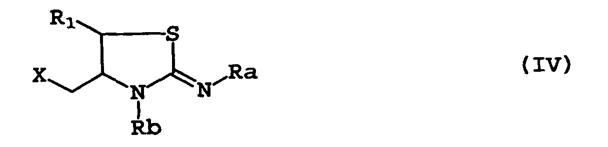 in der R₁ die gleichen Bedeutungen wie in Anspruch 8 besitzt, X ein Halogenatom oder ein Rest Tosyl ist, Ra und Rb Wasserstoffatome oder Schutzgruppen für die Aminfunktion darstellen, zur Reaktion bringt, anschließend gegebenenfalls die Schutzgruppen entfernt, das Produkt isoliert und gegebenenfalls in ein pharmazeutisch akzeptables Salz überführt.

17. Verfahren zur Herstellung der Verbindungen der Formel (I) nach Anspruch 8, worin R₂ ein Rest -CH₂-S-R₄ ist, **dadurch gekennzeichnet, daß** man eine Verbindung der Formel (IV) 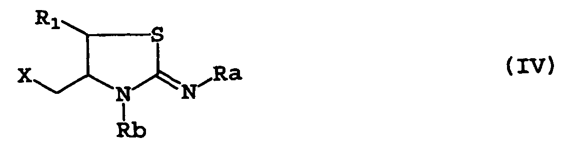 in der R₁ die gleichen Bedeutungen wie in Anspruch 8 besitzt, X ein Halogenatom oder ein Rest Tosyl ist, Ra und Rb Wasserstoffatome oder Schutzgruppen für die Aminfunktion darstellen, mit einem Derivat der Formel HS-R₄, in der R₄ die gleiche Bedeutung wie in Anspruch 8 besitzt, zur Reaktion bringt, anschließend gegebenenfalls die Schutzgruppen von der Aminfunktion entfernt, das Produkt isoliert und gegebenenfalls in ein pharmazeutisch akzeptables Salz überführt.

18. Verbindungen der Formel

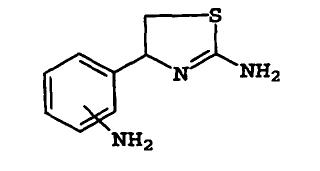

19. Verfahren zur Herstellung der Verbindungen der Formel (II) wie in Anspruch 14 definiert und worin R₁ ein Wasserstoffatom ist, **dadurch gekennzeichnet, daß** man eine Verbindung der Formel (IIa) 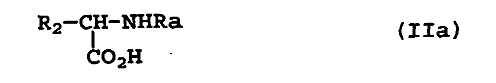 worin R₂ wie in Anspruch 1 definiert ist und Ra entweder ein Wasserstoffatom oder eine Schutzgruppe für die Aminfunktion wie CO₂ tBu darstellt, der Einwirkung eines Reduktionsmittels unterzieht, um die Verbindung der Formel (IIb) 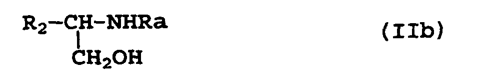 zu erhalten, die man der Einwirkung eines Mittels zur Abspaltung der Schutzgruppe unterzieht, um eine Verbindung der Formel (IIc) 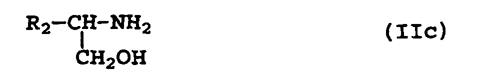 zu erhalten, die man der Einwirkung von tert.-Butylthiocyanat unterzieht, um eine Verbindung der Formel (IId) 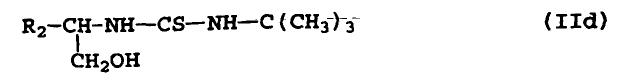 zu erhalten, die der Verbindung der Formel (II) entspricht, worin R₁ ein Wasserstoffatom ist.

20. Als Zwischenverbindungen die Verbindungen der Formel (IId) wie in Anspruch 19 definiert.

21. Als Zwischenverbindungen die Verbindung der Formel (IId) wie in Anspruch 19 definiert und worin R₂ eine Gruppe 4-Pyridylmethyl ist.

## Claims

1. Use of the 2-aminothiazoline derivatives of formula: 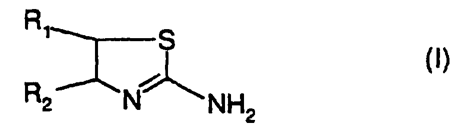 in which either R₁ is a hydrogen atom or an alkyl radical and R₂ is an alkyl, -alk-NH₂, -CH₂-R₃, -CH₂-S-R₄ or phenyl radical substituted with a nitro or -NH-C(=NH)CH₃ radical,
or R₁ is an alkyl radical and R₂ is a hydrogen atom,
R₃ is a (3-6C) cycloalkyl, pyridyl, pyridyl N-oxide, thienyl, thiazolyl, imidazolyl, pyrazinyl, triazolyl or phenyl radical or a phenyl radical substituted with a nitro, hydroxy or carboxyl radical,
R₄ represents a pyridyl or pyridyl N-oxide radical,
alk represents an alkylene radical,
it being understood that the alkyl and alkylene radicals contain 1 to 6 carbon atoms in a straight or branched chain,
the racemic mixtures, enantiomers and diastereomers thereof and the mixtures thereof, the tautomers thereof and the pharmaceutically acceptable salts thereof,
for the preparation of pharmaceutical compositions that are useful for preventing and treating diseases in which an abnormal production of nitrogen monoxide (NO) by induction of inducible NO-synthase (NOS-2) is involved.

2. Use according to Claim 1, **characterized in that**, in formula (I), R₁ is a hydrogen atom or an alkyl radical and R₂ is an -alk-NH₂ radical or phenyl radical substituted with an -NH-C(=NH)CH₃ or -CH₂-R₃ radical and R₃ is a pyridyl, thienyl, thiazolyl, imidazolyl, pyrazinyl, triazolyl or phenyl radical or phenyl radical substituted with a nitro or carboxyl or -CH₂-S-R₄ radical, and R₄ is a pyridyl radical.

3. Use according to either of Claims 1 and 2, **characterized in that** R₂ is a -CH₂-R₃ or -CH₂-S-R₄ chain, R₃ is a 3- or 4-pyridyl, 2- or 3-thienyl, 4- or 5-thiazolyl, 1-imidazolyl, 1-triazolyl, 2-pyrazinyl or phenyl radical or a phenyl radical substituted in position -3 with a nitro or carboxyl radical and R₄ is a 4-pyridyl radical.

4. Use according to Claim 1, **characterized in that** the compound of formula (I) is chosen from the following compounds:
4-(3-pyridylmethyl)-4,5-dihydro-1,3-thiazol-2-ylamine
4-(3-nitrobenzyl)-4,5-dihydro-1,3-thiazol-2-ylamine
4-benzyl-5-methyl-4,5-dihydro-1,3-thiazol-2-ylamine
4-(3-thienylmethyl)-4,5-dihydro-1,3-thiazol-2-ylamine
4-(2-thienylmethyl)-4,5-dihydro-1,3-thiazol-2-ylamine
[3-(2-amino-4,5-dihydrothiazol-4-yl)phenyl](1-iminoethyl)amine
4-benzyl-4,5-dihydro-1,3-thiazol-2-ylamine
4-(3-carboxybenzyl)-4,5-dihydro-1,3-thiazol-2-ylamine
4-(4-aminobutyl)-4,5-dihydro-1,3-thiazol-2-ylamine
4-butyl-4,5-dihydro-1,3-thiazol-2-ylamine
5-methyl-4,5-dihydro-1,3-thiazol-2-ylamine
4-cyclohexylmethyl-4,5-dihydro-1,3-thiazol-2-ylamine
4-(3-nitrophenyl)-4,5-dihydro-1,3-thiazol-2-ylamine
4-(4-pyridylmethyl)-4,5-dihydro-1,3-thiazol-2-ylamine
5-methyl-4-(4-pyridylmethyl)-4,5-dihydro-1,3-thiazol-2-ylamine
5-ethyl-4-(4-pyridylmethyl)-4,5-dihydro-1,3-thiazol-2-ylamine
4-(4-thiazolylmethyl)-4,5-dihydro-1,3-thiazol-2-ylamine
4-(1-imidazolylmethyl)-4,5-dihydro-1,3-thiazol-2-ylamine
4-(2-pyrazinylmethyl)-4,5-dihydro-1,3-thiazol-2-ylamine
4-(5-thiazolylmethyl)-4,5-dihydro-1,3-thiazol-2-ylamine
4-(4-hydroxybenzyl)-4,5-dihydro-1,3-thiazol-2-ylamine
4-(4-pyridylsulphanylmethyl)-4,5-dihydro-1,3-thiazol-2-ylamine
4-(3-aminopropyl)-4,5-dihydro-1,3-thiazol-2-ylamine
4-(1-triazolylmethyl)-4,5-dihydro-1,3-thiazol-2-ylamine
4-(4-pyridylmethyl)-4,5-dihydro-1,3-thiazol-2-ylamine N-oxide
the racemic mixtures, enantiomers, diastereoisomers and tautomers thereof, and mixtures thereof, as well as the pharmaceutically acceptable salts thereof,

5. Use according to Claim 1, **characterized in that** the compound of formula (I) is chosen from the following compounds:
(+)-(4R)-4-(3-pyridylmethyl)-4,5-dihydro-1,3-thiazol-2-ylamine
(+)-(4R)-4-(3-nitrobenzyl)-4,5-dihydro-1,3-thiazol-2-ylamine
(+)-(4R,5S)-4-benzyl-5-methyl-4,5-dihydro-1,3-thiazol-2-ylamine
(+)-(4R,5R)-4-benzyl-5-methyl-4,5-dihydro-1,3-thiazol-2-ylamine
(-)-(4R)-4-(3-thienylmethyl)-4,5-dihydro-1,3-thiazol-2-ylamine
(4R)-4-(2-thienylmethyl)-4,5-dihydro-1,3-thiazol-2-ylamine
[3-(2-amino-4,5-dihydrothiazol-4-yl)phenyl](1-iminoethyl)amine
(+)-(4R)-4-benzyl-4,5-dihydro-1,3-thiazol-2-ylamine
(+)-(4R)-4-(3-carboxybenzyl)-4,5-dihydro-1,3-thiazol-2-ylamine
(+)-(4R)-4-(4-aminobutyl)-4,5-dihydro-1,3-thiazol-2-ylamine
(-)-(4S)-4-(3-nitrobenzyl)-4,5-dihydro-1,3-thiazol-2-ylamine
(-)-(4S,5S)-4-benzyl-5-methyl-4,5-dihydro-1,3-thiazol-2-ylamine
(-)-(4S)-4-(4-aminobutyl)-4,5-dihydro-1,3-thiazol-2-ylamine
(4S,5R)-4-benzyl-5-methyl-4,5-dihydro-1,3-thiazol-2-ylamine
(-)-(4R)-4-butyl-4,5-dihydro-1,3-thiazol-2-ylamine
(+)-(5S)-5-methyl-4,5-dihydro-1,3-thiazol-2-ylamine
(-)-(4S)-4-cyclohexylmethyl-4,5-dihydro-1,3-thiazol-2-ylamine
(+)-(4R)-4-cyclohexylmethyl-4,5-dihydro-1,3-thiazol-2-ylamine
4-(3-nitrophenyl)-4,5-dihydro-1,3-thiazol-2-ylamine
(+)-(4R)-4-(4-pyridylmethyl)-4,5-dihydro-1,3-thiazol-2-ylamine
(+)-(4R,5R)-5-methyl-4-(4-pyridylmethyl)-4,5-dihydro-1,3-thiazol-2-ylamine
(+)-(4R,5R)-5-ethyl-4-(4-pyridylmethyl)-4,5-dihydro-1,3-thiazol-2-ylamine
(+)-4-(5-thiazolylmethyl)-4,5-dihydro-1,3-thiazol-2-ylamine
(-)-4-(5-thiazolylmethyl)-4,5-dihydro-1,3-thiazol-2-ylamine
(+)-(4R)-4-(2-pyrazinylmethyl)-4,5-dihydro-1,3-thiazol-2-ylamine
(4R)-4-(1-imidazolylmethyl)-4,5-dihydro-1,3-thiazol-2-ylamine
(4S)-4-(1-imidazolylmethyl)-4,5-dihydro-1,3-thiazol-2-ylamine
(+)-(4R)-4-(4-thiazolylmethyl)-4,5-dihydro-1,3-thiazol-2-ylamine
(+)-(4R)-4-(3-aminopropyl)-4,5-dihydro-1,3-thiazol-2-ylamine
(+)-(4R)-4-(4-hydroxybenzyl)-4,5-dihydro-1,3-thiazol-2-ylamine
(+)-4-4-pyridylsulphanylmethyl)-4,5-dihydro-1,3-thiazol-2-ylamine
(4R)-4-(4-pyridylmethyl)-4,5-dihydro-1,3-thiazol-2-ylamine N-oxide
(+)-4-(1-triazolylmethyl)-4,5-dihiydro-1,3-thiazol-2-ylamine
the tautomers thereof, as well as the pharmaceutically acceptable salts thereof.

6. Use according to Claim 2, **characterized in that** the compound of formula (I) is chosen from the following compounds:
4-(3-pyridylmethyl)-4,5-dihydro-1,3-thiazol-2-ylamine
4-(3-nitrobenzyl)-4,5-dihydro-1,3-thiazol-2-ylamine
4-benzyl-5-methyl-4,5-dihydro-1,3-thiazol-2-ylamine
4-(3-thienylmethyl)-4,5-dihydro-1,3-thiazol-2-ylamine
4-(2-thienylmethyl)-4,5-dihydro-1,3-thiazol-2-ylamine
[3-(2-amino-4,5-dihydrothiazol-4-yl)phenyl](1-iminoethyl)amine
4-benzyl-4,5-dihydro-1,3-thiazol-2-ylamine
4-(3-carboxybenzyl)-4,5-dihydro-1,3-thiazol-2-ylamine
4-(4-aminobutyl)-4,5-dihydro-1,3-thiazol-2-ylamine
4-(4-pyridylmethyl)-4,5-dihydro-1,3-thiazol-2-ylamine
5-methyl-4-(4-pyridylmethyl)-4,5-dihydro-1,3-thiazol-2-ylamine
5-ethyl-4-(4-pyridylmethyl)-4,5-dihydro-1,3-thiazol-2-ylamine
4-(4-thiazolylmethyl)-4,5-dihydro-1,3-thiazol-2-ylamine
4-(1-imidazolylmethyl)-4,5-dihydro-1,3-thiazol-2-ylamine
4-(2-pyrazinylmethyl)-4,5-dihydro-1,3-thiazol-2-ylamine
4-(5-thiazolylmethyl)-4,5-dihydro-1,3-thiazol-2-ylamine
4-(1-triazolylmethyl)-4,5-dihydro-1,3-thiazol-2-ylamine
4-(4-pyridylsulphanylmethyl)-4,5-dihydro-1,3-thiazol-2-ylamine
the racemic mixtures, enantiomers, diastereoisomers and tautomers thereof, as well as the pharmaceutically acceptable salts thereof.

7. Use according to Claim 2, **characterized in that** the compound of formula (I) is chosen from the following compounds:
(+)-(4R)-4-(3-pyridylmethyl)-4,5-dihydro-1,3-thiazol-2-ylamine
(+)-(4R)-4-(3-nitrobenzyl)-4,5-dihydro-1,3-thiazol-2-ylamine
(+)-(4R,5S)-4-benzyl-5-methyl-4,5-dihydro-1,3-thiazol-2-ylamine
(+)-(4R,5R)-4-benzyl-5-methyl-4,5-dihydro-1,3-thiazol-2-ylamine
(-)-(4R)-4-(3-thienylmethyl)-4,5-dihydro-1,3-thiazol-2-ylamine
(4R)-4-(2-thienylmethyl)-4,5-dihydro-1,3-thiazol-2-ylamine
[3-(2-amino-4,5-dihydrothiazol-4-yl)phenyl](1-iminoethyl)amine
(+)-(4R)-4-benzyl-4,5-dihydro-1,3-thiazol-2-ylamine
(+)-(4R)-4-(3-carboxybenzyl)-4,5-dihydro-1,3-thiazol-2-ylamine
(+)-(4R)-4-(4-aminobutyl)-4,5-dihydro-1,3-thiazol-2-ylamine
(-)-(4S)-4-(3-nitrobenzyl)-4,5-dihydro-1,3-thiazol-2-ylamine
(-)-(4S,5S)-4-benzyl-5-methyl-4,5-dihydro-1,3-thiazol-2-ylamine
(-)-(4S)-4-(4-aminobutyl)-4,5-dihydro-1,3-thiazol-2-ylamine
(4S,5R)-4-benzyl-5-methyl-4,5-dihydro-1,3-thiazol-2-ylamine
(+)-(4R)-4-(4-pyridylmethyl)-4,5-dihydro-1,3-thiazol-2-ylamine
(+)-(4R,5R)-5-methyl-4-(4-pyridylmethyl)-4,5-dihydro-1,3-thiazol-2-ylamine
(+)-(4R,5R)-5-ethyl-4-(4-pyridylmethyl)-4,5-dihydro-1,3-thiazol-2-ylamine
(+)-4-(5-thiazolylmethyl)-4,5-dihydro-1,3-thiazol-2-ylamine
(-)-4-(5-thiazolylmethyl)-4,5-dihydro-1,3-thiazol-2-ylamine
(+)-(4R)-4-(2-pyrazinylmethyl)-4,5-dihydro-1,3-thiazol-2-ylamine
(4R)-4-(1-imidazolylmethyl)-4,5-dihydro-1,3-thiazol-2-ylamine
(4S)-4-(1-imidazolylmethyl)-4,5-dihydro-1,3-thiazol-2-ylamine
(+)-(4R) -4-(4-thiazolylmethyl)-4,5-dihydro-1,3-thiazol-2-ylamine
(+)-4-(4-pyridylsulphanylmethyl)-4,5-dihydro-1,3-thiazol-2-ylamine
(+)-4-(1-triazolylmethyl)-4,5-dihydro-1,3-thiazol-2-ylamine
the tautomers thereof, as well as the pharmaceutically acceptable salts thereof.

8. Compounds of formula: 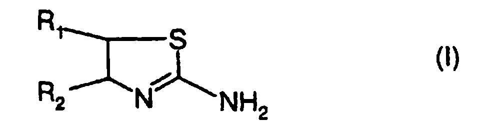 in which either R₁ is a hydrogen atom or an alkyl radical and R₂ is an alkyl, -alk-NH₂, -CH₂-R₃, -CH₂-S-R₄ or phenyl radical substituted with a nitro or -NH-C(=NH)CH₃ radical,
or R₁ is an alkyl radical and R₂ is a hydrogen atom,
R₃ is a (3-6C) cycloalkyl, pyridyl, thienyl, thiazolyl, imidazolyl, pyrazinyl, triazolyl or phenyl radical or a phenyl radical substituted with a nitro, hydroxy or carboxyl radical,
R₄ represents a pyridyl radical,
alk represents an alkylene radical,
the racemic mixtures, enantiomers and diastereomers thereof and the mixtures thereof, the tautomers thereof and the pharmaceutically acceptable salts thereof,
with the exception of the compounds for which R₁ is a hexyl or methyl radical and R₂ is hydrogen, or R₁ and R₂ are methyl, and the racemic mixtures of the compounds for which R₁ is hydrogen and R₂ is methyl, ethyl or n-propyl, it being understood that the alkyl and alkylene radicals contain 1 to 6 carbon atoms in a straight or branched chain.

9. Compounds according to Claim 8, for which R₁ is a hydrogen atom or an alkyl radical and R₂ is an -alk-NH₂ radical or a phenyl radical substituted with an -NH-C(=NH)CH₃ or -CH₂-R₃ radical for which R₃ is a pyridyl, thienyl, thiazolyl, imidazolyl, triazolyl, pyrazinyl or phenyl radical or phenyl radical substituted with a nitro or carboxyl or -CH₂-S-R₄ radical, for which R₄ is a pyridyl radical, it being understood that the alkyl and alkylene radicals contain 1 to 6 carbon atoms in a straight or branched chain, the racemic mixtures, enantiomers and diastereoisomers thereof and the mixtures thereof, the tautomers thereof and the pharmaceutically acceptable salts thereof.

10. Compounds according to either of Claims 8 and 9, **characterized in that** R₃ is a 3- or 4-pyridyl, 2- or 3-thienyl, 4- or 5-thiazolyl, 1-imidazolyl, 1-triazolyl, 2-pyrazinyl or phenyl radical or a phenyl radical substituted in position -3 with a nitro or carboxyl radical and R₄ is a 4-pyridyl radical.

11. Compounds according to Claim 8, chosen from the following:
4-(3-pyridylmethyl)-4,5-dihydro-1,3-thiazol-2-ylamine
4-(3-nitrobenzyl)-4,5-dihydro-1,3-thiazol-2-ylamine
4-benzyl-5-methyl-4,5-dihydro-1,3-thiazol-2-ylamine
4-(3-thienylmethyl)-4,5-dihydro-1,3-thiazol-2-ylamine
4-(2-thienylmethyl)-4,5-dihydro-1,3-thiazol-2-ylamine
[3-(2-amino-4,5-dihydrothiazol-4-yl)phenyl](1-iminoethyl)amine
4-benzyl-4,5-dihydro-1,3-thiazol-2-ylamine
4-(3-carboxybenzyl)-4,5-dihydro-1,3-thiazol-2-ylamine
4-(4-aminobutyl)-4,5-dihydro-1,3-thiazol-2-ylamine
4-(4-pyridylmethyl)-4,5-dihydro-1,3-thiazol-2-ylamine
5-methyl-4-(4-pyridylmethyl)-4,5-dihydro-1,3-thiazol-2-ylamine
5-ethyl-4-(4-pyridylmethyl)-4,5-dihydro-1,3-thiazol-2-ylamine
4-(4-thiazolylmethyl)-4,5-dihydro-1,3-thiazol-2-ylamine
4-(1-imidazolylmethyl)-4,5-dihydro-1,3-thiazol-2-ylamine
4-(2-pyrazinylmethyl)-4,5-dihydro-1,3-thiazol-2-ylamine
4-(5-thiazolylmethyl)-4,5-dihydro-1,3-thiazol-2-ylamine
4-(1-triazolylmethyl)-4,5-dihydro-1,3-thiazol-2-ylamine
4-(4-pyridylsulphanylmethyl)-4,5-dihydro-1,3-thiazol-2-ylamine
the racemic mixtures, entaniomers and diastereroisomers thereof and mixtures thereof, the tautomers thereof and the pharmaceutically acceptable salts thereof.

12. Compounds according to Claim 8, chosen from the following:
(+)-(4R)-4-(3-pyridylmethyl)-4,5-dihydro-1,3-thiazol-2-ylamine
(+)-(4R)-4-(3-nitrobenzyl)-4,5-dihydro-1,3-thiazol-2-ylamine
(+)-(4R,5S)-4-benzyl-5-methyl-4,5-dihydro-1,3-thiazol-2-ylamine
(+)-(4R,5R)-4-benzyl-5-methyl-4,5-dihydro-1,3-thiazol-2-ylamine
(-)-(4R)-4-(3-thienylmethyl)-4,5-dihydro-1,3-thiazol-2-ylamine
(4R)-4-(2-thienylmethyl)-4,5-dihydro-1,3-thiazol-2-ylamine??
[3-(2-amino-4,5-dihydrothiazol-4-yl)phenyl]-(1-iminoethyl)amine
(+)-(4R)-4-benzyl-4,5-dihydro-1,3-thiazol-2-ylamine
(+)-(4R)-4-(3-carboxybenzyl)-4,5-dihydro-1,3-thiazol-2-ylamine
(+)-(4R)-4-(4-aminobutyl)-4,5-dihydro-1,3-thiazol-2-ylamine
(-)-(4S)-4-(3-nitrobenzyl)-4,5-dihydro-1,3-thiazol-2-ylamine
(-)-(4S,5S)-4-benzyl-5-methyl-4,5-dihydro-1,3-thiazol-2-ylamine
(-)-(4S)-4-(4-aminobutyl)-4,5-dihydro-1,3-thiazol-2-ylamine
(4S,5R)-4-benzyl-5-methyl-4,5-dihydro-1,3-thiazol-2-ylamine
(+)-(4R)-4-(4-pyridylmethyl)-4,5-dihydro-1,3-thiazol-2-ylamine
(+)-(4R,5R)-5-methyl-4-(4-pyridylmethyl)-4,5-dihydro-1,3-thiazol-2-ylamine
(+)-(4R,5R)-5-ethyl-4-(4-pyridylmethyl)-4,5-dihydro-1,3-thiazol-2-ylamine
(+)-4-(5-thiazolylmethyl)-4,5-dihydro-1,3-thiazol-2-ylamine
(-)-4-(5-thiazolylmethyl)-4,5-dihydro-1,3-thiazol-2-ylamine
(+)-(4R)-4-(2-pyrazinylmethyl)-4,5-dihydro-1,3-thiazol-2-ylamine
(4R)-4-(1-imidazolylmethyl)-4,5-dihydro-1,3-thiazol-2-ylamine
(4S)-4-(1-imidazolylmethyl)-4,5-dihydro-1,3-thiazol-2-ylamine
(+)-(4R)-4-(4-thiazolylmethyl)-4,5-dihydro-1,3-thiazol-2-ylamine
(+)-4-(4-pyridylsulphanylmethyl)-4,5-dihydro-1,3-thiazol-2-ylamine
(+)-4-(1-triazolylmethyl)-4,5-dihydro-1,3-thiazol-2-ylamine
the tautomers thereof and the pharmaceutically acceptable salts thereof.

13. Pharmaceutical compositions containing, as active ingredient, at least one compound of formula 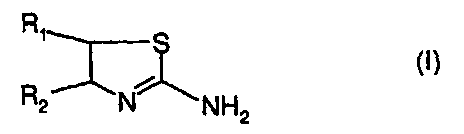 in which either R₁ is a hydrogen atom or an alkyl radical and R₂ is an alkyl, -alk-NH₂, -CH₂-R₃ or -CH₂-S-F₄ radical or a phenyl radical substituted with a nitro or -NH-C(=NH)CH₃ radical,
or R₁ is an alkyl radical and R₂ is a hydrogen atom,
R₃ is a cycloalkyl (3-6C), pyridyl, thienyl, thiazolyl, imidazolyl, pyrazinyl, triazolyl or phenyl radical or a phenyl radical substituted with a nitro, hydroxyl or carboxyl radical,
R₄ represents a pyridyl radical,
alk represents an alkylene radical,
racemic mixtures, enantiomers and diastereoisomers thereof and mixtures thereof, tautomers thereof and pharmaceutically acceptable salts thereof,
with the exception of the racemic compounds for which R₁ is a methyl radical and R₂ is a hydrogen atom or R₁ is hydrogen and R₂ is methyl, ethyl or n-propyl,
it being understood that the alkyl and alkylene radicals contain 1 to 6 carbon atoms in a straight or branched chain.

14. Process for preparing the compounds of formula (I) according to Claim 8, **characterized in that** a derivative of formula: 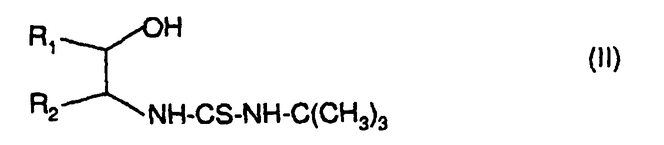 in which R₁ and R₂ have the same meanings as in Claim 8, is cyclized, and the product is isolated and optionally converted into a pharmaceutically acceptable salt.

15. Process for preparing the compounds of formula (I) according to Claim 8, for which R₂ is a phenyl radical substituted with an -NH-C(=NH)CH₃ radical, **characterized in that** a derivative of formula: 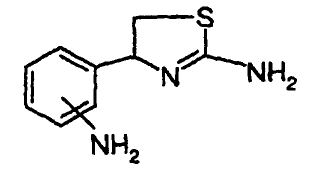 is reacted with benzyl ethanimidothioate hydrochloride, and the product is isolated and optionally converted into a pharmaceutically acceptable salt.

16. Process for preparing the compounds of formula (I) according to Claim 8, for which R₂ is a radical -CH₂-R₃ in which R₃ is a 1-imidazolyl or 1-(1,2,4-triazolyl) radical, **characterized in that** imidazole or 1,2,4-triazole is reacted with a derivative of formula: 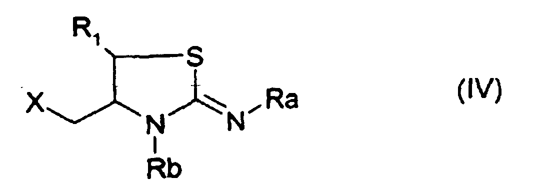 in which R₁ has the same meanings as in Claim 8, X is a halogen atom or a tosyl radical and Ra and Rb are hydrogen atoms or protecting groups for the amine function, optionally followed by a deprotection, and the product is isolated and optionally converted into a pharmaceutically acceptable salt.

17. Process for preparing the compounds of formula (I) according to Claim 8, for which R₂ is a radical -CH₂-S-R₄, **characterized in that** a compound of formula: 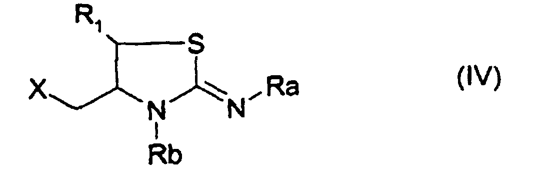 in which R₁ has the same meanings as in Claim 8, X is a halogen atom or a tosyl radical and Ra and Rb are hydrogen atoms or protecting groups for the amine function, is reacted with a derivative of formula HS-R₄ in which R₄ has the same meaning as in Claim 8, optionally followed by a deprotection of the amine function, and the product is isolated and optionally converted into a pharmaceutically acceptable salt.

18. Compounds of formula:

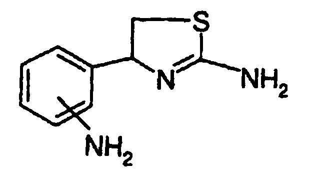

19. Process for preparing the compounds of formula (II) as defined in Claim 14 and in which R₁ is a hydrogen atom, **characterized in that** a compound of formula (IIa) 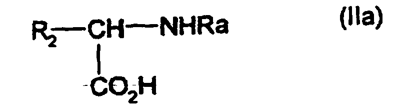 R₂ being as defined in Claim 1 and Ra being either a hydrogen atom or a protecting group for the amine function such as CO₂tBu, is subjected to the action of a reducing agent to give the compounds of formula (IIb) 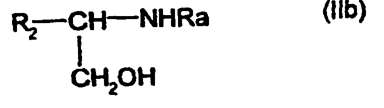 which compound is subjected to the action of a deprotecting agent to give a compound of formula (IIc) 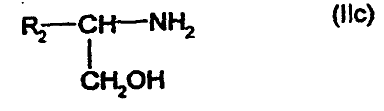 which compound is subjected to the action of tert-butyl thiocyanate to give a compound of formula (IId) 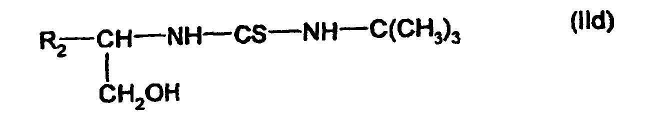 corresponding to the compound of formula (II) with R₁ representing a hydrogen atom.

20. As intermediate compounds, the compounds of formula (IId) as defined in Claim 19.

21. As an intermediate compound, the compound of formula (IId) as defined in Claim 19 and in which R₂ represents a 4-methylpyridyl group.
